# EUROPEAN PATENT SPECIFICATION

(11) **EP 1 040 351 B1**
(45) Date of publication and mention of the grant of the patent: **18.07.2007**
(21) Application number: 98963882.0
(22) Date of filing: 11.12.1998
(51) Int. Cl.: G01N 33/53, G01N 33/566, C07K 14/435, C07K 19/00

(54) **C-TERMINAL REGION OF AGOUTI-RELATED TRANSCRIPT (ART) PROTEIN**
C-TERMINALE REGION DES "AGOUTI-RELATED-TRANSCRIPT"-PROTEINS (ART)
REGION C-TERMINALE DE PROTEINE DE TRANSCRIPT LIE A L'AGOUTI (ART)

(30) Priority: 16.12.1997 US 69747 P
(43) Date of publication of application: 04.10.2000
(73) Proprietor: Merck & Co., Inc., Rahway, New Jersey 07065 (US)
(72) Inventor: FONG, Tung, Ming, Rahway, NJ 07065 (US); VAN DER PLOEG, Leonardus, H., T., Rahway, NJ 07065 (US); TOTA, Michael, R., Rahway, NJ 07065 (US)
(74) Representative: Horgan, James Michael Frederic
(86) International application number: PCT/US1998/026457
(87) International publication number: WO 1999/031508

(56) References cited:
- WO-A-97/43412
- WO-A-97/47316
- US-A- 5 766 877
- OLLMANN M M ET AL: "ANTAGONISM OF CENTRAL MELANOCORTIN RECEPTORS IN VITRO AND IN VIVO BY AGOUTI-RELATED PROTEIN" SCIENCE, AMERICAN ASSOCIATION FOR THE ADVANCEMENT OF SCIENCE,, US, vol. 278, 3 October 1997 (1997-10-03), pages 135-138, XP002923098 ISSN: 0036-8075
- WILLARD DERRIL H ET AL: "Agouti structure and function: Characterization of a potent alpha-melanocyte stimulating hormone receptor antagonist." BIOCHEMISTRY, vol. 34, no. 38, 1995, pages 12341-12346, XP002216829 ISSN: 0006-2960
- BODI J ET AL: "New Strategy for the Synthesis of Large Peptides as Applied to the C-terminal Cysteine-Rich 41 Amino Acid Fragment of the Mouse Agouti Protein" TETRAHEDRON LETTERS, ELSEVIER SCIENCE PUBLISHERS, AMSTERDAM, NL, vol. 38, no. 18, 5 May 1997 (1997-05-05), pages 3293-3296, XP004059834 ISSN: 0040-4039
- HASKELL-LUEVANO C ET AL: "Discovery of Prototype Peptidomimetic Agonists at the Human Melanocortin Receptors MC1R and MC4R" JOURNAL OF MEDICINAL CHEMISTRY, AMERICAN CHEMICAL SOCIETY. WASHINGTON, US, vol. 40, no. 14, 4 July 1997 (1997-07-04), pages 2133-2139, XP002084110 ISSN: 0022-2623
- PERRY W L ET AL: "A transgenic mouse assay for agouti protein activity." GENETICS. UNITED STATES MAY 1995, vol. 140, no. 1, May 1995 (1995-05), pages 267-274, XP001118193 ISSN: 0016-6731
- KWON H.Y. et al., "Molecular Structure and Chromosomal Mapping of the Human Homolog of the Agouti Gene", PROC. NATL. ACAD. SCI. USA, October 1994, Vol. 91, pages 9760-9764, XP002918707
- STARK M.J.R. et al., "The Killer Toxin of Kluyveromyces Lactis: Characterization of the Toxin Subunits and Identification of the Genes Which Encode Them", THE EMBO JOURNAL, 1986, Vol. 5, No. 3, pages 1995-2002, XP002918708
- SHUTTER J.R. et al., "Hypothalamic Expression of ART, a Novel Gene Related to Agouti, is Up-Regulated in Obese and Diabetic Mutant Mice", GENES AND DEVELOPMENT, 1997, Vol. 11, pages 593-602, XP002918709

### SEQUENCE LISTING

(1) GENERAL INFORMATION:
   (i) APPLICANT: Merck & Co., Inc.
   (ii) TITLE OF INVENTION: C-TERMINAL REGION OF AGOUTI-RELATED TRANSCRIPT (ART) PROTEIN
   (iii) NUMBER OF SEQUENCES: 24
   (iv) CORRESPONDENCE ADDRESS:
      (A) ADDRESSEE: Merck & Co., Inc.
      (B) STREET: P.O. Box 2000, 126 E. Lincoln Ave.
      (C) CITY: Rahway
      (D) STATE: NJ
      (E) COUNTRY: USA
      (F) ZIP: 07065-0900
   (v) COMPUTER READABLE FORM:
      (A) MEDIUM TYPE: Diskette
      (B) COMPUTER: IBM Compatible
      (C) OPERATING SYSTEM: Windows
      (D) SOFTWARE: FastSEQ for Windows Version 2.0b
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: 60/069,747
      (B) FILING DATE: 16-DEC-1997
   (viii) ATTORNEY/AGENT INFORMATION:
      (A) NAME: Coppola, Joseph A
      (B) REGISTRATION NUMBER: 38,413
      (C) REFERENCE/DOCKET NUMBER: 20146 PCT
   (ix) TELECOMMUNICATION INFORMATION:
      (A) TELEPHONE: 732-594-6734
      (B) TELEFAX: 732-594-4720
      (C) TELEX:
(2) INFORMATION FOR SEQ ID NO:1:
   (i) SEQUENCE CHARACTERISTICS:
      (A) LENGTH: 118 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:1:
   Met Asn Ile Phe Tyr Ile Phe Leu Phe Leu Leu Ser Phe Val Gln Gly
    1               5                  10                  15
   Leu Glu His Thr His Arg Arg Gly Ser Leu Val Lys Arg Ser Ser Leu
               20                  25                  30
   Gln Asp Arg Glu Pro Arg Ser Ser Arg Arg Cys Val Arg Leu His Glu
           35                  40                  45
   Ser Cys Leu Gly Gln Gln Val Pro Cys Cys Asp Pro Cys Ala Thr Cys
       50                  55                  60
   Tyr Cys Arg Phe Phe Asn Ala Phe Cys Tyr Cys Arg Lys Leu Gly Thr
   65                  70                  75                  80
   Ala Met Asn Pro Cys Ser Arg Thr Leu Val Pro Arg Gly Ser Glu Gln
                   85                  90                  95
   Lys Leu Ile Ser Glu Glu Asp Leu Asn Leu Arg Arg Ala Ser Leu Gly
               100                 105                 110
   His His His His His His
           115
(2) INFORMATION FOR SEQ ID NO:2:
   (i) SEQUENCE CHARACTERISTICS:
      (A) LENGTH: 120 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:2:
   Met Leu Thr Ala Ala Leu Leu Ser Cys Ala Leu Leu Leu Ala Leu Pro
    1               5                  10                  15
   Ala Thr Arg Gly Ala Gln Met Gly Leu Ala Leu Gln Asp Arg Glu Pro
               20                  25                  30
   Arg Ser Ser Arg Arg Cys Val Arg Leu His Glu Ser Cys Leu Gly Gln
           35                  40                  45
   Gln Val Pro Cys Cys Asp Pro Cys Ala Thr Cys Tyr Cys Arg Phe Phe
       50                  55                  60
   Asn Ala Phe Cys Tyr Cys Arg Lys Leu Gly Thr Ala Met Asn Pro Cys
   65                  70                  75                  80
   Ser Arg Thr Leu Val Pro Arg Gly Ser Gly Ser Glu Leu Gly Thr Lys
                   85                  90                  95
   Leu Gly Pro Glu Gln Lys Leu Ile Ser Glu Glu Asp Leu Asn Ser Ala
               100                 105                 110
   Val Asp His His His His His His
           115                 120
(2) INFORMATION FOR SEQ ID NO:3:
   (i) SEQUENCE CHARACTERISTICS:
      (A) LENGTH: 121 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:3:
   Met Leu Thr Ala Ala Leu Leu Ser Cys Ala Leu Leu Leu Ala Leu Pro
    1               5                  10                  15
   Ala Thr Arg Gly Ala Gln Met Gly Leu Ala Leu Gln Asp Arg Glu Pro
               20                  25                  30
   Arg Ser Ser Arg Arg Cys Val Arg Leu His Glu Ser Cys Leu Gly Gln
           35                  40                  45
   Gln Val Pro Cys Cys Asp Pro Cys Ala Thr Cys Tyr Cys Arg Phe Phe
       50                  55                  60
   Asn Ala Phe Cys Tyr Cys Arg Lys Leu Gly Thr Ala Met Asn Pro Cys
   65                  70                  75                  80
   Ser Arg Thr Leu Val Pro Arg Gly Ser Gly Ser Leu Arg Arg Ala Ser
                   85                  90                  95
   Leu Gly Lys Leu Glu Gln Lys Leu Ile Ser Glu Glu Asp Leu Asn Ser
               100                 105                 110
   Ala Val Asp His His His His His His
           115                 120
(2) INFORMATION FOR SEQ ID NO:4:
   (i) SEQUENCE CHARACTERISTICS:
      (A) LENGTH: 654 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:4:
(2) INFORMATION FOR SEQ ID NO:5:
   (i) SEQUENCE CHARACTERISTICS:
      (A) LENGTH: 715 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:5:
   Met Leu Thr Ala Ala Leu Leu Ser Cys Ala Leu Leu Leu Ala Leu Pro
    1               5                  10                  15
   Ala Thr Arg Gly Ala Gln Met Gly Leu Ala Pro Met Glu Gly Ile Arg
               20                  25                  30
   Arg Pro Asp Gln Ala Leu Leu Pro Glu Leu Pro Gly Leu Gly Leu Arg
           35                  40                  45
   Ala Pro Leu Lys Lys Thr Asn Ala Glu Gln Ala Glu Glu Asp Leu Leu
       50                  55                  60
   Gln Glu Ala Gln Ala Leu Ala Glu Val Leu Asp Leu Gln Asp Arg Glu
   65                  70                  75                  80
   Pro Arg Ser Ser Arg Arg Cys Val Arg Leu His Glu Ser Cys Leu Gly
                   85                  90                  95
   Gln Gln Val Pro Cys Cys Asp Pro Cys Ala Thr Cys Tyr Cys Arg Phe
               100                 105                 110
   Phe Asn Ala Phe Cys Tyr Cys Arg Lys Leu Gly Thr Ala Met Asn Pro
           115                 120                 125
   Cys Ser Arg Thr Leu Val Pro Arg Gly Ser Gly Ser Met Ser Ile Glu
       130                 135                 140
   Asn Asn Ile Leu Ile Gly Pro Pro Pro Tyr Tyr Pro Leu Glu Glu Gly
   145                 150                 155                 160
   Thr Ala Gly Glu Gln Leu His Arg Ala Ile Ser Arg Tyr Ala Ala Val
                   165                 170                 175
   Pro Gly Thr Leu Ala Tyr Thr Asp Val His Thr Glu Leu Glu Val Thr
               180                 185                 190
   Tyr Lys Glu Phe Leu Asp Val Thr Cys Arg Leu Ala Glu Ala Met Lys
           195                 200                 205
   Asn Tyr Gly Leu Gly Leu Gln His Thr Ile Ser Val Cys Ser Glu Asn
       210                 215                 220
   Cys Val Gln Phe Phe Met Pro Ile Cys Ala Ala Leu Tyr Val Gly Val
   225                 230                 235                 240
   Ala Thr Ala Pro Thr Asn Asp Ile Tyr Asn Glu Arg Glu Leu Tyr Asn
                   245                 250                 255
   Ser Leu Ser Ile Ser Gln Pro Thr Val Val Phe Thr Ser Arg Asn Ser
               260                 265                 270
   Leu Gln Lys Ile Leu Gly Val Gln Ser Arg Leu Pro Ile Ile Lys Lys
           275                 280                 285
   Ile Ile Ile Leu Asp Gly Lys Lys Asp Tyr Leu Gly Tyr Gln Ser Met
       290                 295                 300
   Gln Ser Phe Met Lys Glu His Val Pro Ala Asn Phe Asn Val Ser Ala
   305                 310                 315                 320
   Phe Lys Pro Leu Ser Phe Asp Leu Asp Arg Val Ala Cys Ile Met Asn
                   325                 330                 335
   Ser Ser Gly Ser Thr Gly Leu Pro Lys Gly Val Pro Ile Ser His Arg
               340                 345                 350
   Asn Thr Ile Tyr Arg Phe Ser His Cys Arg Asp Pro Val Phe Gly Asn
           355                 360                 365
   Gln Ile Ile Pro Asp Thr Thr Ile Leu Cys Ala Val Pro Phe His His
       370                 375                 380
   Ala Phe Gly Thr Phe Thr Asn Leu Gly Tyr Leu Ile Cys Gly Phe His
   385                 390                 395                 400
   Val Val Leu Met Tyr Arg Phe Asn Glu His Leu Phe Leu Gln Thr Leu
                   405                 410                 415
   Gln Asp Tyr Lys Cys Gln Ser Ala Leu Leu Val Pro Thr Val Leu Ala
               420                 425                 430
   Phe Leu Ala Lys Asn Pro Leu Val Asp Lys Tyr Asp Leu Ser Asn Leu
           435                 440                 445
   His Glu Ile Ala Ser Gly Gly Ala Pro Leu Ser Lys Glu Ile Ser Glu
       450                 455                 460
   Ile Ala Ala Lys Arg Phe Lys Leu Pro Gly Ile Arg Gln Gly Tyr Gly
   465                 470                 475                 480
   Leu Thr Glu Thr Thr Cys Ala Ile Val Ile Thr Ala Glu Gly Glu Phe
                   485                 490                 495
   Lys Leu Gly Ala Val Gly Lys Val Val Pro Phe Tyr Ser Leu Lys Val
               500                 505                 510
   Leu Asp Leu Asn Thr Gly Lys Lys Leu Gly Pro Asn Glu Arg Gly Glu
           515                 520                 525
   Ile Cys Phe Lys Gly Pro Met Ile Met Lys Gly Tyr Ile Asn Asn Pro
       530                 535                 540
   Glu Ala Thr Arg Glu Leu Ile Asp Glu Glu Gly Trp Ile His Ser Gly
   545                 550                 555                 560
   Asp Ile Gly Tyr Phe Asp Glu Asp Gly His Val Tyr Ile Val Asp Arg
                   565                 570                 575
   Leu Lys Ser Leu Ile Lys Tyr Lys Gly Tyr Gln Val Pro Pro Ala Glu
               580                 585                 590
   Leu Glu Ala Leu Leu Leu Gln His Pro Phe Ile Glu Asp Ala Gly Val
           595                 600                 605
   Ala Gly Val Pro Asp Glu Val Ala Gly Asp Leu Pro Gly Ala Val Val
       610                 615                 620
   Val Leu Lys Glu Gly Lys Ser Ile Thr Glu Lys Glu Ile Gln Asp Tyr
   625                 630                 635                 640
   Val Ala Gly Gln Val Thr Ser Ser Lys Lys Leu Arg Gly Gly Val Glu
                   645                 650                 655
   Phe Val Lys Glu Val Pro Lys Gly Phe Thr Gly Lys Ile Asp Thr Arg
               660                 665                 670
   Lys Ile Lys Glu Ile Leu Ile Lys Ala Gln Lys Gly Lys Ser Lys Ser
           675                 680                 685
   Lys Ala Lys Leu Gly Pro Glu Gln Lys Leu Ile Ser Glu Glu Asp Leu
       690                 695                 700
   Asn Ser Ala Val Asp His His His His His His
   705                 710                 715
(2) INFORMATION FOR SEQ ID NO:6:
   (i) SEQUENCE CHARACTERISTICS:
      (A) LENGTH: 83 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:6:
   Met Leu Thr Ala Ala Leu Leu Ser Cys Ala Leu Leu Leu Ala Leu Pro
    1               5                  10                  15
   Ala Thr Arg Gly Ala Gln Met Gly Leu Ala Leu Gln Asp Arg Glu Pro
               20                  25                  30
   Arg Ser Ser Arg Arg Cys Val Arg Leu His Glu Ser Cys Leu Gly Gln
           35                  40                  45
   Gln Val Pro Cys Cys Asp Pro Cys Ala Thr Cys Tyr Cys Arg Phe Phe
       50                  55                  60
   Asn Ala Phe Cys Tyr Cys Arg Lys Leu Gly Thr Ala Met Asn Pro Cys
   65                  70                  75                  80
   Ser Arg Thr
(2) INFORMATION FOR SEQ ID NO:7:
   (i) SEQUENCE CHARACTERISTICS:
      (A) LENGTH: 57 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:7:
   Leu Gln Asp Arg Glu Pro Arg Ser Ser Arg Arg Cys Val Arg Leu His
    1               5                  10                  15
   Glu Ser Cys Leu Gly Gln Gln Val Pro Cys Cys Asp Pro Cys Ala Thr
               20                  25                  30
   Cys Tyr Cys Arg Phe Phe Asn Ala Phe Cys Tyr Cys Arg Lys Leu Gly
           35                  40                  45
   Thr Ala Met Asn Pro Cys Ser Arg Thr
       50                  55
(2) INFORMATION FOR SEQ ID NO:8:
   (i) SEQUENCE CHARACTERISTICS:
      (A) LENGTH: 83 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:8:
   Met Leu Thr Ala Met Leu Leu Ser Cys Val Leu Leu Leu Ala Leu Pro
    1               5                  10                  15
   Pro Thr Leu Gly Val Gln Met Gly Val Ala Pro Gln Asn Arg Glu Ser
               20                  25                  30
   Arg Ser Pro Arg Arg Cys Val Arg Leu His Glu Ser Cys Leu Gly Gln
           35                  40                  45
   Gln Val Pro Cys Cys Asp Pro Cys Ala Thr Cys Tyr Cys Arg Phe Phe
       50                  55                  60
   Asn Ala Phe Cys Tyr Cys Arg Lys Leu Gly Thr Ala Met Asn Leu Cys
   65                  70                  75                  80
   Ser Arg Thr
(2) INFORMATION FOR SEQ ID NO:9:
   (i) SEQUENCE CHARACTERISTICS:
      (A) LENGTH: 57 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:9:
   Pro Gln Asn Arg Glu Ser Arg Ser Pro Arg Arg Cys Val Arg Leu His
    1               5                  10                  15
   Glu Ser Cys Leu Gly Gln Gln Val Pro Cys Cys Asp Pro Cys Ala Thr
               20                  25                  30
   Cys Tyr Cys Arg Phe Phe Asn Ala Phe Cys Tyr Cys Arg Lys Leu Gly
           35                  40                  45
   Thr Ala Met Asn Leu Cys Ser Arg Thr
       50                  55
(2) INFORMATION FOR SEQ ID NO:10:
   (i) SEQUENCE CHARACTERISTICS:
      (A) LENGTH: 118 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:10:
   Met Asn Ile Phe Tyr Ile Phe Leu Phe Leu Leu Ser Phe Val Gln Gly
    1               5                  10                  15
   Leu Glu His Thr His Arg Arg Gly Ser Leu Val Lys Arg Ser Ser Pro
               20                  25                  30
   Gln Asn Arg Glu Ser Arg Ser Pro Arg Arg Cys Val Arg Leu His Glu
           35                  40                  45
   Ser Cys Leu Gly Gln Gln Val Pro Cys Cys Asp Pro Cys Ala Thr Cys
       50                  55                  60
   Tyr Cys Arg Phe Phe Asn Ala Phe Cys Tyr Cys Arg Lys Leu Gly Thr
   65                  70                  75                  80
   Ala Met Asn Leu Cys Ser Arg Thr Leu Val Pro Arg Gly Ser Glu Gln
                   85                  90                  95
   Lys Leu Ile Ser Glu Glu Asp Leu Asn Leu Arg Arg Ala Ser Leu Gly
               100                 105                 110
   His His His His His His
           115
(2) INFORMATION FOR SEQ ID NO:11:
   (i) SEQUENCE CHARACTERISTICS:
      (A) LENGTH: 113 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:11:
   Met Leu Thr Ala Met Leu Leu Ser Cys Val Leu Leu Leu Ala Leu Pro
    1               5                  10                  15
   Pro Thr Leu Gly Val Gln Met Gly Val Ala Pro Gln Asn Arg Glu Ser
               20                  25                  30
   Arg Ser Pro Arg Arg Cys Val Arg Leu His Glu Ser Cys Leu Gly Gln
           35                  40                  45
   Gln Val Pro Cys Cys Asp Pro Cys Ala Thr Cys Tyr Cys Arg Phe Phe
       50                  55                  60
   Asn Ala Phe Cys Tyr Cys Arg Lys Leu Gly Thr Ala Met Asn Leu Cys
   65                  70                  75                  80
   Ser Arg Thr Leu Val Pro Arg Gly Ser Glu Gln Lys Leu Ile Ser Glu
                   85                  90                  95
   Glu Asp Leu Asn Leu Arg Arg Ala Ser Leu Ser His His His His His
               100                 105                 110
   His
(2) INFORMATION FOR SEQ ID NO:12:
   (i) SEQUENCE CHARACTERISTICS:
      (A) LENGTH: 117 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:12:
   Met Leu Thr Ala Met Leu Leu Ser Cys Val Leu Leu Leu Ala Leu Pro
    1               5                  10                  15
   Pro Thr Leu Gly Val Gln Met Gly Val Ala Pro Gln Asn Arg Glu Ser
               20                  25                  30
   Arg Ser Pro Arg Arg Cys Val Arg Leu His Glu Ser Cys Leu Gly Gln
           35                  40                  45
   Gln Val Pro Cys Cys Asp Pro Cys Ala Thr Cys Tyr Cys Arg Phe Phe
       50                  55                  60
   Asn Ala Phe Cys Tyr Cys Arg Lys Leu Gly Thr Ala Met Asn Leu Cys
   65                  70                  75                  80
   Ser Arg Thr Leu Val Pro Arg Gly Ser Gly Ser Leu Arg Arg Ala Ser
                   85                  90                  95
   Leu Gly Lys Leu Glu Gln Lys Leu Ile Ser Glu Glu Asp Leu Asn His
               100                 105                 110
   His His His His His
           115
(2) INFORMATION FOR SEQ ID NO:13:
   (i) SEQUENCE CHARACTERISTICS:
      (A) LENGTH: 620 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:13:
   Met Leu Thr Ala Met Leu Leu Ser Cys Val Leu Leu Leu Ala Leu Pro
    1               5                  10                  15
   Pro Thr Leu Gly Val Gln Met Gly Val Ala Pro Leu Lys Gly Ile Arg
               20                  25                  30
   Arg Pro Asp Gln Ala Leu Phe Pro Glu Phe Pro Gly Leu Ser Leu Asn
           35                  40                  45
   Gly Leu Lys Lys Thr Asn Ala Asp Arg Ala Glu Glu Val Leu Leu Gln
       50                  55                  60
   Lys Ala Glu Ala Leu Ala Glu Val Leu Asp Pro Gln Asn Arg Glu Ser
   65                  70                  75                  80
   Arg Ser Pro Arg Arg Cys Val Arg Leu His Glu Ser Cys Leu Gly Gln
                   85                  90                  95
   Gln Val Pro Cys Cys Asp Pro Cys Ala Thr Cys Tyr Cys Arg Phe Phe
               100                 105                 110
   Asn Ala Phe Cys Tyr Cys Arg Lys Leu Gly Thr Ala Met Asn Leu Cys
           115                 120                 125
   Ser Arg Thr Ile Ile Pro Val Glu Glu Glu Asn Pro Asp Phe Trp Asn
       130                 135                 140
   Arg Gln Ala Ala Glu Ala Leu Gly Ala Ala Lys Lys Leu Gln Pro Ala
   145                 150                 155                 160
   Gln Thr Ala Ala Lys Asn Leu Ile Ile Phe Leu Gly Asp Gly Met Gly
                   165                 170                 175
   Val Ser Thr Val Thr Ala Ala Arg Ile Leu Lys Gly Gln Lys Lys Asp
               180                 185                 190
   Lys Leu Gly Pro Glu Thr Phe Leu Ala Met Asp Arg Phe Pro Tyr Val
           195                 200                 205
   Ala Leu Ser Lys Thr Tyr Ser Val Asp Lys His Val Pro Asp Ser Gly
       210                 215                 220
   Ala Thr Ala Thr Ala Tyr Leu Cys Gly Val Lys Gly Asn Phe Gln Thr
   225                 230                 235                 240
   Ile Gly Leu Ser Ala Ala Ala Arg Phe Asn Gln Cys Asn Thr Thr Arg
                   245                 250                 255
   Gly Asn Glu Val Ile Ser Val Met Asn Arg Ala Lys Lys Ala Gly Lys
               260                 265                 270
   Ser Val Gly Val Val Thr Thr Thr Arg Val Gln His Ala Ser Pro Ala
           275                 280                 285
   Gly Ala Tyr Ala His Thr Val Asn Arg Asn Trp Tyr Ser Asp Ala Asp
       290                 295                 300
   Val Pro Ala Ser Ala Arg Gln Glu Gly Cys Gln Asp Ile Ala Thr Gln
   305                 310                 315                 320
   Leu Ile Ser Asn Met Asp Ile Asp Val Ile Leu Gly Gly Gly Arg Lys
                   325                 330                 335
   Tyr Met Phe Pro Met Gly Thr Pro Asp Pro Glu Tyr Pro Asp Asp Tyr
               340                 345                 350
   Ser Gln Gly Gly Thr Arg Leu Asp Gly Lys Asn Leu Val Gln Glu Trp
           355                 360                 365
   Leu Ala Lys His Gln Gly Ala Arg Tyr Val Trp Asn Arg Thr Glu Leu
       370                 375                 380
   Met Gln Ala Ser Leu Asp Pro Ser Val Thr His Leu Met Gly Leu Phe
   385                 390                 395                 400
   Glu Pro Gly Asp Met Lys Tyr Glu Ile His Arg Asp Ser Thr Leu Asp
                   405                 410                 415
   Pro Ser Leu Met Glu Met Thr Glu Ala Ala Leu Arg Leu Leu Ser Arg
               420                 425                 430
   Asn Pro Arg Gly Phe Phe Leu Phe Val Glu Gly Gly Arg Ile Asp His
           435                 440                 445
   Gly His His Glu Ser Arg Ala Tyr Arg Ala Leu Thr Glu Thr Ile Met
       450                 455                 460
   Phe Asp Asp Ala Ile Glu Arg Ala Gly Gln Leu Thr Ser Glu Glu Asp
   465                 470                 475                 480
   Thr Leu Ser Leu Val Thr Ala Asp His Ser His Val Phe Ser Phe Gly
                   485                 490                 495
   Gly Tyr Pro Leu Arg Gly Ser Ser Ile Phe Gly Leu Ala Pro Gly Lys
               500                 505                 510
   Ala Arg Asp Arg Lys Ala Tyr Thr Val Leu Leu Tyr Gly Asn Gly Pro
           515                 520                 525
   Gly Tyr Val Leu Lys Asp Gly Ala Arg Pro Asp Val Thr Glu Ser Glu
       530                 535                 540
   Ser Gly Ser Pro Glu Tyr Arg Gln Gln Ser Ala Val Pro Leu Asp Gly
   545                 550                 555                 560
   Glu Thr His Ala Gly Glu Asp Val Ala Val Phe Ala Arg Gly Pro Gln
                   565                 570                 575
   Ala His Leu Val His Gly Val Gln Glu Gln Thr Phe Ile Ala His Val
               580                 585                 590
   Met Ala Phe Ala Ala Cys Leu Glu Pro Tyr Thr Ala Cys Asp Leu Ala
           595                 600                 605
   Pro Ser Ala Gly Thr Thr Asp Ala Ala His Pro Gly
       610                 615                 620
(2) INFORMATION FOR SEQ ID NO:14:
   (i) SEQUENCE CHARACTERISTICS:
      (A) LENGTH: 683 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:14:
   Met Leu Thr Ala Met Leu Leu Ser Cys Val Leu Leu Leu Ala Leu Pro
    1               5                  10                  15
   Pro Thr Leu Gly Val Gln Met Gly Val Ala Pro Leu Lys Gly Ile Arg
               20                  25                  30
   Arg Pro Asp Gln Ala Leu Phe Pro Glu Phe Pro Gly Leu Ser Leu Asn
               35                  40                  45
   Gly Leu Lys Lys Thr Asn Ala Asp Arg Ala Glu Glu Val Leu Leu Gln
           50                  55                  60
   Lys Ala Glu Ala Leu Ala Glu Val Leu Asp Pro Gln Asn Arg Glu Ser
   65                  70                  75                  80
   Arg Ser Pro Arg Arg Cys Val Arg Leu His Glu Ser Cys Leu Gly Gln
                   85                  90                  95
   Gln Val Pro Cys Cys Asp Pro Cys Ala Thr Cys Tyr Cys Arg Phe Phe
               100                 105                 110
   Asn Ala Phe Cys Tyr Cys Arg Lys Leu Gly Thr Ala Met Asn Leu Cys
           115                 120                 125
   Ser Arg Thr Met Ser Ile Glu Asn Asn Ile Leu Ile Gly Pro Pro Pro
       130                 135                 140
   Tyr Tyr Pro Leu Glu Glu Gly Thr Ala Gly Glu Gln Leu His Arg Ala
   145                 150                 155                 160
   Ile Ser Arg Tyr Ala Ala Val Pro Gly Thr Leu Ala Tyr Thr Asp Val
                   165                 170                 175
   His Thr Glu Leu Glu Val Thr Tyr Lys Glu Phe Leu Asp Val Thr Cys
               180                 185                 190
   Arg Leu Ala Glu Ala Met Lys Asn Tyr Gly Leu Gly Leu Gln His Thr
           195                 200                 205
   Ile Ser Val Cys Ser Glu Asn Cys Val Gln Phe Phe Met Pro Ile Cys
       210                 215                 220
   Ala Ala Leu Tyr Val Gly Val Ala Thr Ala Pro Thr Asn Asp Ile Tyr
   225                 230                 235                 240
   Asn Glu Arg Glu Leu Tyr Asn Ser Leu Ser Ile Ser Gln Pro Thr Val
                   245                 250                 255
   Val Phe Thr Ser Arg Asn Ser Leu Gln Lys Ile Leu Gly Val Gln Ser
               260                 265                 270
   Arg Leu Pro Ile Ile Lys Lys Ile Ile Ile Leu Asp Gly Lys Lys Asp
           275                 280                 285
   Tyr Leu Gly Tyr Gln Ser Met Gln Ser Phe Met Lys Glu His Val Pro
       290                 295                 300
   Ala Asn Phe Asn Val Ser Ala Phe Lys Pro Leu Ser Phe Asp Leu Asp
   305                 310                 315                 320
   Arg Val Ala Cys Ile Met Asn Ser Ser Gly Ser Thr Gly Leu Pro Lys
                   325                 330                 335
   Gly Val Pro Ile Ser His Arg Asn Thr Ile Tyr Arg Phe Ser His Cys
               340                 345                 350
   Arg Asp Pro Val Phe Gly Asn Gln Ile Ile Pro Asp Thr Thr Ile Leu
           355                 360                 365
   Cys Ala Val Pro Phe His His Ala Phe Gly Thr Phe Thr Asn Leu Gly
       370                 375                 380
   Tyr Leu Ile Cys Gly Phe His Val Val Leu Met Tyr Arg Phe Asn Glu
   385                 390                 395                 400
   His Leu Phe Leu Gln Thr Leu Gln Asp Tyr Lys Cys Gln Ser Ala Leu
                   405                 410                 415
   Leu Val Pro Thr Val Leu Ala Phe Leu Ala Lys Asn Pro Leu Val Asp
               420                 425                 430
   Lys Tyr Asp Leu Ser Asn Leu His Glu Ile Ala Ser Gly Gly Ala Pro
           435                 440                 445
   Leu Ser Lys Glu Ile Ser Glu Ile Ala Ala Lys Arg Phe Lys Leu Pro
       450                 455                 460
   Gly Ile Arg Gln Gly Tyr Gly Leu Thr Glu Thr Thr Cys Ala Ile Val
   465                 470                 475                 480
   Ile Thr Ala Glu Gly Glu Phe Lys Leu Gly Ala Val Gly Lys Val Val
                   485                 490                 495
   Pro Phe Tyr Ser Leu Lys Val Leu Asp Leu Asn Thr Gly Lys Lys Leu
               500                 505                 510
   Gly Pro Asn Glu Arg Gly Glu Ile Cys Phe Lys Gly Pro Met Ile Met
           515                 520                 525
   Lys Gly Tyr Ile Asn Asn Pro Glu Ala Thr Arg Glu Leu Ile Asp Glu
       530                 535                 540
   Glu Gly Trp Ile His Ser Gly Asp Ile Gly Tyr Phe Asp Glu Asp Gly
   545                 550                 555                 560
   His Val Tyr Ile Val Asp Arg Leu Lys Ser Leu Ile Lys Tyr Lys Gly
                   565                 570                 575
   Tyr Gln Val Pro Pro Ala Glu Leu Glu Ala Leu Leu Leu Gln His Pro
               580                 585                 590
   Phe Ile Glu Asp Ala Gly Val Ala Gly Val Pro Asp Glu Val Ala Gly
           595                 600                 605
   Asp Leu Pro Gly Ala Val Val Val Leu Lys Glu Gly Lys Ser Ile Thr
       610                 615                 620
   Glu Lys Glu Ile Gln Asp Tyr Val Ala Gly Gln Val Thr Ser Ser Lys
   625                 630                 635                 640
   Lys Leu Arg Gly Gly Val Glu Phe Val Lys Glu Val Pro Lys Gly Phe
                   645                 650                 655
   Thr Gly Lys Ile Asp Thr Arg Lys Ile Lys Glu Ile Leu Ile Lys Ala
               660                 665                 670
   Gln Lys Gly Lys Ser Lys Ser Lys Ala Lys Leu
           675                 680
(2) INFORMATION FOR SEQ ID NO:15:
   (i) SEQUENCE CHARACTERISTICS:
      (A) LENGTH: 605 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:15:
   Met Leu Thr Ala Ala Leu Leu Ser Cys Ala Leu Leu Leu Ala Leu Pro
    1               5                  10                  15
   Ala Thr Arg Gly Ala Gln Met Gly Leu Ala Leu Gln Asp Arg Glu Pro
               20                  25                  30
   Arg Ser Ser Arg Arg Cys Val Arg Leu His Glu Ser Cys Leu Gly Gln
           35                  40                  45
   Gln Val Pro Cys Cys Asp Pro Cys Ala Thr Cys Tyr Cys Arg Phe Phe
       50                  55                  60
   Asn Ala Phe Cys Tyr Cys Arg Lys Leu Gly Thr Ala Met Asn Pro Cys
   65                  70                  75                  80
   Ser Arg Thr Leu Val Pro Arg Gly Ser Gly Ser Ile Ile Pro Val Glu
                   85                  90                  95
   Glu Glu Asn Pro Asp Phe Trp Asn Arg Gln Ala Ala Glu Ala Leu Gly
               100                 105                 110
   Ala Ala Lys Lys Leu Gln Pro Ala Gln Thr Ala Ala Lys Asn Leu Ile
           115                 120                 125
   Ile Phe Leu Gly Asp Gly Met Gly Val Ser Thr Val Thr Ala Ala Arg
       130                 135                 140
   Ile Leu Lys Gly Gln Lys Lys Asp Lys Leu Gly Pro Glu Thr Phe Leu
   145                 150                 155                 160
   Ala Met Asp Arg Phe Pro Tyr Val Ala Leu Ser Lys Thr Tyr Ser Val
                   165                 170                 175
   Asp Lys His Val Pro Asp Ser Gly Ala Thr Ala Thr Ala Tyr Leu Cys
               180                 185                 190
   Gly Val Lys Gly Asn Phe Gln Thr Ile Gly Leu Ser Ala Ala Ala Arg
           195                 200                 205
   Phe Asn Gln Cys Asn Thr Thr Arg Gly Asn Glu Val Ile Ser Val Met
       210                 215                 220
   Asn Arg Ala Lys Lys Ala Gly Lys Ser Val Gly Val Val Thr Thr Thr
   225                 230                 235                 240
   Arg Val Gln His Ala Ser Pro Ala Gly Ala Tyr Ala His Thr Val Asn
                   245                 250                 255
   Arg Asn Trp Tyr Ser Asp Ala Asp Val Pro Ala Ser Ala Arg Gln Glu
               260                 265                 270
   Gly Cys Gln Asp Ile Ala Thr Gln Leu Ile Ser Asn Met Asp Ile Asp
           275                 280                 285
   Val Ile Leu Gly Gly Gly Arg Lys Tyr Met Phe Pro Met Gly Thr Pro
       290                 295                 300
   Asp Pro Glu Tyr Pro Asp Asp Tyr Ser Gln Gly Gly Thr Arg Leu Asp
   305                 310                 315                 320
   Gly Lys Asn Leu Val Gln Glu Trp Leu Ala Lys His Gln Gly Ala Arg
                   325                 330                 335
   Tyr Val Trp Asn Arg Thr Glu Leu Met Gln Ala Ser Leu Asp Pro Ser
               340                 345                 350
   Val Thr His Leu Met Gly Leu Phe Glu Pro Gly Asp Met Lys Tyr Glu
           355                 360                 365
   Ile His Arg Asp Ser Thr Leu Asp Pro Ser Leu Met Glu Met Thr Glu
       370                 375                 380
   Ala Ala Leu Arg Leu Leu Ser Arg Asn Pro Arg Gly Phe Phe Leu Phe
   385                 390                 395                 400
   Val Glu Gly Gly Arg Ile Asp His Gly His His Glu Ser Arg Ala Tyr
                   405                 410                 415
   Arg Ala Leu Thr Glu Thr Ile Met Phe Asp Asp Ala Ile Glu Arg Ala
               420                 425                 430
   Gly Gln Leu Thr Ser Glu Glu Asp Thr Leu Ser Leu Val Thr Ala Asp
           435                 440                 445
   His Ser His Val Phe Ser Phe Gly Gly Tyr Pro Leu Arg Gly Ser Ser
       450                 455                 460
   Ile Phe Gly Leu Ala Pro Gly Lys Ala Arg Asp Arg Lys Ala Tyr Thr
   465                 470                 475                 480
   Val Leu Leu Tyr Gly Asn Gly Pro Gly Tyr Val Leu Lys Asp Gly Ala
                   485                 490                 495
   Arg Pro Asp Val Thr Glu Ser Glu Ser Gly Ser Pro Glu Tyr Arg Gln
               500                 505                 510
   Gln Ser Ala Val Pro Leu Asp Gly Glu Thr His Ala Gly Glu Asp Val
           515                 520                 525
   Ala Val Phe Ala Arg Gly Pro Gln Ala His Leu Val His Gly Val Gln
       530                 535                 540
   Glu Gln Thr Phe Ile Ala His Val Met Ala Phe Ala Ala Cys Leu Glu
   545                 550                 555                 560
   Pro Tyr Thr Ala Cys Asp Leu Ala Pro Ser Ala Gly Thr Thr Asp Ala
                   565                 570                 575
   Ala His Pro Gly Lys Leu Gly Pro Glu Gln Lys Leu Ile Ser Glu Glu
               580                 585                 590
   Asp Leu Asn Ser Ala Val Asp His His His His His His
           595                 600                 605
(2) INFORMATION FOR SEQ ID NO:16:
   (i) SEQUENCE CHARACTERISTICS:
      (A) LENGTH: 666 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:16:
   Met Leu Thr Ala Ala Leu Leu Ser Cys Ala Leu Leu Leu Ala Leu Pro
    1               5                  10                  15
   Ala Thr Arg Gly Ala Gln Met Gly Leu Ala Leu Gln Asp Arg Glu Pro
               20                  25                  30
   Arg Ser Ser Arg Arg Cys Val Arg Leu His Glu Ser Cys Leu Gly Gln
           35                  40                  45
   Gln Val Pro Cys Cys Asp Pro Cys Ala Thr Cys Tyr Cys Arg Phe Phe
       50                  55                  60
   Asn Ala Phe Cys Tyr Cys Arg Lys Leu Gly Thr Ala Met Asn Pro Cys
   65                  70                  75                  80
   Ser Arg Thr Leu Val Pro Arg Gly Ser Gly Ser Met Ser Ile Glu Asn
                   85                  90                  95
   Asn Ile Leu Ile Gly Pro Pro Pro Tyr Tyr Pro Leu Glu Glu Gly Thr
               100                 105                 110
   Ala Gly Glu Gln Leu His Arg Ala Ile Ser Arg Tyr Ala Ala Val Pro
           115                 120                 125
   Gly Thr Leu Ala Tyr Thr Asp Val His Thr Glu Leu Glu Val Thr Tyr
       130                 135                 140
   Lys Glu Phe Leu Asp Val Thr Cys Arg Leu Ala Glu Ala Met Lys Asn
   145                 150                 155                 160
   Tyr Gly Leu Gly Leu Gln His Thr Ile Ser Val Cys Ser Glu Asn Cys
                   165                 170                 175
   Val Gln Phe Phe Met Pro Ile Cys Ala Ala Leu Tyr Val Gly Val Ala
               180                 185                 190
   Thr Ala Pro Thr Asn Asp Ile Tyr Asn Glu Arg Glu Leu Tyr Asn Ser
           195                 200                 205
   Leu Ser Ile Ser Gln Pro Thr Val Val Phe Thr Ser Arg Asn Ser Leu
       210                 215                 220
   Gln Lys Ile Leu Gly Val Gln Ser Arg Leu Pro Ile Ile Lys Lys Ile
   225                 230                 235                 240
   Ile Ile Leu Asp Gly Lys Lys Asp Tyr Leu Gly Tyr Gln Ser Met Gln
                   245                 250                 255
   Ser Phe Met Lys Glu His Val Pro Ala Asn Phe Asn Val Ser Ala Phe
               260                 265                 270
   Lys Pro Leu Ser Phe Asp Leu Asp Arg Val Ala Cys Ile Met Asn Ser
           275                 280                 285
   Ser Gly Ser Thr Gly Leu Pro Lys Gly Val Pro Ile Ser His Arg Asn
       290                 295                 300
   Thr Ile Tyr Arg Phe Ser His Cys Arg Asp Pro Val Phe Gly Asn Gln
   305                 310                 315                 320
   Ile Ile Pro Asp Thr Thr Ile Leu Cys Ala Val Pro Phe His His Ala
                   325                 330                 335
   Phe Gly Thr Phe Thr Asn Leu Gly Tyr Leu Ile Cys Gly Phe His Val
               340                 345                 350
   Val Leu Met Tyr Arg Phe Asn Glu His Leu Phe Leu Gln Thr Leu Gln
           355                 360                 365
   Asp Tyr Lys Cys Gln Ser Ala Leu Leu Val Pro Thr Val Leu Ala Phe
       370                 375                 380
   Leu Ala Lys Asn Pro Leu Val Asp Lys Tyr Asp Leu Ser Asn Leu His
   385                 390                 395                 400
   Glu Ile Ala Ser Gly Gly Ala Pro Leu Ser Lys Glu Ile Ser Glu Ile
                   405                 410                 415
   Ala Ala Lys Arg Phe Lys Leu Pro Gly Ile Arg Gln Gly Tyr Gly Leu
               420                 425                 430
   Thr Glu Thr Thr Cys Ala Ile Val Ile Thr Ala Glu Gly Glu Phe Lys
           435                 440                 445
   Leu Gly Ala Val Gly Lys Val Val Pro Phe Tyr Ser Leu Lys Val Leu
       450                 455                 460
   Asp Leu Asn Thr Gly Lys Lys Leu Gly Pro Asn Glu Arg Gly Glu Ile
   465                 470                 475                 480
   Cys Phe Lys Gly Pro Met Ile Met Lys Gly Tyr Ile Asn Asn Pro Glu
                   485                 490                 495
   Ala Thr Arg Glu Leu Ile Asp Glu Glu Gly Trp Ile His Ser Gly Asp
               500                 505                 510
   Ile Gly Tyr Phe Asp Glu Asp Gly His Val Tyr Ile Val Asp Arg Leu
           515                 520                 525
   Lys Ser Leu Ile Lys Tyr Lys Gly Tyr Gln Val Pro Pro Ala Glu Leu
       530                 535                 540
   Glu Ala Leu Leu Leu Gln His Pro Phe Ile Glu Asp Ala Gly Val Ala
   545                 550                 555                 560
   Gly Val Pro Asp Glu Val Ala Gly Asp Leu Pro Gly Ala Val Val Val
                   565                 570                 575
   Leu Lys Glu Gly Lys Ser Ile Thr Glu Lys Glu Ile Gln Asp Tyr Val
               580                 585                 590
   Ala Gly Gln Val Thr Ser Ser Lys Lys Leu Arg Gly Gly Val Glu Phe
           595                 600                 605
   Val Lys Glu Val Pro Lys Gly Phe Thr Gly Lys Ile Asp Thr Arg Lys
       610                 615                 620
   Ile Lys Glu Ile Leu Ile Lys Ala Gln Lys Gly Lys Ser Lys Ser Lys
   625                 630                 635                 640
   Ala Lys Leu Gly Pro Glu Gln Lys Leu Ile Ser Glu Glu Asp Leu Asn
                   645                 650                 655
   Ser Ala Val Asp His His His His His His
               660                 665
(2) INFORMATION FOR SEQ ID NO:17:
   (i) SEQUENCE CHARACTERISTICS:
      (A) LENGTH: 621 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:17:
   Met Leu Thr Ala Ala Leu Leu Ser Cys Ala Leu Leu Leu Ala Leu Pro
    1               5                  10                  15
   Ala Thr Arg Gly Ala Gln Met Gly Leu Ala Pro Met Glu Gly Ile Arg
               20                  25                  30
   Arg Pro Asp Gln Ala Leu Leu Pro Glu Leu Pro Gly Leu Gly Leu Arg
           35                  40                  45
   Ala Pro Leu Lys Lys Thr Asn Ala Glu Gln Ala Glu Glu Asp Leu Leu
       50                  55                  60
   Gln Glu Ala Gln Ala Leu Ala Glu Val Leu Asp Leu Gln Asp Arg Glu
   65                  70                  75                  80
   Pro Arg Ser Ser Arg Arg Cys Val Arg Leu His Glu Ser Cys Leu Gly
                   85                  90                  95
   Gln Gln Val Pro Cys Cys Asp Pro Cys Ala Thr Cys Tyr Cys Arg Phe
               100                 105                 110
   Phe Asn Ala Phe Cys Tyr Cys Arg Lys Leu Gly Thr Ala Met Asn Pro
           115                 120                 125
   Cys Ser Arg Thr Ile Ile Pro Val Glu Glu Glu Asn Pro Asp Phe Trp
       130                 135                 140
   Asn Arg Gln Ala Ala Glu Ala Leu Gly Ala Ala Lys Lys Leu Gln Pro
   145                 150                 155                 160
   Ala Gln Thr Ala Ala Lys Asn Leu Ile Ile Phe Leu Gly Asp Gly Met
                   165                 170                 175
   Gly Val Ser Thr Val Thr Ala Ala Arg Ile Leu Lys Gly Gln Lys Lys
               180                 185                 190
   Asp Lys Leu Gly Pro Glu Thr Phe Leu Ala Met Asp Arg Phe Pro Tyr
           195                 200                 205
   Val Ala Leu Ser Lys Thr Tyr Ser Val Asp Lys His Val Pro Asp Ser
       210                 215                 220
   Gly Ala Thr Ala Thr Ala Tyr Leu Cys Gly Val Lys Gly Asn Phe Gln
   225                 230                 235                 240
   Thr Ile Gly Leu Ser Ala Ala Ala Arg Phe Asn Gln Cys Asn Thr Thr
                   245                 250                 255
   Arg Gly Asn Glu Val Ile Ser Val Met Asn Arg Ala Lys Lys Ala Gly
               260                 265                 270
   Lys Ser Val Gly Val Val Thr Thr Thr Arg Val Gln His Ala Ser Pro
           275                 280                 285
   Ala Gly Ala Tyr Ala His Thr Val Asn Arg Asn Trp Tyr Ser Asp Ala
       290                 295                 300
   Asp Val Pro Ala Ser Ala Arg Gln Glu Gly Cys Gln Asp Ile Ala Thr
   305                 310                 315                 320
   Gln Leu Ile Ser Asn Met Asp Ile Asp Val Ile Leu Gly Gly Gly Arg
                   325                 330                 335
   Lys Tyr Met Phe Pro Met Gly Thr Pro Asp Pro Glu Tyr Pro Asp Asp
               340                 345                 350
   Tyr Ser Gln Gly Gly Thr Arg Leu Asp Gly Lys Asn Leu Val Gln Glu
           355                 360                 365
   Trp Leu Ala Lys His Gln Gly Ala Arg Tyr Val Trp Asn Arg Thr Glu
       370                 375                 380
   Leu Met Gln Ala Ser Leu Asp Pro Ser Val Thr His Leu Met Gly Leu
   385                 390                 395                 400
   Phe Glu Pro Gly Asp Met Lys Tyr Glu Ile His Arg Asp Ser Thr Leu
                   405                 410                 415
   Asp Pro Ser Leu Met Glu Met Thr Glu Ala Ala Leu Arg Leu Leu Ser
               420                 425                 430
   Arg Asn Pro Arg Gly Phe Phe Leu Phe Val Glu Gly Gly Arg Ile Asp
           435                 440                 445
   His Gly His His Glu Ser Arg Ala Tyr Arg Ala Leu Thr Glu Thr Ile
       450                 455                 460
   Met Phe Asp Asp Ala Ile Glu Arg Ala Gly Gln Leu Thr Ser Glu Glu
   465                 470                 475                 480
   Asp Thr Leu Ser Leu Val Thr Ala Asp His Ser His Val Phe Ser Phe
                   485                 490                 495
   Gly Gly Tyr Pro Leu Arg Gly Ser Ser Ile Phe Gly Leu Ala Pro Gly
               500                 505                 510
   Lys Ala Arg Asp Arg Lys Ala Tyr Thr Val Leu Leu Tyr Gly Asn Gly
           515                 520                 525
   Pro Gly Tyr Val Leu Lys Asp Gly Ala Arg Pro Asp Val Thr Glu Ser
       530                 535                 540
   Glu Ser Gly Ser Pro Glu Tyr Arg Gln Gln Ser Ala Val Pro Leu Asp
   545                 550                 555                 560
   Gly Glu Thr His Ala Gly Glu Asp Val Ala Val Phe Ala Arg Gly Pro
                   565                 570                 575
   Gln Ala His Leu Val His Gly Val Gln Glu Gln Thr Phe Ile Ala His
               580                 585                 590
   Val Met Ala Phe Ala Ala Cys Leu Glu Pro Tyr Thr Ala Cys Asp Leu
           595                 600                 605
   Ala Pro Ser Ala Gly Thr Thr Asp Ala Ala His Pro Gly
       610                 615                 620
(2) INFORMATION FOR SEQ ID NO:18:
   (i) SEQUENCE CHARACTERISTICS:
      (A) LENGTH: 684 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:18:
   Met Leu Thr Ala Ala Leu Leu Ser Cys Ala Leu Leu Leu Ala Leu Pro
    1               5                  10                  15
   Ala Thr Arg Gly Ala Gln Met Gly Leu Ala Pro Met Glu Gly Ile Arg
               20                  25                  30
   Arg Pro Asp Gln Ala Leu Leu Pro Glu Leu Pro Gly Leu Gly Leu Arg
           35                  40                  45
   Ala Pro Leu Lys Lys Thr Asn Ala Glu Gln Ala Glu Glu Asp Leu Leu
       50                  55                  60
   Gln Glu Ala Gln Ala Leu Ala Glu Val Leu Asp Leu Gln Asp Arg Glu
   65                  70                  75                  80
   Pro Arg Ser Ser Arg Arg Cys Val Arg Leu His Glu Ser Cys Leu Gly
                   85                  90                  95
   Gln Gln Val Pro Cys Cys Asp Pro Cys Ala Thr Cys Tyr Cys Arg Phe
               100                 105                 110
   Phe Asn Ala Phe Cys Tyr Cys Arg Lys Leu Gly Thr Ala Met Asn Pro
           115                 120                 125
   Cys Ser Arg Thr Met Ser Ile Glu Asn Asn Ile Leu Ile Gly Pro Pro
       130                 135                 140
   Pro Tyr Tyr Pro Leu Glu Glu Gly Thr Ala Gly Glu Gln Leu His Arg
   145                 150                 155                 160
   Ala Ile Ser Arg Tyr Ala Ala Val Pro Gly Thr Leu Ala Tyr Thr Asp
                   165                 170                 175
   Val His Thr Glu Leu Glu Val Thr Tyr Lys Glu Phe Leu Asp Val Thr
               180                 185                 190
   Cys Arg Leu Ala Glu Ala Met Lys Asn Tyr Gly Leu Gly Leu Gln His
           195                 200                 205
   Thr Ile Ser Val Cys Ser Glu Asn Cys Val Gln Phe Phe Met Pro Ile
       210                 215                 220
   Cys Ala Ala Leu Tyr Val Gly Val Ala Thr Ala Pro Thr Asn Asp Ile
   225                 230                 235                 240
   Tyr Asn Glu Arg Glu Leu Tyr Asn Ser Leu Ser Ile Ser Gln Pro Thr
                   245                 250                 255
   Val Val Phe Thr Ser Arg Asn Ser Leu Gln Lys Ile Leu Gly Val Gln
               260                 265                 270
   Ser Arg Leu Pro Ile Ile Lys Lys Ile Ile Ile Leu Asp Gly Lys Lys
           275                 280                 285
   Asp Tyr Leu Gly Tyr Gln Ser Met Gln Ser Phe Met Lys Glu His Val
       290                 295                 300
   Pro Ala Asn Phe Asn Val Ser Ala Phe Lys Pro Leu Ser Phe Asp Leu
   305                 310                 315                 320
   Asp Arg Val Ala Cys Ile Met Asn Ser Ser Gly Ser Thr Gly Leu Pro
                   325                 330                 335
   Lys Gly Val Pro Ile Ser His Arg Asn Thr Ile Tyr Arg Phe Ser His
               340                 345                 350
   Cys Arg Asp Pro Val Phe Gly Asn Gln Ile Ile Pro Asp Thr Thr Ile
           355                 360                 365
   Leu Cys Ala Val Pro Phe His His Ala Phe Gly Thr Phe Thr Asn Leu
       370                 375                 380
   Gly Tyr Leu Ile Cys Gly Phe His Val Val Leu Met Tyr Arg Phe Asn
   385                 390                 395                 400
   Glu His Leu Phe Leu Gln Thr Leu Gln Asp Tyr Lys Cys Gln Ser Ala
                   405                 410                 415
   Leu Leu Val Pro Thr Val Leu Ala Phe Leu Ala Lys Asn Pro Leu Val
               420                 425                 430
   Asp Lys Tyr Asp Leu Ser Asn Leu His Glu Ile Ala Ser Gly Gly Ala
           435                 440                 445
   Pro Leu Ser Lys Glu Ile Ser Glu Ile Ala Ala Lys Arg Phe Lys Leu
       450                 455                 460
   Pro Gly Ile Arg Gln Gly Tyr Gly Leu Thr Glu Thr Thr Cys Ala Ile
   465                 470                 475                 480
   Val Ile Thr Ala Glu Gly Glu Phe Lys Leu Gly Ala Val Gly Lys Val
                   485                 490                 495
   Val Pro Phe Tyr Ser Leu Lys Val Leu Asp Leu Asn Thr Gly Lys Lys
               500                 505                 510
   Leu Gly Pro Asn Glu Arg Gly Glu Ile Cys Phe Lys Gly Pro Met Ile
           515                 520                 525
   Met Lys Gly Tyr Ile Asn Asn Pro Glu Ala Thr Arg Glu Leu Ile Asp
       530                 535                 540
   Glu Glu Gly Trp Ile His Ser Gly Asp Ile Gly Tyr Phe Asp Glu Asp
   545                 550                 555                 560
   Gly His Val Tyr Ile Val Asp Arg Leu Lys Ser Leu Ile Lys Tyr Lys
                   565                 570                 575
   Gly Tyr Gln Val Pro Pro Ala Glu Leu Glu Ala Leu Leu Leu Gln His
               580                 585                 590
   Pro Phe Ile Glu Asp Ala Gly Val Ala Gly Val Pro Asp Glu Val Ala
           595                 600                 605
   Gly Asp Leu Pro Gly Ala Val Val Val Leu Lys Glu Gly Lys Ser Ile
       610                 615                 620
   Thr Glu Lys Glu Ile Gln Asp Tyr Val Ala Gly Gln Val Thr Ser Ser
   625                 630                 635                 640
   Lys Lys Leu Arg Gly Gly Val Glu Phe Val Lys Glu Val Pro Lys Gly
                   645                 650                 655
   Phe Thr Gly Lys Ile Asp Thr Arg Lys Ile Lys Glu Ile Leu Ile Lys
               660                 665                 670
   Ala Gln Lys Gly Lys Ser Lys Ser Lys Ala Lys Leu
           675                 680
(2) INFORMATION FOR SEQ ID NO:19:
   (i) SEQUENCE CHARACTERISTICS:
      (A) LENGTH: 621 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:19:
   Met Leu Thr Ala Ala Leu Leu Ser Cys Ala Leu Leu Leu Ala Leu Pro
   1               5                   10                  15
   Ala Thr Arg Gly Ala Gln Met Gly Leu Ala Ile Ile Pro Val Glu Glu
               20                  25                  30
   Glu Asn Pro Asp Phe Trp Asn Arg Gln Ala Ala Glu Ala Leu Gly Ala
           35                  40                  45
   Ala Lys Lys Leu Gln Pro Ala Gln Thr Ala Ala Lys Asn Leu Ile Ile
       50                  55                  60
   Phe Leu Gly Asp Gly Met Gly Val Ser Thr Val Thr Ala Ala Arg Ile
   65                  70                  75                  80
   Leu Lys Gly Gln Lys Lys Asp Lys Leu Gly Pro Glu Thr Phe Leu Ala
                   85                  90                  95
   Met Asp Arg Phe Pro Tyr Val Ala Leu Ser Lys Thr Tyr Ser Val Asp
               100                 105                 110
   Lys His Val Pro Asp Ser Gly Ala Thr Ala Thr Ala Tyr Leu Cys Gly
           115                 120                 125
   Val Lys Gly Asn Phe Gln Thr Ile Gly Leu Ser Ala Ala Ala Arg Phe
       130                 135                 140
   Asn Gln Cys Asn Thr Thr Arg Gly Asn Glu Val Ile Ser Val Met Asn
   145                 150                 155                 160
   Arg Ala Lys Lys Ala Gly Lys Ser Val Gly Val Val Thr Thr Thr Arg
                   165                 170                 175
   Val Gln His Ala Ser Pro Ala Gly Ala Tyr Ala His Thr Val Asn Arg
                   180                 185                 190
   Asn Trp Tyr Ser Asp Ala Asp Val Pro Ala Ser Ala Arg Gln Glu Gly
               195                 200                 205
   Cys Gln Asp Ile Ala Thr Gln Leu Ile Ser Asn Met Asp Ile Asp Val
       210                 215                 220
   Ile Leu Gly Gly Gly Arg Lys Tyr Met Phe Pro Met Gly Thr Pro Asp
   225                 230                 235                 240
   Pro Glu Tyr Pro Asp Asp Tyr Ser Gln Gly Gly Thr Arg Leu Asp Gly
                   245                 250                 255
   Lys Asn Leu Val Gln Glu Trp Leu Ala Lys His Gln Gly Ala Arg Tyr
               260                 265                 270
   Val Trp Asn Arg Thr Glu Leu Met Gln Ala Ser Leu Asp Pro Ser Val
           275                 280                 285
   Thr His Leu Met Gly Leu Phe Glu Pro Gly Asp Met Lys Tyr Glu Ile
       290                 295                 300
   His Arg Asp Ser Thr Leu Asp Pro Ser Leu Met Glu Met Thr Glu Ala
   305                 310                 315                 320
   Ala Leu Arg Leu Leu Ser Arg Asn Pro Arg Gly Phe Phe Leu Phe Val
                   325                 330                 335
   Glu Gly Gly Arg Ile Asp His Gly His His Glu Ser Arg Ala Tyr Arg
               340                 345                 350
   Ala Leu Thr Glu Thr Ile Met Phe Asp Asp Ala Ile Glu Arg Ala Gly
           355                 360                 365
   Gln Leu Thr Ser Glu Glu Asp Thr Leu Ser Leu Val Thr Ala Asp His
       370                 375                 380
   Ser His Val Phe Ser Phe Gly Gly Tyr Pro Leu Arg Gly Ser Ser Ile
   385                 390                 395                 400
   Phe Gly Leu Ala Pro Gly Lys Ala Arg Asp Arg Lys Ala Tyr Thr Val
                   405                 410                 415
   Leu Leu Tyr Gly Asn Gly Pro Gly Tyr Val Leu Lys Asp Gly Ala Arg
               420                 425                 430
   Pro Asp Val Thr Glu Ser Glu Ser Gly Ser Pro Glu Tyr Arg Gln Gln
           435                 440                 445
   Ser Ala Val Pro Leu Asp Gly Glu Thr His Ala Gly Glu Asp Val Ala
       450                 455                 460
   Val Phe Ala Arg Gly Pro Gln Ala His Leu Val His Gly Val Gln Glu
   465                 470                 475                 480
   Gln Thr Phe Ile Ala His Val Met Ala Phe Ala Ala Cys Leu Glu Pro
                   485                 490                 495
   Tyr Thr Ala Cys Asp Leu Ala Pro Ser Ala Gly Thr Thr Asp Ala Ala
               500                 505                 510
   His Pro Gly Pro Met Glu Gly Ile Arg Arg Pro Asp Gln Ala Leu Leu
           515                 520                 525
   Pro Glu Leu Pro Gly Leu Gly Leu Arg Ala Pro Leu Lys Lys Thr Asn
       530                 535                 540
   Ala Glu Gln Ala Glu Glu Asp Leu Leu Gln Glu Ala Gln Ala Leu Ala
   545                 550                 555                 560
   Glu Val Leu Asp Leu Gln Asp Arg Glu Pro Arg Ser Ser Arg Arg Cys
                   565                 570                 575
   Val Arg Leu His Glu Ser Cys Leu Gly Gln Gln Val Pro Cys Cys Asp
               580                 585                 590
   Pro Cys Ala Thr Cys Tyr Cys Arg Phe Phe Asn Ala Phe Cys Tyr Cys
           595                 600                 605
   Arg Lys Leu Gly Thr Ala Met Asn Pro Cys Ser Arg Thr
       610                 615                 620
(2) INFORMATION FOR SEQ ID NO:20:
   (i) SEQUENCE CHARACTERISTICS:
      (A) LENGTH: 684 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:20:
   Met Leu Thr Ala Ala Leu Leu Ser Cys Ala Leu Leu Leu Ala Leu Pro
    1               5                  10                  15
   Ala Thr Arg Gly Ala Gln Met Gly Leu Ala Met Ser Ile Glu Asn Asn
               20                  25                  30
   Ile Leu Ile Gly Pro Pro Pro Tyr Tyr Pro Leu Glu Glu Gly Thr Ala
           35                  40                  45
   Gly Glu Gln Leu His Arg Ala Ile Ser Arg Tyr Ala Ala Val Pro Gly
       50                  55                  60
   Thr Leu Ala Tyr Thr Asp Val His Thr Glu Leu Glu Val Thr Tyr Lys
   65                  70                  75                  80
   Glu Phe Leu Asp Val Thr Cys Arg Leu Ala Glu Ala Met Lys Asn Tyr
                   85                  90                  95
   Gly Leu Gly Leu Gln His Thr Ile Ser Val Cys Ser Glu Asn Cys Val
               100                 105                 110
   Gln Phe Phe Met Pro Ile Cys Ala Ala Leu Tyr Val Gly Val Ala Thr
           115                 120                 125
   Ala Pro Thr Asn Asp Ile Tyr Asn Glu Arg Glu Leu Tyr Asn Ser Leu
       130                 135                 140
   Ser Ile Ser Gln Pro Thr Val Val Phe Thr Ser Arg Asn Ser Leu Gln
   145                 150                 155                 160
   Lys Ile Leu Gly Val Gln Ser Arg Leu Pro Ile Ile Lys Lys Ile Ile
                   165                 170                 175
   Ile Leu Asp Gly Lys Lys Asp Tyr Leu Gly Tyr Gln Ser Met Gln Ser
               180                 185                 190
   Phe Met Lys Glu His Val Pro Ala Asn Phe Asn Val Ser Ala Phe Lys
           195                 200                 205
   Pro Leu Ser Phe Asp Leu Asp Arg Val Ala Cys Ile Met Asn Ser Ser
       210                 215                 220
   Gly Ser Thr Gly Leu Pro Lys Gly Val Pro Ile Ser His Arg Asn Thr
   225                 230                 235                 240
   Ile Tyr Arg Phe Ser His Cys Arg Asp Pro Val Phe Gly Asn Gln Ile
                   245                 250                 255
   Ile Pro Asp Thr Thr Ile Leu Cys Ala Val Pro Phe His His Ala Phe
               260                 265                 270
   Gly Thr Phe Thr Asn Leu Gly Tyr Leu Ile Cys Gly Phe His Val Val
           275                 280                 285
   Leu Met Tyr Arg Phe Asn Glu His Leu Phe Leu Gln Thr Leu Gln Asp
       290                 295                 300
   Tyr Lys Cys Gln Ser Ala Leu Leu Val Pro Thr Val Leu Ala Phe Leu
   305                 310                 315                 320
   Ala Lys Asn Pro Leu Val Asp Lys Tyr Asp Leu Ser Asn Leu His Glu
                   325                 330                 335
   Ile Ala Ser Gly Gly Ala Pro Leu Ser Lys Glu Ile Ser Glu Ile Ala
               340                 345                 350
   Ala Lys Arg Phe Lys Leu Pro Gly Ile Arg Gln Gly Tyr Gly Leu Thr
           355                 360                 365
   Glu Thr Thr Cys Ala Ile Val Ile Thr Ala Glu Gly Glu Phe Lys Leu
       370                 375                 380
   Gly Ala Val Gly Lys Val Val Pro Phe Tyr Ser Leu Lys Val Leu Asp
   385                 390                 395                 400
   Leu Asn Thr Gly Lys Lys Leu Gly Pro Asn Glu Arg Gly Glu Ile Cys
                   405                 410                 415
   Phe Lys Gly Pro Met Ile Met Lys Gly Tyr Ile Asn Asn Pro Glu Ala
               420                 425                 430
   Thr Arg Glu Leu Ile Asp Glu Glu Gly Trp Ile His Ser Gly Asp Ile
           435                 440                 445
   Gly Tyr Phe Asp Glu Asp Gly His Val Tyr Ile Val Asp Arg Leu Lys
       450                 455                 460
   Ser Leu Ile Lys Tyr Lys Gly Tyr Gln Val Pro Pro Ala Glu Leu Glu
   465                 470                 475                 480
   Ala Leu Leu Leu Gln His Pro Phe Ile Glu Asp Ala Gly Val Ala Gly
                   485                 490                 495
   Val Pro Asp Glu Val Ala Gly Asp Leu Pro Gly Ala Val Val Val Leu
               500                 505                 510
   Lys Glu Gly Lys Ser Ile Thr Glu Lys Glu Ile Gln Asp Tyr Val Ala
           515                 520                 525
   Gly Gln Val Thr Ser Ser Lys Lys Leu Arg Gly Gly Val Glu Phe Val
       530                 535                 540
   Lys Glu Val Pro Lys Gly Phe Thr Gly Lys Ile Asp Thr Arg Lys Ile
   545                 550                 555                 560
   Lys Glu Ile Leu Ile Lys Ala Gln Lys Gly Lys Ser Lys Ser Lys Ala
                   565                 570                 575
   Lys Leu Pro Met Glu Gly Ile Arg Arg Pro Asp Gln Ala Leu Leu Pro
               580                 585                 590
   Glu Leu Pro Gly Leu Gly Leu Arg Ala Pro Leu Lys Lys Thr Asn Ala
           595                 600                 605
   Glu Gln Ala Glu Glu Asp Leu Leu Gln Glu Ala Gln Ala Leu Ala Glu
       610                 615                 620
   Val Leu Asp Leu Gln Asp Arg Glu Pro Arg Ser Ser Arg Arg Cys Val
   625                 630                 635                 640
   Arg Leu His Glu Ser Cys Leu Gly Gln Gln Val Pro Cys Cys Asp Pro
                   645                 650                 655
   Cys Ala Thr Cys Tyr Cys Arg Phe Phe Asn Ala Phe Cys Tyr Cys Arg
               660                 665                 670
   Lys Leu Gly Thr Ala Met Asn Pro Cys Ser Arg Thr
           675                 680
(2) INFORMATION FOR SEQ ID NO:21:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:21:
      GGGCTCGGCG GTCCTGCAGG GCCAAGCCCA TCTGGGC 37
(2) INFORMATION FOR SEQ ID NO:22:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:22:
      TAATACGACT CACTATAGGG 20
(2) INFORMATION FOR SEQ ID NO:23:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:23:
      CTGCAGGACC GCGAGCCC 18
(2) INFORMATION FOR SEQ ID NO:24:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:24:
      GTCGACGGCG CTATTCAG 18

### SEQUENCE LISTING

(1) GENERAL INFORMATION:
   (i) APPLICANT: Merck & Co., Inc.
   (ii) TITLE OF INVENTION: C-TERMINAL REGION OF AGOUTI-RELATED TRANSCRIPT (ART) PROTEIN
   (iii) NUMBER OF SEQUENCES: 24
   (iv) CORRESPONDENCE ADDRESS:
      (A) ADDRESSEE: Merck & Co., Inc.
      (B) STREET: P.O. Box 2000, 126 E. Lincoln Ave.
      (C) CITY: Rahway
      (D) STATE: NJ
      (E) COUNTRY: USA
      (F) ZIP: 07065-0900
   (v) COMPUTER READABLE FORM:
      (A) MEDIUM TYPE: Diskette
      (B) COMPUTER: IBM Compatible
      (C) OPERATING SYSTEM: Windows
      (D) SOFTWARE: FastSEQ for Windows Version 2.0b
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: 60/069,747
      (B) FILING DATE: 16-DEC-1997
   (viii) ATTORNEY/AGENT INFORMATION:
      (A) NAME: Coppola, Joseph A
      (B) REGISTRATION NUMBER: 38,413
      (C) REFERENCE/DOCKET NUMBER: 20146 PCT
   (ix) TELECOMMUNICATION INFORMATION:
      (A) TELEPHONE: 732-594-6734
      (B) TELEFAX: 732-594-4720
      (C) TELEX:
(2) INFORMATION FOR SEQ ID NO:1:
   (i) SEQUENCE CHARACTERISTICS:
      (A) LENGTH: 118 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:1:
   Met Asn Ile Phe Tyr Ile Phe Leu Phe Leu Leu Ser Phe Val Gln Gly
    1               5                  10                  15
   Leu Glu His Thr His Arg Arg Gly Ser Leu Val Lys Arg Ser Ser Leu
                       20                      25                      30
   Gln Asp Arg Glu Pro Arg Ser Ser Arg Arg Cys Val Arg Leu His Glu
                   35                      40                      45
   Ser Cys Leu Gly Gln Gln Val Pro Cys Cys Asp Pro Cys Ala Thr Cys
               50                      55                      60
   Tyr Cys Arg Phe Phe Asn Ala Phe Cys Tyr Cys Arg Lys Leu Gly Thr
   65                      70                      75                      80
   Ala Met Asn Pro Cys Ser Arg Thr Leu Val Pro Arg Gly Ser Glu Gln
                       85                      90                      95
   Lys Leu Ile Ser Glu Glu Asp Leu Asn Leu Arg Arg Ala Ser Leu Gly
                   100                     105                     110
   His His His His His His
                   115
(2) INFORMATION FOR SEQ ID NO:2:
   (i) SEQUENCE CHARACTERISTICS:
      (A) LENGTH: 120 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:2:
   Met Leu Thr Ala Ala Leu Leu Ser Cys Ala Leu Leu Leu Ala Leu Pro
    1               5                  10                  15
   Ala Thr Arg Gly Ala Gln Met Gly Leu Ala Leu Gln Asp Arg Glu Pro
               20                  25                  30
   Arg Ser Ser Arg Arg Cys Val Arg Leu His Glu Ser Cys Leu Gly Gln
           35                  40                  45
   Gln Val Pro Cys Cys Asp Pro Cys Ala Thr Cys Tyr Cys Arg Phe Phe
       50                  55                  60
   Asn Ala Phe Cys Tyr Cys Arg Lys Leu Gly Thr Ala Met Asn Pro Cys
   65                  70                  75                  80
   Ser Arg Thr Leu Val Pro Arg Gly Ser Gly Ser Glu Leu Gly Thr Lys
                   85                  90                  95
   Leu Gly Pro Glu Gln Lys Leu Ile Ser Glu Glu Asp Leu Asn Ser Ala
               100                 105                 110
   Val Asp His His His His His His
           115                 120
(2) INFORMATION FOR SEQ ID NO:3:
   (i) SEQUENCE CHARACTERISTICS:
      (A) LENGTH: 121 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:3:
   Met Leu Thr Ala Ala Leu Leu Ser Cys Ala Leu Leu Leu Ala Leu Pro
    1               5                  10                  15
   Ala Thr Arg Gly Ala Gln Met Gly Leu Ala Leu Gln Asp Arg Glu Pro
               20                  25                  30
   Arg Ser Ser Arg Arg Cys Val Arg Leu His Glu Ser Cys Leu Gly Gln
           35                  40                  45
   Gln Val Pro Cys Cys Asp Pro Cys Ala Thr Cys Tyr Cys Arg Phe Phe
        50                  55                  60
   Asn Ala Phe Cys Tyr Cys Arg Lys Leu Gly Thr Ala Met Asn Pro Cys
   65                  70                  75                  80
   Ser Arg Thr Leu Val Pro Arg Gly Ser Gly Ser Leu Arg Arg Ala Ser
                   85                  90                  95
   Leu Gly Lys Leu Glu Gln Lys Leu Ile Ser Glu Glu Asp Leu Asn Ser
               100                 105                 110
   Ala Val Asp His His His His His His
           115                 120
(2) INFORMATION FOR SEQ ID NO:4:
   (i) SEQUENCE CHARACTERISTICS:
      (A) LENGTH: 654 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:4:
(2) INFORMATION FOR SEQ ID NO:5:
   (i) SEQUENCE CHARACTERISTICS:
      (A) LENGTH: 715 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:5:
   Met Leu Thr Ala Ala Leu Leu Ser Cys Ala Leu Leu Leu Ala Leu Pro
   1               5                   10                  15
   Ala Thr Arg Gly Ala Gln Met Gly Leu Ala Pro Met Glu Gly Ile Arg
               20                  25                  30
   Arg Pro Asp Gln Ala Leu Leu Pro Glu Leu Pro Gly Leu Gly Leu Arg
           35                  40                  45
   Ala Pro Leu Lys Lys Thr Asn Ala Glu Gln Ala Glu Glu Asp Leu Leu
       50                  55                  60
   Gln Glu Ala Gln Ala Leu Ala Glu Val Leu Asp Leu Gln Asp Arg Glu
   65                  70                  75                  80
   Pro Arg Ser Ser Arg Arg Cys Val Arg Leu His Glu Ser Cys Leu Gly
                   85                  90                  95
   Gln Gln Val Pro Cys Cys Asp Pro Cys Ala Thr Cys Tyr Cys Arg Phe
               100                 105                 110
   Phe Asn Ala Phe Cys Tyr Cys Arg Lys Leu Gly Thr Ala Met Asn Pro
           115                 120                 125
   Cys Ser Arg Thr Leu Val Pro Arg Gly Ser Gly Ser Met Ser Ile Glu
       130                 135                 140
   Asn Asn Ile Leu Ile Gly Pro Pro Pro Tyr Tyr Pro Leu Glu Glu Gly
   145                 150                 155                 160
   Thr Ala Gly Glu Gln Leu His Arg Ala Ile Ser Arg Tyr Ala Ala Val
                   165                 170                 175
   Pro Gly Thr Leu Ala Tyr Thr Asp Val His Thr Glu Leu Glu Val Thr
               180                 185                 190
   Tyr Lys Glu Phe Leu Asp Val Thr Cys Arg Leu Ala Glu Ala Met Lys
           195                 200                 205
   Asn Tyr Gly Leu Gly Leu Gln His Thr Ile Ser Val Cys Ser Glu Asn
       210                 215                 220
   Cys Val Gln Phe Phe Met Pro Ile Cys Ala Ala Leu Tyr Val Gly Val
   225                 230                 235                 240
   Ala Thr Ala Pro Thr Asn Asp Ile Tyr Asn Glu Arg Glu Leu Tyr Asn
                   245                 250                 255
   Ser Leu Ser Ile Ser Gln Pro Thr Val Val Phe Thr Ser Arg Asn Ser
               260                 265                 270
   Leu Gln Lys Ile Leu Gly Val Gln Ser Arg Leu Pro Ile Ile Lys Lys
           275                 280                 285
   Ile Ile Ile Leu Asp Gly Lys Lys Asp Tyr Leu Gly Tyr Gln Ser Met
       290                 295                 300
   Gln Ser Phe Met Lys Glu His Val Pro Ala Asn Phe Asn Val Ser Ala
   305                 310                 315                 320
   Phe Lys Pro Leu Ser Phe Asp Leu Asp Arg Val Ala Cys Ile Met Asn
                   325                 330                 335
   Ser Ser Gly Ser Thr Gly Leu Pro Lys Gly Val Pro Ile Ser His Arg
               340                 345                 350
   Asn Thr Ile Tyr Arg Phe Ser His Cys Arg Asp Pro Val Phe Gly Asn
           355                 360                 365
   Gln Ile Ile Pro Asp Thr Thr Ile Leu Cys Ala Val Pro Phe His His
       370                 375                 380
   Ala Phe Gly Thr Phe Thr Asn Leu Gly Tyr Leu Ile Cys Gly Phe His
   385                 390                 395                 400
   Val Val Leu Met Tyr Arg Phe Asn Glu His Leu Phe Leu Gln Thr Leu
                   405                 410                 415
   Gln Asp Tyr Lys Cys Gln Ser Ala Leu Leu Val Pro Thr Val Leu Ala
               420                 425                 430
   Phe Leu Ala Lys Asn Pro Leu Val Asp Lys Tyr Asp Leu Ser Asn Leu
           435                 440                 445
   His Glu Ile Ala Ser Gly Gly Ala Pro Leu Ser Lys Glu Ile Ser Glu
       450                 455                 460
   Ile Ala Ala Lys Arg Phe Lys Leu Pro Gly Ile Arg Gln Gly Tyr Gly
   465                 470                 475                 480
   Leu Thr Glu Thr Thr Cys Ala Ile Val Ile Thr Ala Glu Gly Glu Phe
                   485                 490                 495
   Lys Leu Gly Ala Val Gly Lys Val Val Pro Phe Tyr Ser Leu Lys Val
               500                 505                 510
   Leu Asp Leu Asn Thr Gly Lys Lys Leu Gly Pro Asn Glu Arg Gly Glu
           515                 520                 525
   Ile Cys Phe Lys Gly Pro Met Ile Met Lys Gly Tyr Ile Asn Asn Pro
       530                 535                 540
   Glu Ala Thr Arg Glu Leu Ile Asp Glu Glu Gly Trp Ile His Ser Gly
   545                 550                 555                 560
   Asp Ile Gly Tyr Phe Asp Glu Asp Gly His Val Tyr Ile Val Asp Arg
                   565                 570                 575
   Leu Lys Ser Leu Ile Lys Tyr Lys Gly Tyr Gln Val Pro Pro Ala Glu
               580                 585                 590
   Leu Glu Ala Leu Leu Leu Gln His Pro Phe Ile Glu Asp Ala Gly Val
           595                 600                 605
   Ala Gly Val Pro Asp Glu Val Ala Gly Asp Leu Pro Gly Ala Val Val
       610                 615                 620
   Val Leu Lys Glu Gly Lys Ser Ile Thr Glu Lys Glu Ile Gln Asp Tyr
   625                 630                 635                 640
   Val Ala Gly Gln Val Thr Ser Ser Lys Lys Leu Arg Gly Gly Val Glu
                   645                 650                 655
   Phe Val Lys Glu Val Pro Lys Gly Phe Thr Gly Lys Ile Asp Thr Arg
               660                 665                 670
   Lys Ile Lys Glu Ile Leu Ile Lys Ala Gln Lys Gly Lys Ser Lys Ser
           675                 680                 685
   Lys Ala Lys Leu Gly Pro Glu Gln Lys Leu Ile Ser Glu Glu Asp Leu
       690                 695                 700
   Asn Ser Ala Val Asp His His His His His His
   705                 710                 715
(2) INFORMATION FOR SEQ ID NO:6:
   (i) SEQUENCE CHARACTERISTICS:
      (A) LENGTH: 83 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:6:
   Met Leu Thr Ala Ala Leu Leu Ser Cys Ala Leu Leu Leu Ala Leu Pro
    1               5                  10                  15
   Ala Thr Arg Gly Ala Gln Met Gly Leu Ala Leu Gln Asp Arg Glu Pro
               20                  25                  30
   Arg Ser Ser Arg Arg Cys Val Arg Leu His Glu Ser Cys Leu Gly Gln
           35                  40                  45
   Gln Val Pro Cys Cys Asp Pro Cys Ala Thr Cys Tyr Cys Arg Phe Phe
       50                  55                  60
   Asn Ala Phe Cys Tyr Cys Arg Lys Leu Gly Thr Ala Met Asn Pro Cys
   65                  70                  75                  80
   Ser Arg Thr
(2) INFORMATION FOR SEQ ID NO:7:
   (i) SEQUENCE CHARACTERISTICS:
      (A) LENGTH: 57 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:7:
   Leu Gln Asp Arg Glu Pro Arg Ser Ser Arg Arg Cys Val Arg Leu His
    1               5                  10                  15
   Glu Ser Cys Leu Gly Gln Gln Val Pro Cys Cys Asp Pro Cys Ala Thr
               20                  25                  30
   Cys Tyr Cys Arg Phe Phe Asn Ala Phe Cys Tyr Cys Arg Lys Leu Gly
           35                  40                  45
   Thr Ala Met Asn Pro Cys Ser Arg Thr
       50                  55
(2) INFORMATION FOR SEQ ID NO:8:
   (i) SEQUENCE CHARACTERISTICS:
      (A) LENGTH: 83 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:8:
   Met Leu Thr Ala Met Leu Leu Ser Cys Val Leu Leu Leu Ala Leu Pro
    1               5                  10                  15
   Pro Thr Leu Gly Val Gln Met Gly Val Ala Pro Gln Asn Arg Glu Ser
               20                  25                  30
   Arg Ser Pro Arg Arg Cys Val Arg Leu His Glu Ser Cys Leu Gly Gln
           35                  40                  45
   Gln Val Pro Cys Cys Asp Pro Cys Ala Thr Cys Tyr Cys Arg Phe Phe
       50                  55                  60
   Asn Ala Phe Cys Tyr Cys Arg Lys Leu Gly Thr Ala Met Asn Leu Cys
   65                  70                  75                  80
   Ser Arg Thr
(2) INFORMATION FOR SEQ ID NO:9:
   (i) SEQUENCE CHARACTERISTICS:
      (A) LENGTH: 57 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:9:
   Pro Gln Asn Arg Glu Ser Arg Ser Pro Arg Arg Cys Val Arg Leu His
    1               5                  10                  15
   Glu Ser Cys Leu Gly Gln Gln Val Pro Cys Cys Asp Pro Cys Ala Thr
               20                  25                  30
   Cys Tyr Cys Arg Phe Phe Asn Ala Phe Cys Tyr Cys Arg Lys Leu Gly
           35                  40                  45
   Thr Ala Met Asn Leu Cys Ser Arg Thr
       50                  55
(2) INFORMATION FOR SEQ ID NO:10:
   (i) SEQUENCE CHARACTERISTICS:
      (A) LENGTH: 118 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:10:
   Met Asn Ile Phe Tyr Ile Phe Leu Phe Leu Leu Ser Phe Val Gln Gly
    1               5                  10                  15
   Leu Glu His Thr His Arg Arg Gly Ser Leu Val Lys Arg Ser Ser Pro
               20                  25                  30
   Gln Asn Arg Glu Ser Arg Ser Pro Arg Arg Cys Val Arg Leu His Glu
           35                  40                  45
   Ser Cys Leu Gly Gln Gln Val Pro Cys Cys Asp Pro Cys Ala Thr Cys
       50                  55                  60
   Tyr Cys Arg Phe Phe Asn Ala Phe Cys Tyr Cys Arg Lys Leu Gly Thr
   65                  70                  75                  80
   Ala Met Asn Leu Cys Ser Arg Thr Leu Val Pro Arg Gly Ser Glu Gln
                   85                  90                  95
   Lys Leu Ile Ser Glu Glu Asp Leu Asn Leu Arg Arg Ala Ser Leu Gly
               100                 105                 110
   His His His His His His
           115
(2) INFORMATION FOR SEQ ID NO:11:
   (i) SEQUENCE CHARACTERISTICS:
      (A) LENGTH: 113 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:11:
   Met Leu Thr Ala Met Leu Leu Ser Cys Val Leu Leu Leu Ala Leu Pro
    1               5                  10                  15
   Pro Thr Leu Gly Val Gln Met Gly Val Ala Pro Gln Asn Arg Glu Ser
               20                  25                  30
   Arg Ser Pro Arg Arg Cys Val Arg Leu His Glu Ser Cys Leu Gly Gln
           35                  40                  45
   Gln Val Pro Cys Cys Asp Pro Cys Ala Thr Cys Tyr Cys Arg Phe Phe
       50                  55                  60
   Asn Ala Phe Cys Tyr Cys Arg Lys Leu Gly Thr Ala Met Asn Leu Cys
   65                  70                  75                  80
   Ser Arg Thr Leu Val Pro Arg Gly Ser Glu Gln Lys Leu Ile Ser Glu
                   85                  90                  95
   Glu Asp Leu Asn Leu Arg Arg Ala Ser Leu Ser His His His His His
               100                 105                 110
   His
(2) INFORMATION FOR SEQ ID NO:12:
   (i) SEQUENCE CHARACTERISTICS:
      (A) LENGTH: 117 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:12:
   Met Leu Thr Ala Met Leu Leu Ser Cys Val Leu Leu Leu Ala Leu Pro
    1               5                  10                  15
   Pro Thr Leu Gly Val Gln Met Gly Val Ala Pro Gln Asn Arg Glu Ser
               20                  25                  30
   Arg Ser Pro Arg Arg Cys Val Arg Leu His Glu Ser Cys Leu Gly Gln
           35                  40                  45
   Gln Val Pro Cys Cys Asp Pro Cys Ala Thr Cys Tyr Cys Arg Phe Phe
       50                  55                  60
   Asn Ala Phe Cys Tyr Cys Arg Lys Leu Gly Thr Ala Met Asn Leu Cys
   65                  70                  75                  80
   Ser Arg Thr Leu Val Pro Arg Gly Ser Gly Ser Leu Arg Arg Ala Ser
                   85                  90                  95
   Leu Gly Lys Leu Glu Gln Lys Leu Ile Ser Glu Glu Asp Leu Asn His
               100                 105                 110
   His His His His His
           115
(2) INFORMATION FOR SEQ ID NO:13:
   (i) SEQUENCE CHARACTERISTICS:
      (A) LENGTH: 620 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:13:
   Met Leu Thr Ala Met Leu Leu Ser Cys Val Leu Leu Leu Ala Leu Pro
    1               5                  10                  15
   Pro Thr Leu Gly Val Gln Met Gly Val Ala Pro Leu Lys Gly Ile Arg
               20                  25                  30
   Arg Pro Asp Gln Ala Leu Phe Pro Glu Phe Pro Gly Leu Ser Leu Asn
           35                  40                  45
   Gly Leu Lys Lys Thr Asn Ala Asp Arg Ala Glu Glu Val Leu Leu Gln
       50                  55                  60
   Lys Ala Glu Ala Leu Ala Glu Val Leu Asp Pro Gln Asn Arg Glu Ser
   65                  70                  75                  80
   Arg Ser Pro Arg Arg Cys Val Arg Leu His Glu Ser Cys Leu Gly Gln
                   85                  90                  95
   Gln Val Pro Cys Cys Asp Pro Cys Ala Thr Cys Tyr Cys Arg Phe Phe
               100                 105                 110
   Asn Ala Phe Cys Tyr Cys Arg Lys Leu Gly Thr Ala Met Asn Leu Cys
           115                 120                 125
   Ser Arg Thr Ile Ile Pro Val Glu Glu Glu Asn Pro Asp Phe Trp Asn
       130                 135                 140
   Arg Gln Ala Ala Glu Ala Leu Gly Ala Ala Lys Lys Leu Gln Pro Ala
   145                 150                 155                 160
   Gln Thr Ala Ala Lys Asn Leu Ile Ile Phe Leu Gly Asp Gly Met Gly
                   165                 170                 175
   Val Ser Thr Val Thr Ala Ala Arg Ile Leu Lys Gly Gln Lys Lys Asp
               180                 185                 190
   Lys Leu Gly Pro Glu Thr Phe Leu Ala Met Asp Arg Phe Pro Tyr Val
           195                 200                 205
   Ala Leu Ser Lys Thr Tyr Ser Val Asp Lys His Val Pro Asp Ser Gly
       210                 215                 220
   Ala Thr Ala Thr Ala Tyr Leu Cys Gly Val Lys Gly Asn Phe Gln Thr
   225                 230                 235                 240
   Ile Gly Leu Ser Ala Ala Ala Arg Phe Asn Gln Cys Asn Thr Thr Arg
                   245                 250                 255
   Gly Asn Glu Val Ile Ser Val Met Asn Arg Ala Lys Lys Ala Gly Lys
               260                 265                 270
   Ser Val Gly Val Val Thr Thr Thr Arg Val Gln His Ala Ser Pro Ala
           275                 280                 285
   Gly Ala Tyr Ala His Thr Val Asn Arg Asn Trp Tyr Ser Asp Ala Asp
       290                 295                 300
   Val Pro Ala Ser Ala Arg Gln Glu Gly Cys Gln Asp Ile Ala Thr Gln
   305                 310                 315                 320
   Leu Ile Ser Asn Met Asp Ile Asp Val Ile Leu Gly Gly Gly Arg Lys
                   325                 330                 335
   Tyr Met Phe Pro Met Gly Thr Pro Asp Pro Glu Tyr Pro Asp Asp Tyr
               340                 345                 350
   Ser Gln Gly Gly Thr Arg Leu Asp Gly Lys Asn Leu Val Gln Glu Trp
           355                 360                 365
   Leu Ala Lys His Gln Gly Ala Arg Tyr Val Trp Asn Arg Thr Glu Leu
       370                 375                 380
   Met Gln Ala Ser Leu Asp Pro Ser Val Thr His Leu Met Gly Leu Phe
   385                 390                 395                 400
   Glu Pro Gly Asp Met Lys Tyr Glu Ile His Arg Asp Ser Thr Leu Asp
                   405                 410                 415
   Pro Ser Leu Met Glu Met Thr Glu Ala Ala Leu Arg Leu Leu Ser Arg
               420                 425                 430
   Asn Pro Arg Gly Phe Phe Leu Phe Val Glu Gly Gly Arg Ile Asp His
           435                 440                 445
   Gly His His Glu Ser Arg Ala Tyr Arg Ala Leu Thr Glu Thr Ile Met
       450                 455                 460
   Phe Asp Asp Ala Ile Glu Arg Ala Gly Gln Leu Thr Ser Glu Glu Asp
   465                 470                 475                 480
   Thr Leu Ser Leu Val Thr Ala Asp His Ser His Val Phe Ser Phe Gly
                   485                 490                 495
   Gly Tyr Pro Leu Arg Gly Ser Ser Ile Phe Gly Leu Ala Pro Gly Lys
               500                 505                 510
   Ala Arg Asp Arg Lys Ala Tyr Thr Val Leu Leu Tyr Gly Asn Gly Pro
           515                 520                 525
   Gly Tyr Val Leu Lys Asp Gly Ala Arg Pro Asp Val Thr Glu Ser Glu
       530                 535                 540
   Ser Gly Ser Pro Glu Tyr Arg Gln Gln Ser Ala Val Pro Leu Asp Gly
   545                 550                 555                 560
   Glu Thr His Ala Gly Glu Asp Val Ala Val Phe Ala Arg Gly Pro Gln
                   565                 570                 575
   Ala His Leu Val His Gly Val Gln Glu Gln Thr Phe Ile Ala His Val
               580                 585                 590
   Met Ala Phe Ala Ala Cys Leu Glu Pro Tyr Thr Ala Cys Asp Leu Ala
           595                 600                 605
   Pro Ser Ala Gly Thr Thr Asp Ala Ala His Pro Gly
       610                 615                 620
(2) INFORMATION FOR SEQ ID NO:14:
   (i) SEQUENCE CHARACTERISTICS:
      (A) LENGTH: 683 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:14:
   Met Leu Thr Ala Met Leu Leu Ser Cys Val Leu Leu Leu Ala Leu Pro
    1               5                  10                  15
   Pro Thr Leu Gly Val Gln Met Gly Val Ala Pro Leu Lys Gly Ile Arg
               20                  25                  30
   Arg Pro Asp Gln Ala Leu Phe Pro Glu Phe Pro Gly Leu Ser Leu Asn
           35                  40                  45
   Gly Leu Lys Lys Thr Asn Ala Asp Arg Ala Glu Glu Val Leu Leu Gln
       50                  55                  60
   Lys Ala Glu Ala Leu Ala Glu Val Leu Asp Pro Gln Asn Arg Glu Ser
   65                  70                  75                  80
   Arg Ser Pro Arg Arg Cys Val Arg Leu His Glu Ser Cys Leu Gly Gln
                   85                  90                  95
   Gln Val Pro Cys Cys Asp Pro Cys Ala Thr Cys Tyr Cys Arg Phe Phe
               100                 105                 110
   Asn Ala Phe Cys Tyr Cys Arg Lys Leu Gly Thr Ala Met Asn Leu Cys
           115                 120                 125
   Ser Arg Thr Met Ser Ile Glu Asn Asn Ile Leu Ile Gly Pro Pro Pro
       130                 135                 140
   Tyr Tyr Pro Leu Glu Glu Gly Thr Ala Gly Glu Gln Leu His Arg Ala
   145                 150                 155                 160
   Ile Ser Arg Tyr Ala Ala Val Pro Gly Thr Leu Ala Tyr Thr Asp Val
                   165                 170                 175
   His Thr Glu Leu Glu Val Thr Tyr Lys Glu Phe Leu Asp Val Thr Cys
               180                 185                 190
   Arg Leu Ala Glu Ala Met Lys Asn Tyr Gly Leu Gly Leu Gln His Thr
           195                 200                 205
   Ile Ser Val Cys Ser Glu Asn Cys Val Gln Phe Phe Met Pro Ile Cys
       210                 215                 220
   Ala Ala Leu Tyr Val Gly Val Ala Thr Ala Pro Thr Asn Asp Ile Tyr
   225                 230                 235                 240
   Asn Glu Arg Glu Leu Tyr Asn Ser Leu Ser Ile Ser Gln Pro Thr Val
                   245                 250                 255
   Val Phe Thr Ser Arg Asn Ser Leu Gln Lys Ile Leu Gly Val Gln Ser
               260                 265                 270
   Arg Leu Pro Ile Ile Lys Lys Ile Ile Ile Leu Asp Gly Lys Lys Asp
           275                 280                 285
   Tyr Leu Gly Tyr Gln Ser Met Gln Ser Phe Met Lys Glu His Val Pro
       290                 295                 300
   Ala Asn Phe Asn Val Ser Ala Phe Lys Pro Leu Ser Phe Asp Leu Asp
   305                 310                 315                 320
   Arg Val Ala Cys Ile Met Asn Ser Ser Gly Ser Thr Gly Leu Pro Lys
                   325                 330                 335
   Gly Val Pro Ile Ser His Arg Asn Thr Ile Tyr Arg Phe Ser His Cys
               340                 345                 350
   Arg Asp Pro Val Phe Gly Asn Gln Ile Ile Pro Asp Thr Thr Ile Leu
           355                 360                 365
   Cys Ala Val Pro Phe His His Ala Phe Gly Thr Phe Thr Asn Leu Gly
       370                 375                 380
   Tyr Leu Ile Cys Gly Phe His Val Val Leu Met Tyr Arg Phe Asn Glu
   385                 390                 395                 400
   His Leu Phe Leu Gln Thr Leu Gln Asp Tyr Lys Cys Gln Ser Ala Leu
                   405                 410                 415
   Leu Val Pro Thr Val Leu Ala Phe Leu Ala Lys Asn Pro Leu Val Asp
               420                 425                 430
   Lys Tyr Asp Leu Ser Asn Leu His Glu Ile Ala Ser Gly Gly Ala Pro
           435                 440                 445
   Leu Ser Lys Glu Ile Ser Glu Ile Ala Ala Lys Arg Phe Lys Leu Pro
       450                 455                 460
   Gly Ile Arg Gln Gly Tyr Gly Leu Thr Glu Thr Thr Cys Ala Ile Val
   465                 470                 475                 480
   Ile Thr Ala Glu Gly Glu Phe Lys Leu Gly Ala Val Gly Lys Val Val
                   485                 490                 495
   Pro Phe Tyr Ser Leu Lys Val Leu Asp Leu Asn Thr Gly Lys Lys Leu
               500                 505                 510
   Gly Pro Asn Glu Arg Gly Glu Ile Cys Phe Lys Gly Pro Met Ile Met
           515                 520                 525
   Lys Gly Tyr Ile Asn Asn Pro Glu Ala Thr Arg Glu Leu Ile Asp Glu
       530                 535                 540
   Glu Gly Trp Ile His Ser Gly Asp Ile Gly Tyr Phe Asp Glu Asp Gly
   545                 550                 555                 560
   His Val Tyr Ile Val Asp Arg Leu Lys Ser Leu Ile Lys Tyr Lys Gly
                   565                 570                 575
   Tyr Gln Val Pro Pro Ala Glu Leu Glu Ala Leu Leu Leu Gln His Pro
               580                 585                 590
   Phe Ile Glu Asp Ala Gly Val Ala Gly Val Pro Asp Glu Val Ala Gly
           595                 600                 605
   Asp Leu Pro Gly Ala Val Val Val Leu Lys Glu Gly Lys Ser Ile Thr
       610                 615                 620
   Glu Lys Glu Ile Gln Asp Tyr Val Ala Gly Gln Val Thr Ser Ser Lys
   625                 630                 635                 640
   Lys Leu Arg Gly Gly Val Glu Phe Val Lys Glu Val Pro Lys Gly Phe
                   645                 650                 655
   Thr Gly Lys Ile Asp Thr Arg Lys Ile Lys Glu Ile Leu Ile Lys Ala
               660                 665                 670
   Gln Lys Gly Lys Ser Lys Ser Lys Ala Lys Leu
           675                 680
(2) INFORMATION FOR SEQ ID NO:15:
   (i) SEQUENCE CHARACTERISTICS:
      (A) LENGTH: 605 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:15:
   Met Leu Thr Ala Ala Leu Leu Ser Cys Ala Leu Leu Leu Ala Leu Pro
    1               5                  10                  15
   Ala Thr Arg Gly Ala Gln Met Gly Leu Ala Leu Gln Asp Arg Glu Pro
               20                  25                  30
   Arg Ser Ser Arg Arg Cys Val Arg Leu His Glu Ser Cys Leu Gly Gln
           35                  40                  45
   Gln Val Pro Cys Cys Asp Pro Cys Ala Thr Cys Tyr Cys Arg Phe Phe
       50                  55                  60
   Asn Ala Phe Cys Tyr Cys Arg Lys Leu Gly Thr Ala Met Asn Pro Cys
   65                  70                  75                  80
   Ser Arg Thr Leu Val Pro Arg Gly Ser Gly Ser Ile Ile Pro Val Glu
                   85                  90                  95
   Glu Glu Asn Pro Asp Phe Trp Asn Arg Gln Ala Ala Glu Ala Leu Gly
               100                 105                 110
   Ala Ala Lys Lys Leu Gln Pro Ala Gln Thr Ala Ala Lys Asn Leu Ile
           115                 120                 125
   Ile Phe Leu Gly Asp Gly Met Gly Val Ser Thr Val Thr Ala Ala Arg
       130                 135                 140
   Ile Leu Lys Gly Gln Lys Lys Asp Lys Leu Gly Pro Glu Thr Phe Leu
   145                 150                 155                 160
   Ala Met Asp Arg Phe Pro Tyr Val Ala Leu Ser Lys Thr Tyr Ser Val
                   165                 170                 175
   Asp Lys His Val Pro Asp Ser Gly Ala Thr Ala Thr Ala Tyr Leu Cys
               180                 185                 190
   Gly Val Lys Gly Asn Phe Gln Thr Ile Gly Leu Ser Ala Ala Ala Arg
           195                 200                 205
   Phe Asn Gln Cys Asn Thr Thr Arg Gly Asn Glu Val Ile Ser Val Met
       210                 215                 220
   Asn Arg Ala Lys Lys Ala Gly Lys Ser Val Gly Val Val Thr Thr Thr
   225                 230                 235                 240
   Arg Val Gln His Ala Ser Pro Ala Gly Ala Tyr Ala His Thr Val Asn
                   245                 250                 255
   Arg Asn Trp Tyr Ser Asp Ala Asp Val Pro Ala Ser Ala Arg Gln Glu
               260                 265                 270
   Gly Cys Gln Asp Ile Ala Thr Gln Leu Ile Ser Asn Met Asp Ile Asp
           275                 280                 285
   Val Ile Leu Gly Gly Gly Arg Lys Tyr Met Phe Pro Met Gly Thr Pro
       290                 295                 300
   Asp Pro Glu Tyr Pro Asp Asp Tyr Ser Gln Gly Gly Thr Arg Leu Asp
   305                 310                 315                 320
   Gly Lys Asn Leu Val Gln Glu Trp Leu Ala Lys His Gln Gly Ala Arg
                   325                 330                 335
   Tyr Val Trp Asn Arg Thr Glu Leu Met Gln Ala Ser Leu Asp Pro Ser
               340                 345                 350
   Val Thr His Leu Met Gly Leu Phe Glu Pro Gly Asp Met Lys Tyr Glu
           355                 360                 365
   Ile His Arg Asp Ser Thr Leu Asp Pro Ser Leu Met Glu Met Thr Glu
       370                 375                 380
   Ala Ala Leu Arg Leu Leu Ser Arg Asn Pro Arg Gly Phe Phe Leu Phe
   385                 390                 395                 400
   Val Glu Gly Gly Arg Ile Asp His Gly His His Glu Ser Arg Ala Tyr
                   405                 410                 415
   Arg Ala Leu Thr Glu Thr Ile Met Phe Asp Asp Ala Ile Glu Arg Ala
               420                 425                 430
   Gly Gln Leu Thr Ser Glu Glu Asp Thr Leu Ser Leu Val Thr Ala Asp
           435                 440                 445
   His Ser His Val Phe Ser Phe Gly Gly Tyr Pro Leu Arg Gly Ser Ser
       450                 455                 460
   Ile Phe Gly Leu Ala Pro Gly Lys Ala Arg Asp Arg Lys Ala Tyr Thr
   465                 470                 475                 480
   Val Leu Leu Tyr Gly Asn Gly Pro Gly Tyr Val Leu Lys Asp Gly Ala
                   485                 490                 495
   Arg Pro Asp Val Thr Glu Ser Glu Ser Gly Ser Pro Glu Tyr Arg Gln
               500                 505                 510
   Gln Ser Ala Val Pro Leu Asp Gly Glu Thr His Ala Gly Glu Asp Val
           515                 520                 525
   Ala Val Phe Ala Arg Gly Pro Gln Ala His Leu Val His Gly Val Gln
       530                 535                 540
   Glu Gln Thr Phe Ile Ala His Val Met Ala Phe Ala Ala Cys Leu Glu
   545                 550                 555                 560
   Pro Tyr Thr Ala Cys Asp Leu Ala Pro Ser Ala Gly Thr Thr Asp Ala
                   565                 570                 575
   Ala His Pro Gly Lys Leu Gly Pro Glu Gln Lys Leu Ile Ser Glu Glu
               580                 585                 590
   Asp Leu Asn Ser Ala Val Asp His His His His His His
           595                 600                 605
(2) INFORMATION FOR SEQ ID NO:16:
   (i) SEQUENCE CHARACTERISTICS:
      (A) LENGTH: 666 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:16:
   Met Leu Thr Ala Ala Leu Leu Ser Cys Ala Leu Leu Leu Ala Leu Pro
    1               5                  10                  15
   Ala Thr Arg Gly Ala Gln Met Gly Leu Ala Leu Gln Asp Arg Glu Pro
               20                  25                  30
   Arg Ser Ser Arg Arg Cys Val Arg Leu His Glu Ser Cys Leu Gly Gln
           35                  40                  45
   Gln Val Pro Cys Cys Asp Pro Cys Ala Thr Cys Tyr Cys Arg Phe Phe
       50                  55                  60
   Asn Ala Phe Cys Tyr Cys Arg Lys Leu Gly Thr Ala Met Asn Pro Cys
   65                  70                  75                  80
   Ser Arg Thr Leu Val Pro Arg Gly Ser Gly Ser Met Ser Ile Glu Asn
                   85                  90                  95
   Asn Ile Leu Ile Gly Pro Pro Pro Tyr Tyr Pro Leu Glu Glu Gly Thr
               100                 105                 110
   Ala Gly Glu Gln Leu His Arg Ala Ile Ser Arg Tyr Ala Ala Val Pro
           115                 120                 125
   Gly Thr Leu Ala Tyr Thr Asp Val His Thr Glu Leu Glu Val Thr Tyr
       130                 135                 140
   Lys Glu Phe Leu Asp Val Thr Cys Arg Leu Ala Glu Ala Met Lys Asn
   145                 150                 155                 160
   Tyr Gly Leu Gly Leu Gln His Thr Ile Ser Val Cys Ser Glu Asn Cys
                   165                 170                 175
   Val Gln Phe Phe Met Pro Ile Cys Ala Ala Leu Tyr Val Gly Val Ala
               180                 185                 190
   Thr Ala Pro Thr Asn Asp Ile Tyr Asn Glu Arg Glu Leu Tyr Asn Ser
           195                 200                 205
   Leu Ser Ile Ser Gln Pro Thr Val Val Phe Thr Ser Arg Asn Ser Leu
       210                 215                 220
   Gln Lys Ile Leu Gly Val Gln Ser Arg Leu Pro Ile Ile Lys Lys Ile
   225                 230                 235                 240
   Ile Ile Leu Asp Gly Lys Lys Asp Tyr Leu Gly Tyr Gln Ser Met Gln
                   245                 250                 255
   Ser Phe Met Lys Glu His Val Pro Ala Asn Phe Asn Val Ser Ala Phe
               260                 265                 270
   Lys Pro Leu Ser Phe Asp Leu Asp Arg Val Ala Cys Ile Met Asn Ser
           275                 280                 285
   Ser Gly Ser Thr Gly Leu Pro Lys Gly Val Pro Ile Ser His Arg Asn
       290                 295                 300
   Thr Ile Tyr Arg Phe Ser His Cys Arg Asp Pro Val Phe Gly Asn Gln
   305                 310                 315                 320
   Ile Ile Pro Asp Thr Thr Ile Leu Cys Ala Val Pro Phe His His Ala
                   325                 330                 335
   Phe Gly Thr Phe Thr Asn Leu Gly Tyr Leu Ile Cys Gly Phe His Val
               340                 345                 350
   Val Leu Met Tyr Arg Phe Asn Glu His Leu Phe Leu Gln Thr Leu Gln
           355                 360                 365
   Asp Tyr Lys Cys Gln Ser Ala Leu Leu Val Pro Thr Val Leu Ala Phe
       370                 375                 380
   Leu Ala Lys Asn Pro Leu Val Asp Lys Tyr Asp Leu Ser Asn Leu His
   385                 390                 395                 400
   Glu Ile Ala Ser Gly Gly Ala Pro Leu Ser Lys Glu Ile Ser Glu Ile
                   405                 410                 415
   Ala Ala Lys Arg Phe Lys Leu Pro Gly Ile Arg Gln Gly Tyr Gly Leu
               420                 425                 430
   Thr Glu Thr Thr Cys Ala Ile Val Ile Thr Ala Glu Gly Glu Phe Lys
           435                 440                 445
   Leu Gly Ala Val Gly Lys Val Val Pro Phe Tyr Ser Leu Lys Val Leu
       450                 455                 460
   Asp Leu Asn Thr Gly Lys Lys Leu Gly Pro Asn Glu Arg Gly Glu Ile
   465                 470                 475                 480
   Cys Phe Lys Gly Pro Met Ile Met Lys Gly Tyr Ile Asn Asn Pro Glu
                   485                 490                 495
   Ala Thr Arg Glu Leu Ile Asp Glu Glu Gly Trp Ile His Ser Gly Asp
               500                 505                 510
   Ile Gly Tyr Phe Asp Glu Asp Gly His Val Tyr Ile Val Asp Arg Leu
           515                 520                 525
   Lys Ser Leu Ile Lys Tyr Lys Gly Tyr Gln Val Pro Pro Ala Glu Leu
       530                 535                 540
   Glu Ala Leu Leu Leu Gln His Pro Phe Ile Glu Asp Ala Gly Val Ala
   545                 550                 555                 560
   Gly Val Pro Asp Glu Val Ala Gly Asp Leu Pro Gly Ala Val Val Val
                   565                 570                 575
   Leu Lys Glu Gly Lys Ser Ile Thr Glu Lys Glu Ile Gln Asp Tyr Val
               580                 585                 590
   Ala Gly Gln Val Thr Ser Ser Lys Lys Leu Arg Gly Gly Val Glu Phe
           595                 600                 605
   Val Lys Glu Val Pro Lys Gly Phe Thr Gly Lys Ile Asp Thr Arg Lys
       610                 615                 620
   Ile Lys Glu Ile Leu Ile Lys Ala Gln Lys Gly Lys Ser Lys Ser Lys
   625                 630                 635                 640
   Ala Lys Leu Gly Pro Glu Gln Lys Leu Ile Ser Glu Glu Asp Leu Asn
                   645                 650                 655
   Ser Ala Val Asp His His His His His His
               660                 665
(2) INFORMATION FOR SEQ ID NO:17:
   (i) SEQUENCE CHARACTERISTICS:
      (A) LENGTH: 621 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:17:
   Met Leu Thr Ala Ala Leu Leu Ser Cys Ala Leu Leu Leu Ala Leu Pro
    1               5                  10                  15
   Ala Thr Arg Gly Ala Gln Met Gly Leu Ala Pro Met Glu Gly Ile Arg
               20                  25                  30
   Arg Pro Asp Gln Ala Leu Leu Pro Glu Leu Pro Gly Leu Gly Leu Arg
           35                  40                  45
   Ala Pro Leu Lys Lys Thr Asn Ala Glu Gln Ala Glu Glu Asp Leu Leu
       50                  55                  60
   Gln Glu Ala Gln Ala Leu Ala Glu Val Leu Asp Leu Gln Asp Arg Glu
   65                  70                  75                  80
   Pro Arg Ser Ser Arg Arg Cys Val Arg Leu His Glu Ser Cys Leu Gly
                   85                  90                  95
   Gln Gln Val Pro Cys Cys Asp Pro Cys Ala Thr Cys Tyr Cys Arg Phe
               100                 105                 110
   Phe Asn Ala Phe Cys Tyr Cys Arg Lys Leu Gly Thr Ala Met Asn Pro
           115                 120                 125
   Cys Ser Arg Thr Ile Ile Pro Val Glu Glu Glu Asn Pro Asp Phe Trp
       130                 135                 140
   Asn Arg Gln Ala Ala Glu Ala Leu Gly Ala Ala Lys Lys Leu Gln Pro
   145                 150                 155                 160
   Ala Gln Thr Ala Ala Lys Asn Leu Ile Ile Phe Leu Gly Asp Gly Met
                   165                 170                 175
   Gly Val Ser Thr Val Thr Ala Ala Arg Ile Leu Lys Gly Gln Lys Lys
               180                 185                 190
   Asp Lys Leu Gly Pro Glu Thr Phe Leu Ala Met Asp Arg Phe Pro Tyr
           195                 200                 205
   Val Ala Leu Ser Lys Thr Tyr Ser Val Asp Lys His Val Pro Asp Ser
       210                 215                 220
   Gly Ala Thr Ala Thr Ala Tyr Leu Cys Gly Val Lys Gly Asn Phe Gln
   225                 230                 235                 240
   Thr Ile Gly Leu Ser Ala Ala Ala Arg Phe Asn Gln Cys Asn Thr Thr
                   245                 250                 255
   Arg Gly Asn Glu Val Ile Ser Val Met Asn Arg Ala Lys Lys Ala Gly
               260                 265                 270
   Lys Ser Val Gly Val Val Thr Thr Thr Arg Val Gln His Ala Ser Pro
           275                 280                 285
   Ala Gly Ala Tyr Ala His Thr Val Asn Arg Asn Trp Tyr Ser Asp Ala
       290                 295                 300
   Asp Val Pro Ala Ser Ala Arg Gln Glu Gly Cys Gln Asp Ile Ala Thr
   305                 310                 315                 320
   Gln Leu Ile Ser Asn Met Asp Ile Asp Val Ile Leu Gly Gly Gly Arg
                   325                 330                 335
   Lys Tyr Met Phe Pro Met Gly Thr Pro Asp Pro Glu Tyr Pro Asp Asp
               340                 345                 350
   Tyr Ser Gln Gly Gly Thr Arg Leu Asp Gly Lys Asn Leu Val Gln Glu
           355                 360                 365
   Trp Leu Ala Lys His Gln Gly Ala Arg Tyr Val Trp Asn Arg Thr Glu
       370                 375                 380
   Leu Met Gln Ala Ser Leu Asp Pro Ser Val Thr His Leu Met Gly Leu
   385                 390                 395                 400
   Phe Glu Pro Gly Asp Met Lys Tyr Glu Ile His Arg Asp Ser Thr Leu
                   405                 410                 415
   Asp Pro Ser Leu Met Glu Met Thr Glu Ala Ala Leu Arg Leu Leu Ser
               420                 425                 430
   Arg Asn Pro Arg Gly Phe Phe Leu Phe Val Glu Gly Gly Arg Ile Asp
           435                 440                 445
   His Gly His His Glu Ser Arg Ala Tyr Arg Ala Leu Thr Glu Thr Ile
       450                 455                 460
   Met Phe Asp Asp Ala Ile Glu Arg Ala Gly Gln Leu Thr Ser Glu Glu
   465                 470                 475                 480
   Asp Thr Leu Ser Leu Val Thr Ala Asp His Ser His Val Phe Ser Phe
                   485                 490                 495
   Gly Gly Tyr Pro Leu Arg Gly Ser Ser Ile Phe Gly Leu Ala Pro Gly
               500                 505                 510
   Lys Ala Arg Asp Arg Lys Ala Tyr Thr Val Leu Leu Tyr Gly Asn Gly
           515                 520                 525
   Pro Gly Tyr Val Leu Lys Asp Gly Ala Arg Pro Asp Val Thr Glu Ser
       530                 535                 540
   Glu Ser Gly Ser Pro Glu Tyr Arg Gln Gln Ser Ala Val Pro Leu Asp
   545                 550                 555                 560
   Gly Glu Thr His Ala Gly Glu Asp Val Ala Val Phe Ala Arg Gly Pro
                   565                 570                 575
   Gln Ala His Leu Val His Gly Val Gln Glu Gln Thr Phe Ile Ala His
               580                 585                 590
   Val Met Ala Phe Ala Ala Cys Leu Glu Pro Tyr Thr Ala Cys Asp Leu
           595                 600                 605
   Ala Pro Ser Ala Gly Thr Thr Asp Ala Ala His Pro Gly
       610                 615                 620
(2) INFORMATION FOR SEQ ID NO:18:
   (i) SEQUENCE CHARACTERISTICS:
      (A) LENGTH: 684 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:18:
   Met Leu Thr Ala Ala Leu Leu Ser Cys Ala Leu Leu Leu Ala Leu Pro
    1               5                  10                  15
   Ala Thr Arg Gly Ala Gln Met Gly Leu Ala Pro Met Glu Gly Ile Arg
               20                  25                  30
   Arg Pro Asp Gln Ala Leu Leu Pro Glu Leu Pro Gly Leu Gly Leu Arg
           35                  40                  45
   Ala Pro Leu Lys Lys Thr Asn Ala Glu Gln Ala Glu Glu Asp Leu Leu
       50                  55                  60
   Gln Glu Ala Gln Ala Leu Ala Glu Val Leu Asp Leu Gln Asp Arg Glu
   65                  70                  75                  80
   Pro Arg Ser Ser Arg Arg Cys Val Arg Leu His Glu Ser Cys Leu Gly
                   85                  90                  95
   Gln Gln Val Pro Cys Cys Asp Pro Cys Ala Thr Cys Tyr Cys Arg Phe
               100                 105                 110
   Phe Asn Ala Phe Cys Tyr Cys Arg Lys Leu Gly Thr Ala Met Asn Pro
           115                 120                 125
   Cys Ser Arg Thr Met Ser Ile Glu Asn Asn Ile Leu Ile Gly Pro Pro
       130                 135                 140
   Pro Tyr Tyr Pro Leu Glu Glu Gly Thr Ala Gly Glu Gln Leu His Arg
   145                 150                 155                 160
   Ala Ile Ser Arg Tyr Ala Ala Val Pro Gly Thr Leu Ala Tyr Thr Asp
                   165                 170                 175
   Val His Thr Glu Leu Glu Val Thr Tyr Lys Glu Phe Leu Asp Val Thr
               180                 185                 190
   Cys Arg Leu Ala Glu Ala Met Lys Asn Tyr Gly Leu Gly Leu Gln His
           195                 200                 205
   Thr Ile Ser Val Cys Ser Glu Asn Cys Val Gln Phe Phe Met Pro Ile
       210                 215                 220
   Cys Ala Ala Leu Tyr Val Gly Val Ala Thr Ala Pro Thr Asn Asp Ile
   225                 230                 235                 240
   Tyr Asn Glu Arg Glu Leu Tyr Asn Ser Leu Ser Ile Ser Gln Pro Thr
                   245                 250                 255
   Val Val Phe Thr Ser Arg Asn Ser Leu Gln Lys Ile Leu Gly Val Gln
               260                 265                 270
   Ser Arg Leu Pro Ile Ile Lys Lys Ile Ile Ile Leu Asp Gly Lys Lys
           275                 280                 285
   Asp Tyr Leu Gly Tyr Gln Ser Met Gln Ser Phe Met Lys Glu His Val
       290                 295                 300
   Pro Ala Asn Phe Asn Val Ser Ala Phe Lys Pro Leu Ser Phe Asp Leu
   305                 310                 315                 320
   Asp Arg Val Ala Cys Ile Met Asn Ser Ser Gly Ser Thr Gly Leu Pro
                   325                 330                 335
   Lys Gly Val Pro Ile Ser His Arg Asn Thr Ile Tyr Arg Phe Ser His
               340                 345                 350
   Cys Arg Asp Pro Val Phe Gly Asn Gln Ile Ile Pro Asp Thr Thr Ile
           355                 360                 365
   Leu Cys Ala Val Pro Phe His His Ala Phe Gly Thr Phe Thr Asn Leu
       370                 375                 380
   Gly Tyr Leu Ile Cys Gly Phe His Val Val Leu Met Tyr Arg Phe Asn
   385                 390                 395                 400
   Glu His Leu Phe Leu Gln Thr Leu Gln Asp Tyr Lys Cys Gln Ser Ala
                   405                 410                 415
   Leu Leu Val Pro Thr Val Leu Ala Phe Leu Ala Lys Asn Pro Leu Val
               420                 425                 430
   Asp Lys Tyr Asp Leu Ser Asn Leu His Glu Ile Ala Ser Gly Gly Ala
           435                 440                 445
   Pro Leu Ser Lys Glu Ile Ser Glu Ile Ala Ala Lys Arg Phe Lys Leu
       450                 455                 460
   Pro Gly Ile Arg Gln Gly Tyr Gly Leu Thr Glu Thr Thr Cys Ala Ile
   465                 470                 475                 480
   Val Ile Thr Ala Glu Gly Glu Phe Lys Leu Gly Ala Val Gly Lys Val
                   485                 490                 495
   Val Pro Phe Tyr Ser Leu Lys Val Leu Asp Leu Asn Thr Gly Lys Lys
               500                 505                 510
   Leu Gly Pro Asn Glu Arg Gly Glu Ile Cys Phe Lys Gly Pro Met Ile
           515                 520                 525
   Met Lys Gly Tyr Ile Asn Asn Pro Glu Ala Thr Arg Glu Leu Ile Asp
       530                 535                 540
   Glu Glu Gly Trp Ile His Ser Gly Asp Ile Gly Tyr Phe Asp Glu Asp
   545                 550                 555                 560
   Gly His Val Tyr Ile Val Asp Arg Leu Lys Ser Leu Ile Lys Tyr Lys
                   565                 570                 575
   Gly Tyr Gln Val Pro Pro Ala Glu Leu Glu Ala Leu Leu Leu Gln His
               580                 585                 590
   Pro Phe Ile Glu Asp Ala Gly Val Ala Gly Val Pro Asp Glu Val Ala
           595                 600                 605
   Gly Asp Leu Pro Gly Ala Val Val Val Leu Lys Glu Gly Lys Ser Ile
       610                 615                 620
   Thr Glu Lys Glu Ile Gln Asp Tyr Val Ala Gly Gln Val Thr Ser Ser
   625                 630                 635                     640
   Lys Lys Leu Arg Gly Gly Val Glu Phe Val Lys Glu Val Pro Lys Gly
                   645                 650                 655
   Phe Thr Gly Lys Ile Asp Thr Arg Lys Ile Lys Glu Ile Leu Ile Lys
               660                 665                 670
   Ala Gln Lys Gly Lys Ser Lys Ser Lys Ala Lys Leu
           675                 680
(2) INFORMATION FOR SEQ ID NO:19:
   (i) SEQUENCE CHARACTERISTICS:
      (A) LENGTH: 621 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:19:
   Met Leu Thr Ala Ala Leu Leu Ser Cys Ala Leu Leu Leu Ala Leu Pro
    1               5                  10                  15
   Ala Thr Arg Gly Ala Gln Met Gly Leu Ala Ile Ile Pro Val Glu Glu
               20                  25                  30
   Glu Asn Pro Asp Phe Trp Asn Arg Gln Ala Ala Glu Ala Leu Gly Ala
           35                  40                  45
   Ala Lys Lys Leu Gln Pro Ala Gln Thr Ala Ala Lys Asn Leu Ile Ile
       50                  55                  60
   Phe Leu Gly Asp Gly Met Gly Val Ser Thr Val Thr Ala Ala Arg Ile
   65                  70                  75                  80
   Leu Lys Gly Gln Lys Lys Asp Lys Leu Gly Pro Glu Thr Phe Leu Ala
                   85                  90                  95
   Met Asp Arg Phe Pro Tyr Val Ala Leu Ser Lys Thr Tyr Ser Val Asp
               100                 105                 110
   Lys His Val Pro Asp Ser Gly Ala Thr Ala Thr Ala Tyr Leu Cys Gly
           115                 120                 125
   Val Lys Gly Asn Phe Gln Thr Ile Gly Leu Ser Ala Ala Ala Arg Phe
       130                 135                 140
   Asn Gln Cys Asn Thr Thr Arg Gly Asn Glu Val Ile Ser Val Met Asn
   145                 150                 155                 160
   Arg Ala Lys Lys Ala Gly Lys Ser Val Gly Val Val Thr Thr Thr Arg
                   165                 170                 175
   Val Gln His Ala Ser Pro Ala Gly Ala Tyr Ala His Thr Val Asn Arg
               180                 185                 190
   Asn Trp Tyr Ser Asp Ala Asp Val Pro Ala Ser Ala Arg Gln Glu Gly
           195                 200                 205
   Cys Gln Asp Ile Ala Thr Gln Leu Ile Ser Asn Met Asp Ile Asp Val
       210                 215                 220
   Ile Leu Gly Gly Gly Arg Lys Tyr Met Phe Pro Met Gly Thr Pro Asp
   225                 230                 235                 240
   Pro Glu Tyr Pro Asp Asp Tyr Ser Gln Gly Gly Thr Arg Leu Asp Gly
                   245                 250                 255
   Lys Asn Leu Val Gln Glu Trp Leu Ala Lys His Gln Gly Ala Arg Tyr
               260                 265                 270
   Val Trp Asn Arg Thr Glu Leu Met Gln Ala Ser Leu Asp Pro Ser Val
           275                 280                 285
   Thr His Leu Met Gly Leu Phe Glu Pro Gly Asp Met Lys Tyr Glu Ile
       290                 295                 300
   His Arg Asp Ser Thr Leu Asp Pro Ser Leu Met Glu Met Thr Glu Ala
   305                 310                 315                 320
   Ala Leu Arg Leu Leu Ser Arg Asn Pro Arg Gly Phe Phe Leu Phe Val
                   325                 330                 335
   Glu Gly Gly Arg Ile Asp His Gly His His Glu Ser Arg Ala Tyr Arg
               340                 345                 350
   Ala Leu Thr Glu Thr Ile Met Phe Asp Asp Ala Ile Glu Arg Ala Gly
           355                 360                 365
   Gln Leu Thr Ser Glu Glu Asp Thr Leu Ser Leu Val Thr Ala Asp His
       370                 375                 380
   Ser His Val Phe Ser Phe Gly Gly Tyr Pro Leu Arg Gly Ser Ser Ile
   385                 390                 395                 400
   Phe Gly Leu Ala Pro Gly Lys Ala Arg Asp Arg Lys Ala Tyr Thr Val
                   405                 410                 415
   Leu Leu Tyr Gly Asn Gly Pro Gly Tyr Val Leu Lys Asp Gly Ala Arg
               420                 425                 430
   Pro Asp Val Thr Glu Ser Glu Ser Gly Ser Pro Glu Tyr Arg Gln Gln
           435                 440                 445
   Ser Ala Val Pro Leu Asp Gly Glu Thr His Ala Gly Glu Asp Val Ala
       450                 455                 460
   Val Phe Ala Arg Gly Pro Gln Ala His Leu Val His Gly Val Gln Glu
   465                 470                 475                 480
   Gln Thr Phe Ile Ala His Val Met Ala Phe Ala Ala Cys Leu Glu Pro
                   485                 490                 495
   Tyr Thr Ala Cys Asp Leu Ala Pro Ser Ala Gly Thr Thr Asp Ala Ala
               500                 505                 510
   His Pro Gly Pro Met Glu Gly Ile Arg Arg Pro Asp Gln Ala Leu Leu
           515                 520                 525
   Pro Glu Leu Pro Gly Leu Gly Leu Arg Ala Pro Leu Lys Lys Thr Asn
       530                 535                 540
   Ala Glu Gln Ala Glu Glu Asp Leu Leu Gln Glu Ala Gln Ala Leu Ala
   545                 550                 555                 560
   Glu Val Leu Asp Leu Gln Asp Arg Glu Pro Arg Ser Ser Arg Arg Cys
                   565                 570                 575
   Val Arg Leu His Glu Ser Cys Leu Gly Gln Gln Val Pro Cys Cys Asp
               580                 585                 590
   Pro Cys Ala Thr Cys Tyr Cys Arg Phe Phe Asn Ala Phe Cys Tyr Cys
           595                 600                 605
   Arg Lys Leu Gly Thr Ala Met Asn Pro Cys Ser Arg Thr
       610                 615                 620
(2) INFORMATION FOR SEQ ID NO:20:
   (i) SEQUENCE CHARACTERISTICS:
      (A) LENGTH: 684 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:20:
   Met Leu Thr Ala Ala Leu Leu Ser Cys Ala Leu Leu Leu Ala Leu Pro
    1               5                  10                  15
   Ala Thr Arg Gly Ala Gln Met Gly Leu Ala Met Ser Ile Glu Asn Asn
               20                  25                  30
   Ile Leu Ile Gly Pro Pro Pro Tyr Tyr Pro Leu Glu Glu Gly Thr Ala
           35                  40                  45
   Gly Glu Gln Leu His Arg Ala Ile Ser Arg Tyr Ala Ala Val Pro Gly
       50                  55                  60
   Thr Leu Ala Tyr Thr Asp Val His Thr Glu Leu Glu Val Thr Tyr Lys
   65                  70                  75                  80
   Glu Phe Leu Asp Val Thr Cys Arg Leu Ala Glu Ala Met Lys Asn Tyr
                   85                  90                  95
   Gly Leu Gly Leu Gln His Thr Ile Ser Val Cys Ser Glu Asn Cys Val
               100                 105                 110
   Gln Phe Phe Met Pro Ile Cys Ala Ala Leu Tyr Val Gly Val Ala Thr
           115                 120                 125
   Ala Pro Thr Asn Asp Ile Tyr Asn Glu Arg Glu Leu Tyr Asn Ser Leu
       130                 135                 140
   Ser Ile Ser Gln Pro Thr Val Val Phe Thr Ser Arg Asn Ser Leu Gln
   145                 150                 155                 160
   Lys Ile Leu Gly Val Gln Ser Arg Leu Pro Ile Ile Lys Lys Ile Ile
                   165                 170                 175
   Ile Leu Asp Gly Lys Lys Asp Tyr Leu Gly Tyr Gln Ser Met Gln Ser
               180                 185                 190
   Phe Met Lys Glu His Val Pro Ala Asn Phe Asn Val Ser Ala Phe Lys
           195                 200                 205
   Pro Leu Ser Phe Asp Leu Asp Arg Val Ala Cys Ile Met Asn Ser Ser
       210                 215                 220
   Gly Ser Thr Gly Leu Pro Lys Gly Val Pro Ile Ser His Arg Asn Thr
   225                 230                 235                 240
   Ile Tyr Arg Phe Ser His Cys Arg Asp Pro Val Phe Gly Asn Gln Ile
                   245                 250                 255
   Ile Pro Asp Thr Thr Ile Leu Cys Ala Val Pro Phe His His Ala Phe
               260                 265                 270
   Gly Thr Phe Thr Asn Leu Gly Tyr Leu Ile Cys Gly Phe His Val Val
           275                 280                 285
   Leu Met Tyr Arg Phe Asn Glu His Leu Phe Leu Gln Thr Leu Gln Asp
       290                 295                 300
   Tyr Lys Cys Gln Ser Ala Leu Leu Val Pro Thr Val Leu Ala Phe Leu
   305                 310                 315                 320
   Ala Lys Asn Pro Leu Val Asp Lys Tyr Asp Leu Ser Asn Leu His Glu
                   325                 330                 335
   Ile Ala Ser Gly Gly Ala Pro Leu Ser Lys Glu Ile Ser Glu Ile Ala
               340                 345                 350
   Ala Lys Arg Phe Lys Leu Pro Gly Ile Arg Gln Gly Tyr Gly Leu Thr
           355                 360                 365
   Glu Thr Thr Cys Ala Ile Val Ile Thr Ala Glu Gly Glu Phe Lys Leu
       370                 375                 380
   Gly Ala Val Gly Lys Val Val Pro Phe Tyr Ser Leu Lys Val Leu Asp
   385                 390                 395                 400
   Leu Asn Thr Gly Lys Lys Leu Gly Pro Asn Glu Arg Gly Glu Ile Cys
                   405                 410                 415
   Phe Lys Gly Pro Met Ile Met Lys Gly Tyr Ile Asn Asn Pro Glu Ala
               420                 425                 430
   Thr Arg Glu Leu Ile Asp Glu Glu Gly Trp Ile His Ser Gly Asp Ile
           435                 440                 445
   Gly Tyr Phe Asp Glu Asp Gly His Val Tyr Ile Val Asp Arg Leu Lys
       450                 455                 460
   Ser Leu Ile Lys Tyr Lys Gly Tyr Gln Val Pro Pro Ala Glu Leu Glu
   465                 470                 475                 480
   Ala Leu Leu Leu Gln His Pro Phe Ile Glu Asp Ala Gly Val Ala Gly
                   485                 490                 495
   Val Pro Asp Glu Val Ala Gly Asp Leu Pro Gly Ala Val Val Val Leu
               500                 505                 510
   Lys Glu Gly Lys Ser Ile Thr Glu Lys Glu Ile Gln Asp Tyr Val Ala
           515                 520                 525
   Gly Gln Val Thr Ser Ser Lys Lys Leu Arg Gly Gly Val Glu Phe Val
       530                 535                 540
   Lys Glu Val Pro Lys Gly Phe Thr Gly Lys Ile Asp Thr Arg Lys Ile
   545                 550                 555                 560
   Lys Glu Ile Leu Ile Lys Ala Gln Lys Gly Lys Ser Lys Ser Lys Ala
                   565                 570                 575
   Lys Leu Pro Met Glu Gly Ile Arg Arg Pro Asp Gln Ala Leu Leu Pro
               580                 585                 590
   Glu Leu Pro Gly Leu Gly Leu Arg Ala Pro Leu Lys Lys Thr Asn Ala
           595                 600                 605
   Glu Gln Ala Glu Glu Asp Leu Leu Gln Glu Ala Gln Ala Leu Ala Glu
       610                 615                 620
   Val Leu Asp Leu Gln Asp Arg Glu Pro Arg Ser Ser Arg Arg Cys Val
   625                 630                 635                 640
   Arg Leu His Glu Ser Cys Leu Gly Gln Gln Val Pro Cys Cys Asp Pro
                   645                 650                 655
   Cys Ala Thr Cys Tyr Cys Arg Phe Phe Asn Ala Phe Cys Tyr Cys Arg
               660                 665                 670
   Lys Leu Gly Thr Ala Met Asn Pro Cys Ser Arg Thr
           675                 680
(2) INFORMATION FOR SEQ ID NO:21:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:21:
      GGGCTCGGCG GTCCTGCAGG GCCAAGCCCA TCTGGGC 37
(2) INFORMATION FOR SEQ ID NO:22:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:22:
      TAATACGACT CACTATAGGG 20
(2) INFORMATION FOR SEQ ID NO:23:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:23:
      CTGCAGGACC GCGAGCCC 18
(2) INFORMATION FOR SEQ ID NO:24:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:24:
      GTCGACGGCG CTATTCAG 18

## Description

### STATEMENT REGARDING FEDERALLY-SPONSORED R&D

Not applicable

### REFERENCE TO MICROFICHE APPENDIX

Not applicable

### FIELD OF THE INVENTION

The present invention is directed to polypeptides derived from the C-terminal region of the agouti-related transcript (ART) protein and to uses of such polypeptides, including use as inhibitors of the binding of melanocyte stimulating hormones to melanocortin receptors, and use to identify inhibitors of the binding of ART protein to melanocortin receptors.

### BACKGROUND OF THE INVENTION

ART (agouti related transcript) was originally discovered as an mRNA that is upregulated in the hypothalamus of ob/ob and db/db mice. The ART gene has been cloned from both mice and humans and encodes a protein of 131 amino acids in mice and 132 amino acids in humans (Shutter et al., 1997, Genes and Development 11:593-602). Recombinantly produced ART protein has been shown to be a functional antagonist of the melanocortin-3 receptor (MC3R) and the melanocortin-4 receptor (MC4R) (Fong et al., 1997, Biochem. Biophys. Res. Comm. 237:629-631; Ollman et al., 1997, Science 278:135-138).

MC3R and MC4R belong to a class of G-protein coupled receptors known as the melanocortin receptors, since these receptors activate adenylyl cyclase in response to ligands known as melanocortins (*e.g*., adrenocorticotrophin (ACTH) and the α-, β-, and γ-melanocyte stimulating hormones). MC3R and MC4R are neural melanocortin receptors, with MC3R being expressed in the hypothalamus and limbic system of the brain and MC4R being expressed widely in the brain. In particular, MC4R expression has been found in a number of hypothalamic sites, including the ventromedial, lateral, dorsomedial, and paraventricular nuclei (Mountjoy et al., 1994, Mol. Endocrinol. 8:1298-1308), regions which have been shown to play a role in feeding behavior (Bray, 1987, Nutr. Rev. 45:33-43). Gene targeting experiments have shown that MC4R has an important role in the control of feeding behavior and obesity. Knockout mice lacking MC4R develop an obesity syndrome characterized by hyperphagia, hyperinsulinemia, and hyperglycemia (Huszar et al., 1997, Cell 88:131-141).

In view of this, there is great interest in the ART protein, which appears to be a natural regulator of MC3R and MC4R in humans. It is believed that the ART protein is likely to be a natural regulator of human obesity which functions by antagonizing either MC3R or MC4R. Accordingly, the identification of substances that inhibit the binding of ART protein to MC3R or MC4R is desirable, since such inhibitors are likely to be of value in the control of obesity. Substances that potentiate the effect of ART protein on MC3R or MC4R are also likely to be of value in the control of body weight.

### SUMMARY OF THE INVENTION

The present invention provides novel polypeptides derived from the C-terminal region of the human and mouse ART proteins. Also provided are DNA sequences encoding the novel C-terminal polypeptides. The novel C-terminal polypeptides can be used to inhibit the binding of melanocyte stimulating hormones to melanocortin receptors. Methods of identifying inhibitors of the effect of ART protein on the binding of melanocyte stimulating hormones to melanocortin receptors are also provided.

### BRIEF DESCRIPTION OF THE DRAWINGS

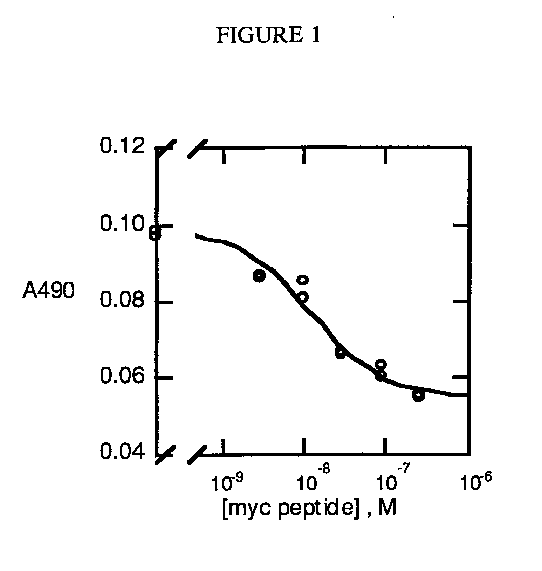

Figure 1 shows the quantitation of c-ART-b from COS-7 cells through the use of an ELISA using an antibody that recognizes the myc epitope in c-ART-b. Shown is the standard curve generated using known amounts of myc peptide. For the c-ART-b preparation made from COS-7 cells, a 10x dilution of the sample gave an absorbance of 0.079, corresponding to 10 nM in the above standard curve. Therefore, the c-ART-b preparation had a concentration of 100 nM.
Figure 2 shows the binding affinity of c-ART-b for the human MC3R. Shown is the inhibition of ¹²⁵I-[Tyr²][Nle⁴, D-Phe7] α-melanocyte stimulating hormone (¹²⁵I-NDP-α-MSH) binding to the human MC3R by c-ART-b.
Figure 3 shows the binding affinity of c-ART-b for the human MC4R. Shown is the inhibition of 125I-NDP-α-MSH binding to the human MC4R by c-ART-b.

### DETAILED DESCRIPTION OF THE INVENTION

The present invention provides C-terminal polypeptides derived from the agouti-related transcript (ART) protein and DNA sequences encoding those polypeptides. The C-terminal polypeptides from the ART protein are referred to herein as "ART polypeptides". In certain embodiments, the ART polypeptides are present in a contiguous polypeptide sequence, *i.e*., a fusion protein, that incorporates, generally at the C-terminus of the fusion protein, one or more amino acid sequences not derived from the ART protein. Such non-ART protein sequences can be, *e.g.*, "tags", such as a protein kinase A site (for easier radioisotope labeling) or an antigenic sequence (*e.g*., a myc epitope) for ELISA quantitation. Other tags are known in the art and ART polypeptides incorporating such other tags are included in the present invention. In other embodiments, the ART polypeptides are present in a fusion protein with another protein that gives rise to an easily detectable signal, *e.g.*, alkaline phosphatase (ART-AP) or luciferase (ART-luc). Fusion proteins such as ART-AP or ART-luc are useful in binding assays since their presence and/or concentration can be detected without the use of radioactivity.

In particular, the present invention includes the following ART polypeptides:
c-ART-a: This polypeptide contains, from N to C terminus: (1) a yeast signal sequence peptide; (2) amino acids 76-132 of the human ART protein; (3) a thrombin site; (4) a myc epitope; (5) a protein kinase A (PKA) site; and (6) a hexahistidine tag. The amino acid sequence of c-ART-a is:
c-AR,T-b: This polypeptide contains, from N to C terminus: (1) amino acids 1-26 of the human ART protein; (2) amino acids 76-132 of the human ART protein; (3) a thrombin site; (4) a myc epitope; and (5) a hexahistidine tag. The amino acid sequence of c-ART-b is:
c-ART-c: This polypeptide contains, from N to C terminus: (1) amino acids 1-26 of the human ART protein; (2) amino acids 76-132 of the human ART protein; (3) a thrombin site; (4) a PKA site; (5) a myc epitope; and (6) a hexahistidine tag. The amino acid sequence of
c-ART-c is:
ART-AP: This polypeptide contains, from N to C terminus: (1) amino acids 1-132 of the human ART protein; (2) a thrombin site; (3) the alkaline phosphatase protein; (4) a myc epitope; and (5) a hexahistidine tag. The amino acid sequence of ART-AP is:
ART-luc: This polypeptide contains, from N to C terminus: (1) amino acids 1-132 of the human ART protein; (2) a thrombin site; (3) the luciferase protein; (4) a myc epitope; and (5) a hexahistidine tag. The amino acid sequence of ART-luc is:

The present invention also includes an ART polypeptide containing amino acids 1-26 and 76-132 of the human ART protein, having the following polypeptide sequence:

The present invention also includes an ART polypeptide containing amino acids 76-132 of the human ART protein, having the following polypeptide sequence:

The present invention also includes an ART polypeptide containing amino acids 1-26 and 75-131 of the mouse ART protein, having the following polypeptide sequence:

The present invention also includes an ART polypeptide containing amino acids 75-131 of the mouse ART protein, having the following polypeptide sequence:

The present invention also includes an ART polypeptide having the following sequence, from N to C terminus: (1) a yeast signal sequence peptide; (2) amino acids 75-131 of the mouse ART protein; (3) a thrombin site; (4) a myc epitope; (5) a PKA site; and (6) a hexahistidine tag. The amino acid sequence of this ART polypeptide is:

The present invention also includes an ART polypeptide having the following sequence, from N to C terminus: (1) amino acids 1-26 of the mouse ART protein; (2) amino acids 75-131 of the mouse ART protein; (3) a thrombin site; (4) a myc epitope; and (5) a hexahistidine tag. The amino acid sequence of this ART polypeptide is:

The present invention also includes an ART polypeptide having the following sequence, from N to C terminus: (1) amino acids 1-26 of the mouse ART protein; (2) amino acids 75-131 of the mouse ART protein; (3) a thrombin site; (4) PKA site; (5) a myc epitope; and (6) a hexahistidine tag. The amino acid sequence of this ART polypeptide is:

The present invention also includes an ART polypeptide, having the following sequence, from N to C terminus: (1) amino acids 1-131 of the mouse ART protein; and (2) the alkaline phosphatase protein. The amino acid sequence of this polypeptide is:

The present invention also includes an ART polypeptide, having the following sequence, from N to C terminus: (1) amino acids 1-131 of the mouse ART protein; and (2) the luciferase protein. The amino acid sequence of this polypeptide is:

The present invention also includes an ART polypeptide, having the following sequence, from N to C terminus: (1) amino acids 1-26 of the human ART protein; (2) amino acids 76-132 of the human ART protein; (3) a thrombin site; (4) the alkaline phosphatase protein; (5) a myc epitope; and (6) a hexahistidine tag. The amino acid sequence of this polypeptide is:

The present invention also includes an ART polypeptide, having the following sequence, from N to C terminus, (1) amino acids 1-26 of the human ART protein; (2) amino acids 76-132 of the human ART protein; (3) a thrombin site; (4) the luciferase protein; (5) a myc epitope; and (6) a hexahistidine tag. The amino acid sequence of this polypeptide is:

The present invention also includes an ART polypeptide having the following sequence, from N to C terminus: (1) amino acids 1-132 of the human ART protein; and (2) the alkaline phosphatase protein. The amino acid sequence of this polypeptide is:

The present invention also includes an ART polypeptide having the following sequence, from N to C terminus: (1) amino acids 1-132 of the human ART protein; and (2) the luciferase protein. The amino acid sequence of this polypeptide is:

The present invention also includes an ART polypeptide having the following sequence, from N to C terminus: (1) amino acids 1-26 of the human ART protein; (2) the alkaline phosphatase protein; (3) amino acids 27-132 of the human ART protein. The amino acid sequence is:

The present invention also includes an ART polypeptide having the following sequence, from N to C terminus: (1) amino acids 1-26 of the human ART protein; (2) the luciferase protein; (3) amino acids 27-132 of the human ART protein. The amino acid sequence is:

The ART polypeptides of the present invention can be in a form that is substantially free from other polypeptides. "Substantially free from other polypeptides" means at least 90%, preferably 95%, more preferably 99%, and even more preferably 99.9%, free of other proteins. Thus, an ART polypeptide preparation that is substantially free from other polypeptides will contain, as a percent of its total polypeptides, no more than 10%, preferably no more than 5%, more preferably no more than 1%, and even more preferably no more than 0.1%, of non-ART polypeptides. Whether a given ART polypeptide preparation is substantially free from other polypeptides can be determined by such conventional techniques as, *e.g*., sodium dodecyl sulfate polyacrylamide gel electrophoresis (SDS-PAGE) combined with appropriate staining methods, *e.g*., silver staining.

It is possible to modify many of the amino acids of the ART polypeptides of the present invention and still retain substantially the same biological activity as possessed by the unmodified ART polypeptide. A modified ART polypeptide has "substantially the same biological activity" as an unmodified ART polypeptide if the modified polypeptide has an IC₅₀ value for the inhibition of ¹²⁵I-labeled NDP-α-MSH binding to MC3R or MC4R that is no more than 5-fold greater than the IC₅₀ value of the unmodified ART polypeptide for the inhibition of ¹²⁵I-labeled NDP-α-MSH binding to MC3R or MC4R.

Thus the present invention includes modified ART polypeptides which have amino acid deletions, additions, or substitutions but that still retain substantially the same biological activity as the unmodified ART polypeptide from which they are derived. It is generally accepted that single amino acid substitutions at noncritical positions do not usually alter the biological activity of a protein or polypeptide (see, *e.g.*, Molecular Biology of the Gene, Watson et al., 1987, Fourth Ed., The Benjamin/Cummings Publishing Co., Inc., page 226; and Cunningham & Wells, 1989, Science 244:1081-1085). Accordingly, the present invention includes modified polypeptides where one amino acid substitution has been made in SEQ.ID.NOs.:1-20 wherein the modified polypeptides still retain substantially the same biological activity as the unmodified ART polypeptides. The present invention also includes modified polypeptides where two amino acid substitutions have been made in SEQ.ID.NOs.:1-20 wherein the polypeptides still retain substantially the same biological activity as the unmodified ART polypeptides. More generally, the present invention includes modified polypeptides where amino acid substitutions have been made in regions of the polypeptides that are not critical, *i.e*., in regions where modifications result in a polypeptide with substantially the same biological activity as the unmodified polypeptide.

In particular, the present invention includes embodiments where the above-described substitutions are conservative substitutions. A "conservative amino acid substitution" refers to the replacement of one amino acid residue by another, chemically similar, amino acid residue. Examples of such conservative substitutions are: substitution of one hydrophobic residue (isoleucine, leucine, valine, or methionine) for another; substitution of one polar residue for another polar residue of the same charge (*e.g*., arginine for lysine; glutamic acid for aspartic acid).

The present invention also includes DNA sequences encoding polypeptides having the amino acid sequences of SEQ.ID.NOs.:1-20, with the proviso that, In the case of the DNA sequences encoding SEQ.ID.NOs.:6-9, the DNA sequences do not encode any contiguous stretch of amino acids from the ART protein other than SEQ.ID.NOs.:6-9.

The DNA sequences of the present invention can be in a form that is substantially free from other nucleic acids. "Substantially free from other nucleic acids" means at least 90%, preferably 95%, more preferably 99%, and even more preferably 99.9%, free of other nucleic acids. Thus, a preparation of DNA sequences encoding an ART polypeptide that is substantially free from other nucleic acids will contain, as a percent of its total nucleic acids, no more than 10%, preferably no more than 5%, more preferably no more than 1%, and even more preferably no more than 0.1%, of nucleic acids other than the DNA sequences encoding ART polypeptides. Whether a given preparation of DNA sequences encoding an ART polypeptide is substantially free from other nucleic acids can be determined by such conventional techniques as, *e.g*., agarose gel electrophoresis combined with appropriate staining methods, *e.g*., ethidium bromide staining.

The DNA sequences of the present invention encoding ART polypeptides can be linked with other DNA sequences, *e.g*., DNA sequences to which DNA sequences encoding the ART protein are not naturally linked, to form "recombinant DNA molecules" encoding ART polypeptides. Such other sequences can include DNA sequences that control transcription or translation such as, *e.g*., translation initiation sequences, promoters for RNA polymerase II, transcription or translation termination sequences, enhancer sequences, sequences that control replication in microorganisms, or that confer antibiotic resistance. The DNA sequences of the present invention can be inserted into vectors such as plasmids, cosmids, viral vectors, or yeast artificial chromosomes.

Included in the present invention are DNA sequences that hybridize to the DNA sequences encoding ART polypeptides under stringent conditions. By way of example and not limitation, a procedure using conditions of high stringency is as follows: Prehybridization of filters containing DNA is carried out for 2 hr. to overnight at 65°C in buffer composed of 6X SSC, 5X Denhardt's solution, and 100 µg/ml denatured salmon sperm DNA. Filters are hybridized for 12 to 48 hrs at 65°C in prehybridization mixture containing 100 µg/ml denatured salmon sperm DNA and 5-20 X 10⁶ cpm of ³²P-labeled probe. Washing of filters is done at 37°C for 1 hr in a solution containing 2X SSC, 0.1% SDS. This is followed by a wash in O.1X SSC, 0.1% SDS at 50°C for 45 min. before autoradiography. Other procedures using conditions of high stringency would include either a hybridization carried out in 5XSSC, 5X Denhardt's solution, 50% formamide at 42°C for 12 to 48 hours or a washing step carried out in 0.2X SSPE, 0.2% SDS at 65°C for 30 to 60 minutes.

Reagents mentioned in the foregoing procedures for carrying out high stringency hybridization are well known in the art. Details of the composition of these reagents can be found in, *e.g*., Sambrook, Fritsch, and Maniatis, 1989, Molecular Cloning: A Laboratory Manual, second edition, Cold Spring Harbor Laboratory Press. In addition to the foregoing, other conditions of high stringency which may be used are well known in the art.

Another aspect of the present invention includes host cells that have been engineered to contain and/or express DNA sequences encoding ART polypeptides. Such recombinant host cells can be cultured under suitable conditions to produce ART polypeptides. An expression vector containing DNA encoding ART polypeptides can be used for expression of ART polypeptides in a recombinant host cell. Recombinant host cells may be prokaryotic or eukaryotic, including but not limited to, bacteria such as *E. coli,* fungal cells such as yeast, mammalian cells including, but not limited to, cell lines of human, bovine, porcine, monkey and rodent origin, and insect cells including but not limited to *Drosophila* and silkworm derived cell lines. Cell lines derived from mammalian species which are suitable for recombinant expression of ART polypeptides and which are commercially available, include but are not limited to, L cells L-M(TK-) (ATCC CCL 1.3), L cells L-M (ATCC CCL 1.2), 293 (ATCC CRL 1573), Raji (ATCC CCL 86), CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C127I (ATCC CRL 1616), BS-C-1 (ATCC CCL 26) and MRC-5 (ATCC CCL 171).

A variety of mammalian expression vectors can be used to express ART polypeptides in mammalian cells. Commercially available mammalian expression vectors which are suitable include, but are not limited to, pMC1neo (Stratagene), pSG5 (Stratagene), pcDNAI and pcDNAIamp, pcDNA3, pcDNA3.1, pCR3.1 (Invitrogen), EBO-pSV2-neo (ATCC 37593), pBPV-1(8-2) (ATCC 37110), pdBPV-MMTneo(342-12) (ATCC 37224), pRSVgpt (ATCC 37199), pRSVneo (ATCC 37198), and pSV2-dhfr (ATCC 37146). Following expression in recombinant cells, ART polypeptides can be purified by conventional techniques to a level that is substantially free from other proteins.

The present invention includes a method of determining whether a substance is an inhibitor of the binding of an ART polypeptide to a melanocortin receptor. Such substances are likely to be useful in the control of body weight. The method takes advantage of the fact that ART polypeptides inhibit the binding of melanocyte stimulating hormones to melanocortin receptors by themselves binding to the receptor. Thus, a substance that antagonizes the inhibitory effect of ART polypeptides on the binding of melanocyte stimulating hormones to melanocortin receptors is likely to act by inhibiting the binding of the ART polypeptide itself to the melanoncortin receptor. The method comprises:
(a) providing cells expressing the melanocortin receptor;
(b) exposing the cells to a chosen concentration of the melanocyte stimulating hormone in the absence of the ART polypeptide and in the absence of the substance and measuring the amount of melanocyte stimulating hormone binding to the cells to obtain a first value for melanocyte stimulating hormone binding;
(c) exposing the cells to the chosen concentration of melanocyte stimulating hormone in the presence of a chosen concentration of the ART polypeptide and in the absence of the substance and measuring the amount of melanocyte stimulating hormone binding to obtain a second value for melanocyte stimulating hormone binding where the second value for melanocyte stimulating hormone binding indicates that less melanocyte stimulating hormone binding has occurred as compared to the first value for melanocyte stimulating hormone binding;
(d) exposing the cells to the chosen concentration of melanocyte stimulating hormone in the presence of the chosen concentration of ART polypeptide and in the presence of the substance and measuring the amount of melanocyte stimulating hormone binding to obtain a third value for melanocyte stimulating hormone binding;
where, if the third value for melanocyte stimulating hormone binding is greater than the second value, then the substance is an inhibitor of the binding of the ART polypeptide to the melanocortin receptor.

In a particular embodiment, the cells expressing the melanocortin receptor are cells that naturally express the melanocortin receptor. In another embodiment, the cells expressing the melanocortin receptor do not naturally express the melanocortin receptor but have been transfected with an expression vector that directs the expression of the melanocortin receptor. Transfection is meant to include any method known in the art for the introduction of the the expression vector directing the expression of the melanocortin receptor into the cells. For example, transfection includes calcium phosphate or calcium chloride mediated transfection, lipofection, infection with a retroviral construct containing the melanocortin receptor, and electroporation.

In a particular embodiment, the melanocortin receptor is selected from the group consisting of: the melanocortin-3 receptor (MC3R) and the melanocortin-4 receptor (MC4R). In a particular embodiment of the above-described method, the melanocortin receptor is not a *Xenopus* melanocortin receptor.

The cells that have been transfected with an expression vector that directs the expression of the melanocortin receptor can be prokaryotic cells or eukaryotic cells. In a particular embodiment, the cells that have been transfected with an expression vector that directs the expression of the melanocortin receptor are selected from the group consisting of: yeast cells, mammalian cells, bacterial cells, and insect cells. In a particular embodiment, the cells that have been transfected with an expression vector that directs the expression of the melanocortin receptor are selected from the group consisting of: human cells, mouse cells, rat cells, bovine cells, porcine cells, hamster cells, and monkey cells. In a particular embodiment, the cells that have been transfected with an expression vector that directs the expression of the melanocortin receptor are selected from the group consisting of: L cells L-M(TK-) (ATCC CCL 1.3), L cells L-M (ATCC CCL 1.2), 293 cells (ATCC CRL 1573), Raji cells (ATCC CCL 86), CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C127I (ATCC CRL 1616), BS-C-1 (ATCC CCL 26) and MRC-5 (ATCC CCL 171). In a particular embodiment, the cells are not *Xenopus* melanophore cells.

In a particular embodiment, the melanocyte stimulating hormone is selected from the group consisting of: α-melanocyte stimulating hormone, β-melanocyte stimulating hormone, and γ-melanocyte stimulating hormone.

In a particular embodiment, the ART polypeptide has an amino acid sequence selected from the group consisting of: SEQ.ID.NOs. 1-19 and 20.

In particular embodiments of the above-described method, the method is practiced *in vitro* and the conditions under which the method is practiced are conditions that are typically used in the art for the study of protein-protein interactions: *e.g*., physiological pH; salt conditions such as those represented by such commonly used buffers as PBS or in tissue culture media; a temperature of about 4°C to about 55°C.

In particular embodiments of the above-described method, the chosen concentration of the melanocyte stimulating hormone is from 0.05 nM to 2.0 nM, preferably from 0.1 nM to 1.0 nM, and more preferably from 0.2 nM to 0.5 nM.

In particular embodiments of the above-described method, the chosen concentration of the ART polypeptide is from 10-12 M to 10-7 M.

In particular embodiments of the above-described method, the method is practiced *in vitro* and the melanocyte stimulating hormone is labeled, *e.g*., enzymatically, radioactively, or the like, and the amount of binding of the melanocyte stimulating hormone to the melanocortin receptor is measured by determining the amount of label bound to the cells containing the melanocortin receptor.

Steps (b), (c), and (d) of the above-described method can be modified in that, rather than exposing intact cells to the melanocyte stimulating hormone, the ART polypeptide, or the substance, membranes can be prepared from the cells and the membranes can be exposed to the melanocyte stimulating hormone, the ART polypeptide, or the substance. Such a modification utilizing membranes rather than intact cells in methods similar to that described above, although directed to the binding interactions of other ligands and receptors, is well known in the art and is described in, *e.g.,* Hess et al, 1992, Biochem. Biophys. Res. Comm. 184:260-268.

As a further modification of the above-described method, RNA encoding the melanocortin receptor can be prepared as, *e.g.,* by in *vitro* transcription using a plasmid containing nucleotide sequences encoding the melanocortin receptor under the control of a bacteriophage T7 promoter, and the RNA can be microinjected into *Xenopus* oocytes in order to cause the expression of the melanocortin receptor in the oocytes. These oocytes then take the place of the cells in the above described method.

Once a substance has been identified as an inhibitor of the binding of the the ART polypeptide to the melanocortin receptor, that substance can be tested to determine whether it is also an agonist of the melanocortin receptor. Such testing would involve exposing cells that express the melanocortin receptor to the substance, in the absence of the melanocyte stimulating hormone and the ART protein or ART polypeptides, and determining whether the melanocortin receptor is thereby activated by the substance. In this way, an inhibitor of the effect of ART protein on MC3R or MC4R can be identified that has no, or little, MC3R or MC4R agonist activity, but that relieves the inhibition of MC3R or MC4R receptor activity produced by ART protein. In a similar manner, it can be determined whether the substance is an antagonist of the melanocortin receptor.

The present invention also includes a method for determining whether a substance is an inhibitor of the binding of an ART polypeptide to a melanocortin receptor where the method comprises:
(a) providing cells expressing a melanocortin receptor;
(b) exposing the cells to an ART polypeptide in the presence and in the absence of the substance under conditions such that if the substance were not present, the ART polypeptide would bind to the melanocortin receptor;
(c) measuring the amount of binding of the ART polypeptide to the melanocortin receptor in the presence and in the absence of the substance;
where a decrease in the amount of binding of the ART polypeptide to the melanocortin receptor in the presence as compared to the absence of the substance indicates that the substance is an inhibitor of the binding of the ART polypeptide to the melanocortin receptor.

In a particular embodiment, the cells expressing the melanocortin receptor are cells that naturally express the melanocortin receptor. In another embodiment, the cells expressing the melanocortin receptor do not naturally express the melanocortin receptor but have been transfected with an expression vector that directs the expression of the melanocortin receptor. Transfection is meant to include any method known in the art for the introduction of the the expression vector directing the expression of the melanocortin receptor into the cells. For example, transfection includes calcium phosphate or calcium chloride mediated transfection, lipofection, infection with a retroviral construct containing the melanocortin receptor, and electroporation.

In a particular embodiment, the melanocortin receptor is selected from the group consisting of: the melanocortin-3 receptor (MC3R) and the melanocortin-4 receptor (MC4R). In a particular embodiment of the above-described method, the melanocortin receptor is not a *Xenopus* melanocortin receptor.

The cells that have been transfected with an expression vector that directs the expression of the melanocortin receptor can be prokaryotic cells or eukaryotic cells. In a particular embodiment, the cells that have been transfected with an expression vector that directs the expression of the melanocortin receptor are selected from the group consisting of: yeast cells, mammalian cells, bacterial cells, and insect cells. In a particular embodiment, the cells that have been transfected with an expression vector that directs the expression of the melanocortin receptor are selected from the group consisting of: human cells, mouse cells, rat cells, bovine cells, porcine cells, hamster cells, and monkey cells. In a particular embodiment, the cells that have been transfected with an expression vector that directs the expression of the melanocortin receptor are selected from the group consisting of: L cells L-M(TK⁻) (ATCC CCL 1.3), L cells L-M (ATCC CCL 1.2), 293 (ATCC CRL 1573), Raji (ATCC CCL 86), CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C1271 (ATCC CRL 1616), BS-C-1 (ATCC CCL 26) and MRC-5 (ATCC CCL 171). In a particular embodiment, the cells are not *Xenopus* melanophore cells.

In a particular embodiment, the ART polypeptide has an amino acid sequence selected from the group consisting of:
SEQ.ID.NOs.l-19 and 20. In particular embodiments of the above-described method, the ART polypeptide is used in a concentration of from 10-12 M to 10⁻⁷ M.

In particular embodiments of the above-described method, the method is practiced *in vitro* and the conditions are conditions that are typically used in the art for the study of protein-protein interactions: *e.g.,* physiological pH; salt conditions such as those represented by such commonly used buffers as PBS or in tissue culture media; a temperature of about 4°C to about 55°C.

In particular embodiments of the above-described method, the method is practiced in *vitro* and the ART polypeptide is labeled, *e.g.,* enzymatically, radioactively, or the like, and the amount of binding of the ART polypeptide to the melanocortin receptor is measured by determining the amount of label bound to the melanocortin receptor. The ART polypeptide will either be radioactively labeled by ³²P, ³³P, or ¹²⁵I (*e.g.,* for c-ART-a or c-ART-c), or non-radioactively labeled (*e.g.,* ART-AP, ART-luc, c-ART-AP or c-ART-luc). In the case of these latter . ART polypeptides, the ART polypeptides can be detected by detecting the enzymatic activity of the alkaline phosphatase or luciferase moieties of the polypeptides.

Step (b) of the above-described method can be modified in that, rather than exposing the cells to an ART polypeptide in the presence and in the absence of the substance, membranes can be prepared from the cells and the membranes can be exposed to an ART polypeptide in the presence and in the absence of the substance. Such a modification utilizing membranes rather than cells in methods similar to that described above, although directed to the binding interactions of other ligands and receptors, is well known in the art and is described in, *e.g.,* Hess et al, 1992, Biochem. Biophys. Res. Comm. 184:260-268.

As a further modification of the above-described method, RNA encoding a melanocortin receptor can be prepared as, *e.g.,* by in *vitro* transcription using a plasmid containing nucleotide sequences encoding a melanocortin receptor under the control of a bacteriophage T7 promoter, and the RNA can be microinjected into *Xenopus* oocytes in order to cause the expression of the melanocortin receptor in the oocytes. These oocytes then take the place of the cells in the above described method.

Once a substance has been identified as an inhibitor of ART binding to a melanocortin receptor, that substance can be tested to determine whether it is also an agonist of the melanocortin receptor. Such testing would involve exposing cells that express the melanocortin receptor to the substance, in the absence of ART protein or ART polypeptides, and determining whether the melanocortin receptor is thereby activated by the substance. In this way, an inhibitor of ART protein binding to MC3R or MC4R may be identified that has no, or little, MC3R or MC4R agonist activity, but that relieves the inhibition of MC3R or MC4R receptor activity produced by ART protein.

The present invention also includes a method for determining whether a substance is an allosteric enhancer of the binding of an ART polypeptide to a melanocortin receptor where the method comprises:
(a) providing cells expressing a melanocortin receptor;
(b) exposing the cells to an ART polypeptide in the presence and in the absence of the substance under conditions such that if the substance were not present, the ART polypeptide would bind to the melanocortin receptor;
(c) measuring the amount of binding of the ART polypeptide to the melanocortin receptor in the presence and in the absence of the substance;
where an increase in the amount of binding of the ART polypeptide to the melanocortin receptor in the presence as compared to the absence of the substance indicates that the substance is an allosteric enhancer of the binding of the ART polypeptide to the melanocortin receptor.

In a particular embodiment, the cells expressing the melanocortin receptor are cells that naturally express the melanocortin receptor. In another embodiment, the cells expressing the melanocortin receptor do not naturally express the melanocortin receptor but have been transfected with an expression vector that directs the expression of the melanocortin receptor. Transfection is meant to include any method known in the art for the introduction of the the expression vector directing the expression of the melanocortin receptor into the cells. For example, transfection includes calcium phosphate or calcium chloride mediated transfection, lipofection, infection with a retroviral construct containing the melanocortin receptor, and electroporation.

In a particular embodiment, the melanocortin receptor is selected from the group consisting of: the melanocortin-3 receptor (MC3R) and the melanocortin-4 receptor (MC4R). In a particular embodiment of the above-described method, the melanocortin receptor is not a *Xenopus* melanocortin receptor.

The cells that have been transfected with an expression vector that directs the expression of the melanocortin receptor can be prokaryotic cells or eukaryotic cells. In a particular embodiment, the cells that have been transfected with an expression vector that directs the expression of the melanocortin receptor are selected from the group consisting of: yeast cells, mammalian cells, bacterial cells, and insect cells. In a particular embodiment, the cells that have been transfected with an expression vector that directs the expression of the melanocortin receptor are selected from the group consisting of: human cells, mouse cells, rat cells, bovine cells, porcine cells, hamster cells, and monkey cells. In a particular embodiment, the cells that have been transfected with an expression vector that directs the expression of the melanocortin receptor are selected from the group consisting of: L cells L-M(TK-) (ATCC CCL 1.3), L cells L-M (ATCC CCL 1.2), 293 (ATCC CRL 1573), Raji (ATCC CCL 86), CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C127I (ATCC CRL 1616), BS-C-1 (ATCC CCL 26) and MRC-5 (ATCC CCL 171). In a particular embodiment, the cells are not *Xenopus* melanophore cells.

In a particular embodiment, the ART polypeptide has an amino acid sequence selected from the group consisting of:
SEQ.ID.NOs.1-19 and 20. In particular embodiments of the above-described method, the ART polypeptide is used in a concentration of from 10⁻¹² M to 10⁻⁷ M.

In particular embodiments of the above-described method, the method is practiced *in vitro* and the conditions are conditions that are typically used in the art for the study of protein-protein interactions: *e.g*., physiological pH; salt conditions such as those represented by such commonly used buffers as PBS or in tissue culture media; a temperature of about 4°C to about 55°C.

In particular embodiments of the above-described method, the method is practiced *in vitro* and the ART polypeptide is labeled, *e.g.,* enzymatically, radioactively, or the like, and the amount of binding of the ART polypeptide to the melanocortin receptor is measured by determining the amount of label bound to the melanocortin receptor.

Step (b) of the above-described method can be modified in that, rather than exposing the cells to an ART polypeptide in the presence and in the absence of the substance, membranes can be prepared from the cells and the membranes can be exposed to an ART polypeptide in the presence and in the absence of the substance. Such a modification utilizing membranes rather than cells in methods similar to that described above, although directed to the binding interactions of other ligands and receptors, is well known in the art and is described in, *e.g.,* Hess et al, 1992, Biochem. Biophys. Res. Comm. 184:260-268.

As a further modification of the above-described method, RNA encoding a melanocortin receptor can be prepared as, *e.g.,* by in *vitro* transcription using a plasmid containing nucleotide sequences encoding a melanocortin receptor under the control of a bacteriophage T7 promoter, and the RNA can be microinjected into *Xenopus* oocytes in order to cause the expression of the melanocortin receptor in the oocytes. These oocytes then take the place of the cells in the above described method.

Melanocortin receptors are G-protein coupled receptors that stimulate Gₛ, leading to the production of cAMP (Cone et al., 1996, Recent Prog. Hormone Res. 51:287-318). Thus, the ART polypeptides of the present invention can be used in a method for determining whether a substance is a functional inhibitor of the antagonistic effect of an ART polypeptide on a melanocortin receptor where the method comprises:
(a) providing cells expressing a melanocortin receptor;
(b) exposing the cells to a melanocyte stimulating hormone, thereby activating the melanocortin receptor and leading to the production of cAMP mediated by the melanocortin receptor;
(c) exposing the cells to an ART polypeptide in the presence and in the absence of the substance under conditions such that if the substance were not present, the ART polypeptide would inhibit the production of cAMP mediated by the melanocortin receptor;
(d) measuring the amount of cAMP produced the presence and in the absence of the substance;
where an increase in the amount of cAMP produced in the presence as compared to the absence of the substance indicates that the substance is a functional inhibitor of the antagonistic effect of the ART polypeptide on the melanocortin receptor.

In a particular embodiment, the cells expressing the melanocortin receptor are cells that naturally express the melanocortin receptor. In another embodiment, the cells expressing the melanocortin receptor do not naturally express the melanocortin receptor but have been transfected with an expression vector that directs the expression of the melanocortin receptor. Transfection is meant to include any method known in the art for the introduction of the the expression vector directing the expression of the melanocortin receptor into the cells. For example, transfection includes calcium phosphate or calcium chloride mediated transfection, lipofection, infection with a retroviral construct containing the melanocortin receptor, and electroporation.

In a particular embodiment, the melanocortin receptor is selected from the group consisting of: the melanocortin-3 receptor (MC3R) and the melanocortin-4 receptor (MC4R). In a particular embodiment of the above-described method, the melanocortin receptor is not a *Xenopus* melanocortin receptor.

The cells that have been transfected with an expression vector that directs the expression of the melanocortin receptor can be prokaryotic cells or eukaryotic cells. In a particular embodiment, the cells that have been transfected with an expression vector that directs the expression of the melanocortin receptor are selected from the group consisting of: yeast cells, mammalian cells, bacterial cells, and insect cells. In a particular embodiment, the cells that have been transfected with an expression vector that directs the expression of the melanocortin receptor are selected from the group consisting of: human cells, mouse cells, rat cells, bovine cells, porcine cells, hamster cells, and monkey cells. In a particular embodiment, the cells that have been transfected with an expression vector that directs the expression of the melanocortin receptor are selected from the group consisting of: L cells L-M(TK-) (ATCC CCL 1.3), L cells L-M (ATCC CCL 1.2), 293 (ATCC CRL 1573), Raji (ATCC CCL 86), CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C127I (ATCC CRL 1616), BS-C-1 (ATCC CCL 26) and MRC-5 (ATCC CCL 171). In a particular embodiment, the cells are not *Xenopus* melanophore cells.

In a particular embodiment, the melanocyte stimulating hormone is selected from the group consisting of: α-melanocyte stimulating hormone, β-melanocyte stimulating hormone, and γ-melanocyte stimulating hormone.

In a particular embodiment, the ART polypeptide has an amino acid sequence selected from the group consisting of:
SEQ.ID.NOs.1-19 and 20.

In particular embodiments of the above-described method, the method is practiced *in vitro* and the conditions are conditions that are typically used in the art for the study of protein-protein interactions: *e.g*., physiological pH; salt conditions such as those represented by such commonly used buffers as PBS or in tissue culture media; a temperature of about 4°C to about 55°C.

Once a substance has been identified as a functional inhibitor of the antagonistic effect of an ART polypeptide on a melanocortin receptor, that substance can be tested to determine whether it is also an agonist of the melanocortin receptor. Such testing would involve exposing cells that express the melanocortin receptor to the substance, in the absence of ART protein or ART polypeptides, and determining whether the melanocortin receptor is thereby activated by the substance. In this way, an inhibitor of ART protein binding to MC3R or MC4R may be developed that has no, or little, MC3R or MC4R agonist activity, but that relieves the inhibition of MC3R or MC4R receptor activity produced by ART protein. In a similar manner, it can be determined whether the substance is an antagonist of the melanocortin receptor.

The ART polypeptides of the present invention can also be used in a method of determining whether a substance is an inhibitor of the effect of an ART polypeptide that makes use of an assay utilizing a *Xenopus* melanophore cell line (see, *e.g.,* Quillan et al., 1995, Proc. Natl. Acad. Sci. USA 92:2894; Potenza & Lerner, 1992, Pigment Cell Res. 5:372; Ollman et al., 1997, Science 278:135-138). Such a method comprises:
(a) providing a *Xenopus* melanophore cell line;
(b) exposing the *Xenopus* melanophore cell line to a chosen concentration of a melanocyte stimulating hormone in the absence of the ART polypeptide and in the absence of the substance and measuring the amount of pigment dispersion to obtain a first value for pigment dispersion;
(c) exposing the *Xenopus* melanophore cell line to the chosen concentration of α-melanocyte stimulating hormone in the presence of the ART polypeptide and in the absence of the substance and measuring the amount of pigment dispersion to obtain a second value for pigment dispersion where the second value for pigment dispersion indicates that less pigment has been dispersed as compared to the first value for pigment dispersion;
(d) exposing the *Xenopus* melanophore cell line to the chosen concentration of α-melanocyte stimulating hormone in the presence of the ART polypeptide and in the presence of the substance and measuring the amount of pigment dispersion to obtain a third value for pigment dispersion;
where if the third value for pigment dispersion indicates that more pigment has been dispersed as compared with the second value, then the substance is an inhibitor of the effect of the ART polypeptide.

In a particular embodiment, the melanocyte stimulating hormone is selected from the group consisting of: α-melanocyte stimulating hormone, β-melanocyte stimulating hormone, and γ-melanocyte stimulating hormone.

In a particular embodiment, the ART polypeptide has an amino acid sequence selected from the group consisting of:
SEQ.ID.NOs.1-19 and 20.

In particular embodiments of the above-described method, the conditions are conditions that are typically used in the art for the study of protein-protein interactions: *e.g.,* physiological pH; salt conditions such as those represented by such commonly used buffers as PBS or in tissue culture media; a temperature of about 4°C to about 55°C.

Once a substance has been identified as an inhibitor of the effect of an ART polypeptide, that substance can be tested to determine whether it is also an agonist of the *Xenopus* melanocortin receptor. Such testing would involve exposing melanophore cells that express the Xenopus melanocortin receptor to the substance, in the absence of ART protein or ART polypeptides, and determining whether the melanocortin receptor is thereby activated by the substance. In this way, an inhibitor of ART protein binding to the *Xenopus* melanocortin receptor can be identified that may be used as a lead to develop ART binding inhibitors for human MC3R or MC4R that have no, or little, MC3R or MC4R agonist activity, but that relieve the inhibition of MC3R or MC4R receptor activity produced by ART protein. In a similar manner, it can be determined whether the substance is an antagonist of the melanocortin receptor.

The present invention includes a method of determining whether a substance is an inhibitor of the binding of an ART polypeptide to a melanocortin receptor comprising:
(a) providing cells expressing the melanocortin receptor;
(b) exposing the cells to a chosen concentration of the melanocyte stimulating hormone and a chosen concentration of the ART polypeptide in the presence and in the absence of the substance and measuring the amount of melanocyte stimulating hormone binding to the cells in the presence and in the absence of the substance;
where an increase in the amount of melanocyte stimulating hormone binding in the presence of the substance indicates that the substance is an inhibitor of the binding of an ART polypeptide to a melanocortin receptor.

In a particular embodiment, the cells expressing the melanocortin receptor are cells that naturally express the melanocortin receptor. In another embodiment, the cells expressing the melanocortin receptor do not naturally express the melanocortin receptor but have been transfected with an expression vector that directs the expression of the melanocortin receptor. Transfection is meant to include any method known in the art for the introduction of the the expression vector directing the expression of the melanocortin receptor into the cells. For example, transfection includes calcium phosphate or calcium chloride mediated transfection, lipofection, infection with a retroviral construct containing the melanocortin receptor, and electroporation.

In a particular embodiment, the melanocortin receptor is selected from the group consisting of the melanocortin-3 receptor (MC3R) and the melanocortin-4 receptor (MC4R). In a particular embodiment of the above-described method, the melanocortin receptor is not a *Xenopus* melanocortin receptor.

The cells that have been transfected with an expression vector that directs the expression of the melanocortin receptor can be prokaryotic cells or eukaryotic cells. In a particular embodiment, the cells that have been transfected with an expression vector that directs the expression of the melanocortin receptor are selected from the group consisting of yeast cells, mammalian cells, bacterial cells, and insect cells. In a particular embodiment, the cells that have been transfected with an expression vector that directs the expression of the melanocortin receptor are selected from the group consisting of: human cells, mouse cells, rat cells, bovine cells, porcine cells, hamster cells, and monkey cells. In a particular embodiment, the cells that have been transfected with an expression vector that directs the expression of the melanocortin receptor are selected from the group consisting of: L cells L-M(TK-) (ATCC CCL 1.3), L cells L-M (ATCC CCL 1.2), 293 cells (ATCC CRL 1573), Raji cells (ATCC CCL 86), CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C127I (ATCC CRL 1616), BS-C-1 (ATCC CCL 26) and MRC-5 (ATCC CCL 171). In a particular embodiment, the cells are not *Xenopus* melanophore cells.

In a particular embodiment, the melanocyte stimulating hormone is selected from the group consisting of: α-melanocyte stimulating hormone, β-melanocyte stimulating hormone, and γ-melanocyte stimulating hormone.

In a particular embodiment, the ART polypeptide has an amino acid sequence selected from the group consisting of:
SEQ.ID.NOs.1-19 and 20.

In particular embodiments of the above-described method, the method is practiced *in vitro* and the conditions under which the method is practiced are conditions that are typically used in the art for the study of protein-protein interactions: *e.g*. , physiological pH; salt conditions such as those represented by such commonly used buffers as PBS or in tissue culture media; a temperature of about 4°C to about 55°C.

In particular embodiments of the above-described method, the chosen concentration of the melanocyte stimulating hormone is from 0.05 nM to 2.0 nM, preferably from 0.1 nM to 1.0 nM, and more preferably from 0.2 nM to 0.5 nM.

In particular embodiments of the above-described method, the chosen concentration of the ART polypeptide is from 10-12 M to 10⁻⁷ M.

In particular embodiments of the above-described method, the method is practiced *in vitro* and the melanocyte stimulating hormone is labeled, *e.g*., enzymatically, radioactively, or the like, and the amount of binding of the melanocyte stimulating hormone to the melanocortin receptor is measured by determining the amount of label bound to the cells containing the melanocortin receptor.

Step (b) of the above-described method can be modified in that, rather than exposing intact cells to the melanocyte stimulating hormone, the ART polypeptide, or the substance, membranes can be prepared from the cells and the membranes can be exposed to the melanocyte stimulating hormone, the ART polypeptide, or the substance. Such a modification utilizing membranes rather than intact cells in methods similar to that described above, although directed to the binding interactions of other ligands and receptors, is well known in the art and is described in, *e.g.,* Hess et al, 1992, Biochem. Biophys. Res. Comm. 184:260-268.

As a further modification of the above-described method, RNA encoding the melanocortin receptor can be prepared as, *e.g.,* by in *vitro* transcription using a plasmid containing nucleotide sequences encoding the melanocortin receptor under the control of a bacteriophage T7 promoter, and the RNA can be microinjected into *Xenopus* oocytes in order to cause the expression of the melanocortin receptor in the oocytes. These oocytes then take the place of the cells in the above described method.

Once a substance has been identified as an inhibitor of the binding of the the ART polypeptide to the melanocortin receptor, that substance can be tested to determine whether it is also an agonist of the melanocortin receptor. Such testing would involve exposing cells that express the melanocortin receptor to the substance, in the absence of the melanocyte stimulating hormone and the ART protein or ART polypeptides, and determining whether the melanocortin receptor is thereby activated by the substance. In this way, an inhibitor of the effect of ART protein on MC3R or MC4R can be identified that has no, or little, MC3R or MC4R agonist activity, but that relieves the inhibition of MC3R or MC4R receptor activity produced by ART protein. In a similar manner, it can be determined whether the substance is an antagonist of the melanocortin receptor.

Compared to full-length ART protein, the ART polypeptides of the present invention are smaller, and therefore easier to produce and less likely to be degraded. With respect to such embodiments of the invention as, *e.g*., c-ART-b, the non-ART protein amino acid sequences added to the C-terminus of the ART sequences do not impair binding or functional activity, and allow ³²P or ³³P labeling without the need to label the ART sequence. Fusion polypeptides such as, *e.g*., ART-AP or ART-luc, allow the use of non-radioactive methods to detect ART polypeptides in binding assays.

That the ART polypeptides of the present invention having amino acid sequences from non-ART proteins at their C-terminus are functional is surprising. The C-terminus of ART protein is homologous to the C-terminus of the agouti protein, both the ART protein and the agouti protein having a characteristic pattern of cysteine residues in this region. A similar pattern of cysteine residues has been found in certain ion channel blockers from spider and snail toxins. This pattern of cysteines has been proposed to result in the formation of specific disulfide bridges that constrain the toxins into a characteristic three-dimensional structure that is responsible for the toxins' biological activity (Kim et al., 1995, J. Mol. Biol. 250:659-671; hereinafter "Kim"). While the extreme C-terminal amino acids of the toxin studied by Kim were not part of this three-dimensional structure, these extreme C-terminal amino acids were nevertheless "crucially important," since altering them resulted in a loss of activity. See page 665, right column of Kim: "These results suggest that the C-terminal segment of ω-AGA-IVA is crucially important for its blocking action on the P-type calcium channel expressed in rat cerebellar Purkinje cells." Thus, one would have expected that altering the C-terminus of the ART protein, *e.g*., by linking it to sequences from a non-ART protein, would have resulted in an ART fusion polypeptide which would lack the activity of the full-length ART protein, or at least show substantially less activity. The present invention demonstrates that this is not so.

The following non-limiting examples are presented to better illustrate the invention.

### EXAMPLE 1

### Production of a construct expressing c-ART-b

The expression plasmid for c-ART-b was constructed by modifying the ART expression plasmid which was generated by inserting the ART cDNA into the EcoRI and BamHI sites of pcDNA3.1-Myc-His-A (Fong et al., 1997, Biochem. Biophys. Res. Comm. 237:629-631; hereinafter "Fong"). The c-ART-b sequence differs from that of the recombinant ART as described by Fong in that residues 27-75 were deleted from ART. To accomplish that, a first PCR was carried out using the ART expression plasmid as template and two oligos (GGGCTCGGCGGTCCTGCAGGGCCAAGCCCATCTGGGC (SEQ.ID.NO.:21); and the T7 primer TAATACGACTCACTATAGGG (SEQ.ID.NO.:22)) to amplify the DNA fragment encoding ART residues 1-26 followed immediately by residues 76-81. A second PCR was carried out using the ART expression plasmid as template and two other oligos (CTGCAGGACCGCGAGCCC (SEQ.ID.NO.:23); and the pcDNA3.1A primer GTCGACGGCGCTATTCAG (SEQ.ID.NO.:24)) to amplify the DNA fragment encoding ART residues 76-132. A third PCR was then carried out using the first and the second PCR products as template and two oligos (the T7 primer and the pcDNA3.1A primer) to amplify the c-ART-b cDNA. The final PCR product was cleaved by the restriction enzymes EcoRI and BamHI, and ligated to the pcDNA3.1-Myc-His-A vector similarly cleaved by EcoRI and BamHI. The thrombin site sequence was based on the thrombin site in pET-34b (Novagen, Milwaukee, WI). The Myc epitope sequence and hexahistine sequence were contained within the pcDNA3.1-Myc-His-A vector (Invitrogen, Carlsbad, CA).

### EXAMPLE 2

### Expression of c-ART-b

COS-7 cells in T-175 flasks were transiently transfected with the c-ART-b expression plasmid (24 µg) by lipofectamine (Gibco), and grown in Opti-mem media (Gibco) supplemented with 1% fetal bovine serum. Two days after transfection, culture media were collected, centrifuged to remove residual cells, concentrated about 100-fold in Centriprep-3 (Amicon) and stored at 4 °C in the presence of 2.5 mM EGTA, 4 mg/ml leupeptin, and 0.01 mM phosphoramidon. After determination of the concentration of c-ART-b, NaN3 was added to 0.02%. Determination of the concentration of c-ART-b, which contains the Myc sequence, was based on an ELISA standard curve. Briefly, the microtiter plate was coated with 0.2 µg of a Myc peptide (human c-Myc 408-439) overnight, washed, blocked, and followed by incubation with anti-Myc mAb-HRP conjugates (Invitrogen) in the presence of varying concentrations of the free Myc peptide for 2 hours. The bound mAb-HRP was detected using a colorimetric substrate tetramethylbenzidine (BioRad). For c-ART-b concentration determination, a c-ART-b sample replaced the free Myc peptide.

### EXAMPLE 3

### Binding of c-ART-b to MC3R and MC4R

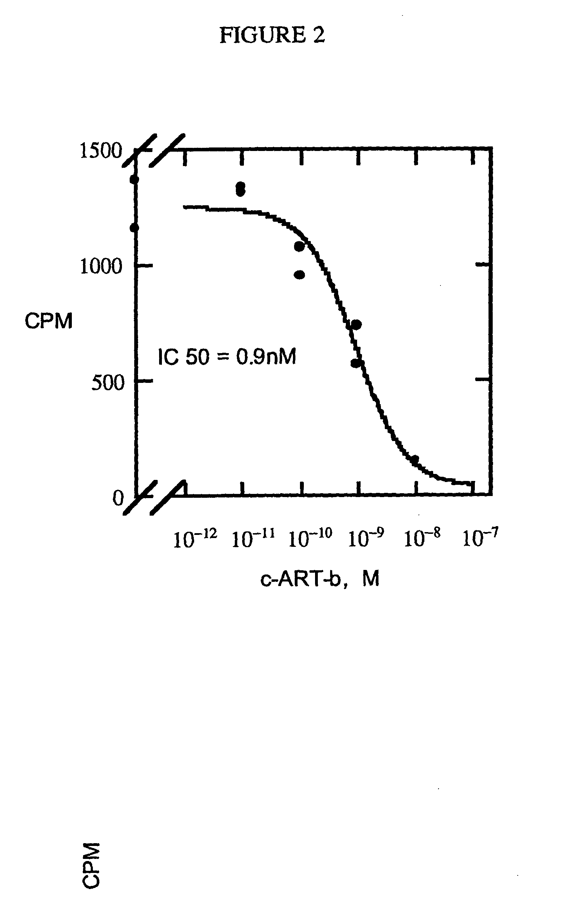

Binding assays were done in the same manner as described in Fong et al., 1997, Biochem. Biophys. Res. Comm. 237:629-631. Binding assays were carried out using membranes prepared from L cells or CHO cells stably expressing human MC3R, MC4R or MC5R. The binding assay mixture contained 0.2 nM of ¹²⁵I-[Tyr²]Nle⁴, D-Phe7] α-melanocyte stimulating hormone (¹²⁵I -NDP-α-MSH), varying concentrations of c-ART-b or full-length ART protein, and an appropriate amount of membranes so that the total bound radioligand was less than 10% of the added radioligand. The above mixture in binding buffer (50 mM Tris, 2 mM CaCl₂, 1 mM MgCl₂, 5 mM KCl, pH 7.2) was incubated at room temperature for 2 hours, followed by filtration through GFC paper. The bound ligand was quantitated in a γ counter. IC₅₀ values were calculated as previously described (Fong et a/., 1996, Mol. Pharmacol. 50:1605-1611). The results are shown in Figure 2. From Figure 2 it can be seen that c-ART-b inhibits the binding of ¹²⁵I-NDP-α-MSH to MC3R.

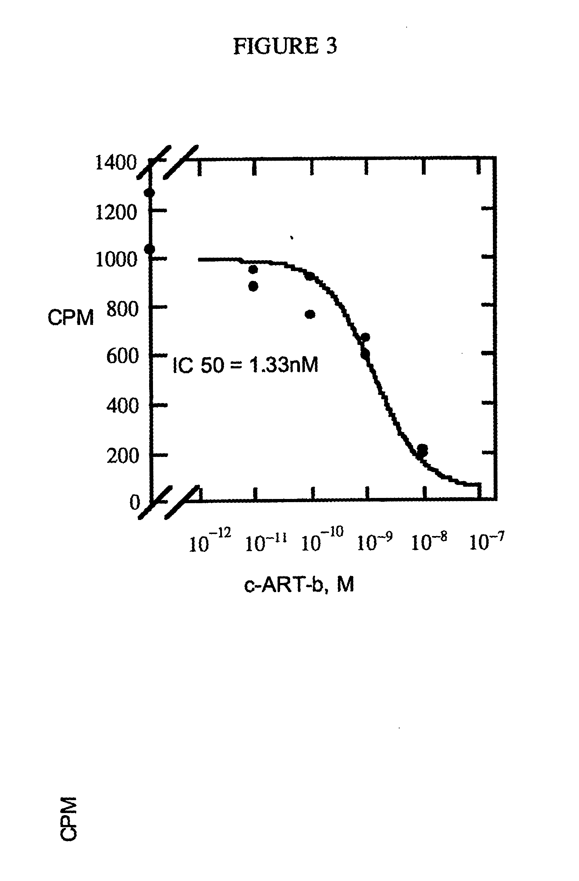

A similar experiment was done to determine whether c-ART-b inhibits the binding of ¹²⁵I-labeled NDP-α-MSH to MC4R. The results are shown in Figure 3. From Figure 3 it can be seen that c-ART-b inhibits the binding of ¹²⁵I-NDP-α-MSH to hMC4R.

Similar experiments were performed with full-length human ART protein. Similar experiments were also performed with full-length ART protein and with c-ART-b for the melanocortin-5 receptor (MC5R). From these experiments, from the results shown in Figures 2 and 3, and from similar experiments, the following IC₅₀ values for the inhibition of 1251-labeled NDP-α-MSH to MC3R, MC4R, and MC5R by full-length ART protein and by c-ART-b can be determined.

**Table 1**

| | hMC3R | hMC4R | hMC5R |
|---|---|---|---|
| full length ART | 1.0Ò0.4 (4) | 0.5Ò0.1 (3) | >40 |
| c-ART-b | 1.9Ò1.0 (2) | 1.4Ò0.1 (2) | not done |

The IC₅₀ values shown in Table 1 are given in nM. The numbers in parentheses represent the number of experiments run. The results shown in Table 1 indicate that, surprisingly, c-ART-b, although missing a significant amount of sequence from the N-terminus of the ART protein, is essentially functionally equivalent to full length ART protein. In addition, c-ART-b is functional despite having a significant amount of non-ART sequences at its C-terminus (a thrombin site, a myc epitope, and a hexahistidine tag).

### EXAMPLE 4

### Functional assay for the binding of c-ART-b to MC3R and MC4R

The ability of c-AR,T-b to inhibit the production of cAMP by α-melanocyte stimulating hormone acting through MC3R or MC4R can be demonstrated by preincubating L Cells stably expressing human MC3R or MC4R with c-AR,T-b for 10 minutes, followed by incubation with 20 nM α-melanocyte stimulating hormone for 45 minutes. The incubation buffer also contains Earle's balanced salt solution, 10 mM HEPES, 5 mM MgCl₂, 1 mg/ml BSA and 0.5 mM IBMX. Following the incubation, cells are lysed by boiling for 4 minutes. Intracellular cAMP concentration is measured by RIA (Huang et al., 1997, J. Receptor Signal Transduc. Res. 17:599-607) using anti-cAMP antibody and ¹²⁵I-cAMP as modified in the scintillation proximity assay format (Amersham).

The present invention is not to be limited in scope by the specific embodiments described herein. Indeed, various modifications of the invention in addition to those described herein will become apparent to those skilled in the art from the foregoing description.

## Claims

1. A fusion protein having an amino acid sequence selected from the group consisting of SEQ. ID. NOs: 1-3, 10-12, 15, and 16.

2. A DNA, sequence encoding the fusion protein of Claim 1.

3. A method of determining whether a substance is an inhibitor of the binding of an ART polypeptide to a melanocortin receptor where the method comprises:
(a) providing cells expressing the melanocortin receptor;
(b) exposing the cells to a chosen concentration of the melanocyte stimulating hormone in the absence of the ART polypeptide and in the absence of the substance and measuring the amount of melanocyte stimulating hormone binding to the cells to obtain a first value for melanocyte stimulating hormone binding;
(c) exposing the cells to the chosen concentration of melanocyte stimulating hormone in the presence of a chosen concentration of the ART polypeptide and in the absence of the substance and measuring the amount of melanocyte stimulating hormone binding to obtain a second value for melanocyte stimulating hormone binding where the second value for melanocyte stimulating hormone binding indicates that less melanocyte stimulating hormone binding has occurred as compared to the first value for melanocyte stimulating hormone binding;
(d) exposing the cells to the chosen concentration of melanocyte stimulating hormone in the presence of the chosen concentration of ART polypeptide and in the presence of the substance and measuring the amount of melanocyte stimulating hormone binding to obtain a third value for melanocyte stimulating hormone binding;
where, if the third value for melanocyte stimulating hormone binding is greater than the second value, then the substance is an inhibitor of the binding of the ART polypeptide to the melanocortin receptor;
where the ART polypeptide is the fusion protein of Claim 1.

4. The method of Claim 3 where the melanocortin receptor is selected from the group consisting of: the melanocortin-3 receptor (MC3R) and the melanocortin-4 receptor (MC4R).

5. A method for determining whether a substance is an inhibitor of the binding of an ART polypeptide to a melanocortin receptor where the method comprises:
(a) providing cells expressing a melanocortin receptor;
(b) exposing the cells to an ART polypeptide in the presence and in the absence of the substance under conditions such that if the substance were not present, the ART polypeptide would bind to the melanocortin receptor;
(c) measuring the amount of binding of the ART polypeptide to the melanocortin receptor in the presence and in the absence of the substance;
where a decrease in the amount of binding of the ART polypeptide to the melanocortin receptor in the presence as compared to the absence of the substance indicates that the substance is an inhibitor of the binding of the ART polypeptide to the melanocortin receptor;
where the ART polypeptide is the fusion protein of Claim 1.

6. The method of Claim 5 where the melanocortin receptor is selected from the group consisting of: the melanocortin-3 receptor (MC3R) and the melanocortin-4 receptor (MC4R).

7. A method for determining whether a substance is an allosteric enhancer of the binding of an ART polypeptide to a melanocortin receptor where the method comprises:
(a) providing cells expressing a melanocortin receptor;
(b) exposing the cells to an ART polypeptide in the presence and in the absence of the substance under conditions such that if the substance were not present, the ART polypeptide would bind to the melanocortin receptor;
(c) measuring the amount of binding of the ART polypeptide to the melanocortin receptor in the presence and in the absence of the substance;
where an increase in the amount of binding of the ART polypeptide to the melanocortin receptor in the presence as compared to the absence of the substance indicates that the substance is an allosteric enhancer of the binding of the ART polypeptide to the melanocortin receptor;
where the ART polypeptide is the fusion protein of Claim 1.

8. The method of Claim 7 where the melanocortin receptor is selected from the group consisting of : the melanocortin-3 receptor (MC3R) and the melanocortin-4 receptor (MC4R).

9. A method for determining whether a substance is a functional inhibitor of the antagonistic effect of an ART polypeptide on a melanocortin receptor where the method comprises:
(a) providing cells expressing a melanocortin receptor;
(b) exposing the cells to a melanocyte stimulating hormone selected from the group consisting of: α-melanocyte stimulating hormone, β-melanocyte stimulating hormone, and γ-melanocyte stimulating hormone, in order to activate the melanocortin receptor, leading to the production of cAMP;
(c) exposing the cells to an ART polypeptide in the presence and in the absence of the substance under conditions such that if the substance were not present, the ART polypeptide would inhibit the production of cAMP mediated by the melanocortin receptor;
(d) measuring the amount of cAMP produced in the presence and in the absence of the substance;
where an increase in the amount of cAMP produced in the presence as compared to the absence of the substance indicates that the substance is a functional inhibitor of the antagonistic effect of the ART polypeptide on the melanocortin receptor;
where the ART polypeptide is the fusion protein of Claim 1.

10. The method of Claim 9 where the melanocortin receptor is selected from the group consisting of: the melanocortin-3 receptor (MC3R) and the melanocortin-4 receptor (MC4R).

11. A method of determining whether a substance is an inhibitor of the effect of an ART polypeptide comprising:
(a) providing a Xenopus melanophore cell line;
(b) exposing the Xenopus melanophore cell line to a chosen concentration of α-melanocyte stimulating hormone in the absence of the ART polypeptide and in the absence of the substance and measuring the amount of pigment dispersion to obtain a first value for pigment dispersion;
(c) exposing the Xenopus melanophore cell line to the chosen concentration of α-melanocyte stimulating hormone in the presence of the ART polypeptide and in the absence of the substance and measuring the amount of pigment dispersion to obtain a second value for pigment dispersion where the second value for pigment dispersion indicates that less pigment has been dispersed as compared to the first value for pigment dispersion;
(d) exposing the Xenopus melanophore cell line to the chosen concentration of α-melanocyte stimulating hormone in the presence of the ART polypeptide and in the presence of the substance and measuring the amount of pigment dispersion to obtain a third value for pigment dispersion;
where if the third value for pigment dispersion indicates that more pigment has been dispersed as compared with the second value, then the substance is an inhibitor of the effect of the ART polypeptide;
where the ART polypeptide is the fusion protein of Claim 1.

12. A method of determining whether a substance is an inhibitor of the binding of an ART polypeptide to a melanocortin receptor comprising :
(a) providing cells expressing the melanocortin receptor;
(b) exposing the cells to a chosen concentration of the melanocyte stimulating hormone and a chosen concentration of the ART polypeptide in the presence and in the absence of the substance and measuring the amount of melanocyte stimulating hormone binding to the cells in the presence and in the absence of the substance;
where an increase in the amount of melanocyte stimulating hormone binding in the presence of the substance indicates that the substance is an inhibitor of the binding of an ART polypeptide to a melanocortin receptor;
where, the ART polypeptide is the fusion protein of Claim 1.

13. The method of Claim 1.2 where the melanocortin receptor is selected from the group consisting of: the melanocortin-3 receptor (MC3R) and the melanocortin-4 receptor (MC4R).

## Patentansprüche

1. Fusionsprotein mit einer Aminosäuresequenz, welche aus der Gruppe ausgewählt ist, die aus den SEQ-ID-Nrn. 1-3, 10-12, 15 und 16 besteht.

2. DNA-Sequenz, welche für das Fusionsprotein nach Anspruch 1 codiert.

3. Verfahren zur Feststellung, ob eine Substanz ein Inhibitor der Bindung eines ART-Polypeptids an einen Melanocortin-Rezeptor ist, wobei das Verfahren umfasst:
(a) Bereitstellen von Zellen, die den Melanocortin-Rezeptor exprimieren;
(b) Aussetzen der Zellen einer ausgewählten Konzentration des melanozytenstimulierenden Hormons in Abwesenheit des ART-Polypeptids und in Abwesenheit der Substanz und Messen des Ausmaßes der Bindung des melanozytenstimulierenden Hormons an die Zellen, um einen ersten Wert für die Bindung des melanozytenstimulierenden Hormons zu erhalten;
(c) Aussetzen der Zellen der ausgewählten Konzentration des melanozytenstimulierenden Hormons in Anwesenheit einer ausgewählten Konzentration des ART-Polypeptids und in Abwesenheit der Substanz und Messen des Ausmaßes der Bindung des melanozytenstimulierenden Hormons, um einen zweiten Wert für die Bindung des melanozytenstimulierenden Hormons zu erhalten, wobei der zweite Wert für die Bindung des melanozytenstimulierenden Hormons anzeigt, dass im Vergleich zu dem ersten Wert für die Bindung des melanozytenstimulierenden Hormons weniger Bindung von melanozytenstimulierendem Hormon erfolgt ist;
(d) Aussetzen der Zellen der ausgewählten Konzentration des melanozytenstimulierenden Hormons in Anwesenheit der ausgewählten Konzentration des ART-Polypeptids und in Anwesenheit der Substanz und Messen des Ausmaßes der Bindung des melanozytenstimulierenden Hormons, um einen dritten Wert für die Bindung des melanozytenstimulierenden Hormons zu erhalten;
wobei die Substanz ein Inhibitor der Bindung des ART-Polypeptids an den Melanocortin-Rezeptor ist, wenn der dritte Wert für die Bindung des melanozytenstimulierenden Hormons größer als der zweite Wert ist;
wobei das ART-Polypeptid das Fusionsprotein nach Anspruch 1 ist.

4. Verfahren nach Anspruch 3, wobei der Melanocortin-Rezeptor aus der Gruppe ausgewählt ist, die aus dem Melanocortin-3-Rezeptor (MC3R) und dem Melanocortin-4-Rezeptor (MC4R) besteht.

5. Verfahren zur Feststellung, ob eine Substanz ein Inhibitor der Bindung eines ART-Polypeptids an einen Melanocortin-Rezeptor ist, wobei das Verfahren umfasst:
(a) Bereitstellen von Zellen, die einen Melanocortin-Rezeptor exprimieren;
(b) Aussetzen der Zellen einem ART-Polypeptid in Anwesenheit und Abwesenheit der Substanz unter solchen Bedingungen, dass das ART-Polypeptid an den Melanocortin-Rezeptor binden würde, wenn die Substanz nicht anwesend wäre;
(c) Messen des Ausmaßes der Bindung des ART-Polypeptids an den Melanocortin-Rezeptor in Anwesenheit und Abwesenheit der Substanz;
wobei eine Verringerung des Ausmaßes der Bindung des ART-Polypeptids an den Melanocortin-Rezeptor in Anwesenheit der Substanz im Vergleich zur Abwesenheit der Substanz anzeigt, dass die Substanz ein Inhibitor der Bindung des ART-Polypeptids an den Melanocortin-Rezeptor ist;
wobei das ART-Polypeptid das Fusionsprotein nach Anspruch 1 ist.

6. Verfahren nach Anspruch 5, wobei der Melanocortin-Rezeptor aus der Gruppe ausgewählt ist, die aus dem Melanocortin-3-Rezeptor (MC3R) und dem Melanocortin-4-Rezeptor (MC4R) besteht.

7. Verfahren zur Feststellung, ob eine Substanz ein allosterischer Enhancer der Bindung eines ART-Polypeptids an einen Melanocortin-Rezeptor ist, wobei das Verfahren umfasst:
(a) Bereitstellen von Zellen, die einen Melanocortin-Rezeptor exprimieren;
(b) Aussetzen der Zellen einem ART-Polypeptid in Anwesenheit und Abwesenheit der Substanz unter solchen Bedingungen, dass das ART-Polypeptid an den Melanocortin-Rezeptor binden würde, wenn die Substanz nicht anwesend wäre;
(c) Messen des Ausmaßes der Bindung des ART-Polypeptids an den Melanocortin-Rezeptor in Anwesenheit und Abwesenheit der Substanz;
wobei eine Erhöhung des Ausmaßes der Bindung des ART-Polypeptids an den Melanocortin-Rezeptor in Anwesenheit der Substanz im Vergleich zur Abwesenheit der Substanz anzeigt, dass die Substanz ein allosterischer Enhancer der Bindung des ART-Polypeptids an den Melanocortin-Rezeptor ist;
wobei das ART-Polypeptid das Fusionsprotein nach Anspruch 1 ist.

8. Verfahren nach Anspruch 7, wobei der Melanocortin-Rezeptor aus der Gruppe ausgewählt ist, die aus dem Melanocortin-3-Rezeptor (MC3R) und dem Melanocortin-4-Rezeptor (MC4R) besteht.

9. Verfahren zur Feststellung, ob eine Substanz ein funktioneller Inhibitor der antagonistischen Wirkung eines ART-Polypeptids auf einen Melanocortin-Rezeptor ist, wobei das Verfahren umfasst:
(a) Bereitstellen von Zellen, die einen Melanocortin-Rezeptor exprimieren;
(b) Aussetzen der Zellen einem melanozytenstimulierenden Hormon, welches aus der Gruppe ausgewählt ist, die aus a-melanozytenstimulierendem Hormon, β-melanozytenstimulierendem Hormon und γ-melanozytenstimulierendem Hormon besteht, um den Melanocortin-Rezeptor zu aktivieren, was zur Bildung von cAMP führt;
(c) Aussetzen der Zellen einem ART-Polypeptid in Anwesenheit und Abwesenheit der Substanz unter solchen Bedingungen, dass das ART-Polypeptid die durch den Melanocortin-Rezeptor vermittelte Bildung von cAMP inhibieren würde, wenn die Substanz nicht anwesend wäre;
(d) Messen der Menge an cAMP, die in Anwesenheit und Abwesenheit der Substanz gebildet wird;
wobei eine Erhöhung der gebildeten Menge an cAMP in Anwesenheit der Substanz im Vergleich zur Abwesenheit der Substanz anzeigt, dass die Substanz ein funktioneller Inhibitor der antagonistischen Wirkung des ART-Polypeptids auf den Melanocortin-Rezeptor ist;
wobei das ART-Polypeptid das Fusionsprotein nach Anspruch 1 ist.

10. Verfahren nach Anspruch 9, wobei der Melanocortin-Rezeptor aus der Gruppe ausgewählt ist, die aus dem Melanocortin-3-Rezeptor (MC3R) und dem Melanocortin-4-Rezeptor (MC4R) besteht.

11. Verfahren zur Feststellung, ob eine Substanz ein Inhibitor der Wirkung eines ART-Polypeptids ist, umfassend:
(a) Bereitstellen einer Xenopus-Melanophor-Zelllinie;
(b) Aussetzen der Xenopus-Melanophor-Zelllinie einer ausgewählten Konzentration von α-melanozytenstimulierendem Hormon in Abwesenheit des ART-Polypeptids und in Abwesenheit der Substanz und Messen des Ausmaßes der Pigment-Dispersion, um einen ersten Wert für die Pigment-Dispersion zu erhalten;
(c) Aussetzen der Xenopus-Melanophor-Zelllinie der ausgewählten Konzentration von α-melanozytenstimulierendem Hormon in Anwesenheit des ART-Polypeptids und in Abwesenheit der Substanz und Messen des Ausmaßes der Pigment-Dispersion, um einen zweiten Wert für die Pigment-Dispersion zu erhalten; wobei der zweite Wert für die Pigment-Dispersion anzeigt, dass im Vergleich zum ersten Wert für die Pigment-Dispersion weniger Pigment dispergiert wurde;
(d) Aussetzen der Xenopus-Melanophor-Zelllinie der ausgewählten Konzentration von α-melanozytenstimulierendem Hormon in Anwesenheit des ART-Polypeptids und in Anwesenheit der Substanz und Messen des Ausmaßes der Pigment-Dispersion, um einen dritten Wert für die Pigment-Dispersion zu erhalten;
wobei die Substanz ein Inhibitor der Wirkung des ART-Polypeptids ist, wenn der dritte Wert für die Pigment-Dispersion anzeigt, dass im Vergleich zum zweiten Wert mehr Pigment dispergiert wurde;
wobei das ART-Polypeptid das Fusionsprotein nach Anspruch 1 ist.

12. Verfahren zur Feststellung, ob eine Substanz ein Inhibitor der Bindung eines ART-Polypeptids an einen Melanocortin-Rezeptor ist, wobei das Verfahren umfasst:
(a) Bereitstellen von Zellen, die den Melanocortin-Rezeptor exprimieren;
(b) Aussetzen der Zellen einer ausgewählten Konzentration des melanozytenstimulierenden Hormons und einer ausgewählten Konzentration des ART-Polypeptids in Anwesenheit und Abwesenheit der Substanz und Messen des Ausmaßes der Bindung des melanozytenstimulierenden Hormons an die Zellen in Anwesenheit und Abwesenheit der Substanz;
wobei eine Erhöhung des Ausmaßes der Bindung des melanozytenstimulierenden Hormons in Anwesenheit der Substanz anzeigt, dass die Substanz ein Inhibitor der Bindung eines ART-Polypeptids an einen Melanocortin-Rezeptor ist;
wobei das ART-Polypeptid das Fusionsprotein nach Anspruch 1 ist.

13. Verfahren nach Anspruch 12, wobei der Melanocortin-Rezeptor aus der Gruppe ausgewählt ist, die aus dem Melanocortin-3-Rezeptor (MC3R) und dem Melanocortin-4-Rezeptor (MC4R) besteht.

## Revendications

1. Protéine hybride ayant une séquence d'acides aminés choisie dans le groupe constitué de SEQ ID NO : 1 à 3, 10 à 12, 15 et 16.

2. Séquence d'ADN codant la protéine hybride selon la revendication 1.

3. Procédé pour déterminer si une substance est un inhibiteur de la liaison d'un polypeptide ART à un récepteur de la mélanocortine, où le procédé comprend :
(a) l'apport de cellules exprimant le récepteur de la mélanocortine ;
(b) l'exposition des cellules à une concentration choisie d'hormone mélanotrope en l'absence du polypeptide ART et en l'absence de la substance et la mesure de la quantité de liaison de l'hormone mélanotrope aux cellules pour obtenir une première valeur pour la liaison de l'hormone mélanotrope ;
(c) l'exposition des cellules à la concentration choisie d'hormone mélanotrope en présence d'une concentration choisie du polypeptide ART et en l'absence de la substance et la mesure de la quantité de liaison de l'hormone mélanotrope pour obtenir une deuxième valeur pour la liaison de l'hormone mélanotrope, où la deuxième valeur pour la liaison de l'hormone mélanotrope indique qu'une liaison inférieure d'hormone mélanotrope s'est produite en comparaison à la première valeur pour la liaison de l'hormone mélanotrope ;
(d) l'exposition des cellules à la concentration choisi d'hormone mélanotrope en présence de la concentration choisie du polypeptide ART et en présence de la substance et la mesure de la quantité de liaison de l'hormone mélanotrope pour obtenir une troisième valeur pour la liaison de l'hormone mélanotrope ;
où, si la troisième valeur pour la liaison de l'hormone mélanotrope est supérieure à la deuxième valeur, alors la substance est un inhibiteur de la liaison du polypeptide ART au récepteur de la mélanocortine ;
où le polypeptide ART est la protéine hybride selon la revendication 1.

4. Procédé selon la revendication 3, dans lequel le récepteur de la mélanocortine est choisi dans le groupe constitué par: le récepteur de la mélanocortine 3 (MC3R) et le récepteur de la mélanocortine 4 (MC4R).

5. Procédé pour déterminer si une substance est un inhibiteur de la liaison d'un polypeptide ART à un récepteur de la mélanocortine, où le procédé comprend :
(a) l'apport de cellules exprimant un récepteur de la mélanocortine ;
(b) l'exposition des cellules à un polypeptide ART en présence et en l'absence de la substance dans des conditions telles que si la substance n'était pas présente, le polypeptide ART se lierait au récepteur de la mélanocortine ;
(c) la mesure de la quantité de liaison du polypeptide ART au récepteur de la mélanocortine en présence et en l'absence de la substance ;
où une baisse de la quantité de liaison du polypeptide ART au récepteur de la mélanocortine en présence de la substance, en comparaison à son absence, indique que la substance est un inhibiteur de la liaison du polypeptide ART au récepteur de la mélanocortine ;
où le polypeptide ART est la protéine hybride selon la revendication 1.

6. Procédé selon la revendication 5, où le récepteur de la mélanocortine est choisi dans le groupe constitué par : le récepteur de la mélanocortine 3 (MC3R) et le récepteur de la mélanocortine 4 (MC4R).

7. Procédé pour déterminer si une substance est un stimulateur allostérique de la liaison d'un polypeptide ART à un récepteur de la mélanocortine, où le procédé comprend :
(a) l'apport de cellules exprimant un récepteur de la mélanocortine ;
(b) l'exposition des cellules à un polypeptide ART en présence et en l'absence de la substance dans des conditions telles que si la substance n'était pas présente, le polypeptide ART se lierait au récepteur de la mélanocortine ;
(c) la mesure de la quantité de liaison du polypeptide ART au récepteur de la mélanocortine en présence et en l'absence de la substance ;
où une augmentation de la quantité de liaison du polypeptide ART au récepteur de la mélanocortine en présence de la substance, en comparaison à son absence, indique que la substance est un stimulateur allostérique de la liaison du polypeptide ART au récepteur de la mélanocortine ;
où le polypeptide ART est la protéine hybride selon la revendication 1.

8. Procédé selon la revendication 7, où le récepteur de la mélanocortine est choisi dans le groupe constitué par : le récepteur de la mélanocortine 3 (MC3R) et le récepteur de la mélanocortine 4 (MC4R).

9. Procédé pour déterminer si une substance est un inhibiteur fonctionnel de l'effet antagoniste d'un polypeptide ART sur un récepteur de la mélanocortine, où le procédé comprend :
(a) l'apport de cellules exprimant un récepteur de la mélanocortine ;
(b) l'exposition des cellules à une hormone mélanotrope choisie dans le groupe constitué par: l'hormone mélanotrope α, l'hormone mélanotrope β et l'hormone mélanotrope γ, afin d'activer le récepteur de la mélanocortine, conduisant à la production d'AMPc ;
(c) l'exposition des cellules à un polypeptide ART en présence et en l'absence de la substance dans des conditions telles que si la substance n'était pas présente, le polypeptide ART inhiberait la production d'AMPc à médiation par le récepteur de la mélanocortine ;
(d) la mesure de la quantité d'AMPc produite en présence et en l'absence de la substance ;
où une augmentation de la quantité d'AMPc produite en présence de la substance, en comparaison à son absence, indique que la substance est un inhibiteur fonctionnel de l'effet antagoniste du polypeptide ART sur le récepteur de la mélanocortine ;
où le polypeptide ART est la protéine hybride selon la revendication 1.

10. Procédé selon la revendication 9, où le récepteur de la mélanocortine est choisi dans le groupe constitué par: le récepteur de la mélanocortine 3(MC3R) et le récepteur de la mélanocortine 4(MC4R).

11. Procédé pour déterminer si une substance est un inhibiteur de l'effet d'un polypeptide ART comprenant :
(a) l'apport d'une lignée de mélanophores de xénope ;
(b) l'exposition de la lignée de mélanophores de xénope à une concentration choisie d'hormone mélanotrope α en l'absence du polypeptide ART et en l'absence de la substance et la mesure de la quantité de dispersion des pigments pour obtenir une première valeur pour la dispersion de pigments ;
(c) l'exposition de la lignée de mélanophores de xénope à la concentration choisie d'hormone mélanotrope α en présence du polypeptide ART et en l'absence de la substance et la mesure de la quantité de dispersion de pigments pour obtenir une deuxième valeur pour la dispersion de pigments, où la deuxième valeur pour la dispersion de pigments indique qu'une quantité inférieure de pigments a été dispersée en comparaison à la première valeur pour la dispersion de pigments ;
(d) l'exposition de la lignée de mélanophores de xénope à la concentration choisie d'hormone mélanotrope α en présence du polypeptide ART et en présence de la substance et la mesure de la quantité de dispersion de pigments pour obtenir une troisième valeur pour la dispersion de pigments ;
où si la troisième valeur pour la dispersion de pigments indique qu'une quantité supérieure de pigments a été dispersée en comparaison à la deuxième valeur, alors la substance est un inhibiteur de l'effet du polypeptide ART ;
où le polypeptide ART est la protéine hybride selon la revendication 1.

12. Procédé pour déterminer si une substance est un inhibiteur de la liaison d'un polypeptide ART à un récepteur de la mélanocortine comprenant:
(a) l'apport de cellules exprimant le récepteur de la mélanocortine ;
(b) l'exposition des cellules à une concentration choisie d'hormone mélanotrope et à une concentration choisie du polypeptide ART en présence et en l'absence de la substance et la mesure de la quantité de liaison de l'hormone mélanotrope aux cellules en présence et en l'absence de la substance ;
où une augmentation de la quantité de liaison de l'hormone mélanotrope en présence de la substance indique que la substance est un inhibiteur de la liaison d'un polypeptide ART à un récepteur de la mélanocortine ;
où le polypeptide ART est la protéine hybride selon la revendication 1.

13. Procédé selon la revendication 12, où le récepteur de la mélanocortine est choisi dans le groupe constitué par : le récepteur de la mélanocortine 3 (MC3R) et le récepteur de la mélanocortine 4 (MC4R).
